# EUROPEAN PATENT APPLICATION

(11) **EP 2 428 204 A2**
(43) Date of publication of application: **14.03.2012**
(21) Application number: 11185836.1
(22) Date of filing: 06.12.2007
(51) Int. Cl.: A61K 9/20, A61K 31/155

(54) **pH sensitive matrix formulation**

(30) Priority: 07.12.2006 US 873928 P; 07.12.2006 US 873872 P
(62) Divisional of application: 07862611.6
(71) Applicant: Schering Corporation, Kenilworth, NJ 07033 (US)
(72) Inventor: Wan, Jiansheng, Rahway, NJ 07065-0907 (US); Chen, Xiaoming, Rahway, NJ 07065-0907 (US); Gupta, Pranav, Rahway, NJ 07065-0907 (US); Monteith, David, Rahway, NJ 07065-0907 (US)
(74) Representative: Horgan, James Michael Frederic

(57) **Abstract**

The present invention provides formulations of therapeutic agents that benefit from a prolonged time of controlled release in the stomach and upper gastrointestinal (GI) tract, and from an enhanced drug exposure to the upper GI tract. The formulations of the invention comprise a therapeutic agent and one or more pH sensitive polymers that are designed for accelerated hydration, expansion, disintegration and dissolution at the higher pH of the upper GI tract, thereby, ensuring that any therapeutic agent that has not been released in the stomach is released in the upper GI tract, thus maximizing absorption of therapeutic agent that has a window of absorption located at the upper GI tract.

## Description

### FIELD OF THE INVENTION

The present invention in certain embodiments relates to formulations for therapeutic agents that benefit from a prolonged time of controlled release in the stomach and upper gastrointestinal (GI) tract, and to formulations that benefit from an enhanced drug exposure to the upper GI tract. One goal of certain embodiments of this invention is to extend the dosage form residence time in the stomach by a size exclusion mechanism and thus to provide continued prolonged drug exposure to the upper GI tract, for purposes of achieving a greater and more prolonged therapeutic effect and thus reducing the frequency of administration required and a more efficient use of the therapeutic agent. The formulations of the invention comprise one or more pH sensitive polymers that are designed for accelerated hydration, expansion, disintegration and dissolution at the higher pH of the upper GI tract, thereby, ensuring that any drug that has not been released in the stomach is released in the upper GI tract, thus maximizing absorption of drug that has a narrow window of absorption located at the upper GI tract.

### BACKGROUND OF INVENTION

For many drugs, maintaining a constant bloodstream and tissue concentration throughout the course of therapy is highly desirable. Immediate release of these drugs can cause blood levels to peak above the level required to elicit the desired response, resulting in undesirable side effects.

Many drugs provide better therapy when they are delivered in a controlled release manner. There are known dosage forms that provide sustained/controlled release of the drug within its desired therapeutic window, thereby reducing side effects commonly associated with immediate release dosage forms. In some sustained release dosage forms, the active ingredient is embedded in a matrix that slowly erodes to release the active ingredient. Other sustained and delayed release dosage forms have a coating.

Conventional orally-administered dosage forms are most readily dissolved or disintegrated in the stomach. The residence time of such a dosage form in the stomach is 1 to 3 hours on average. Gastric retention dosage forms, i.e., dosage forms that are designed to be retained in the stomach for a prolonged period of time, can increase the duration of drug absorption, and thus bioavailability of drugs which are absorbed primarily in the upper gastrointestinal tract.

A composition that is formulated to dissolve upon contact with an aqueous solution will at least partially dissolve in the stomach because it reaches the stomach before it reaches the intestine. However, unless the drug is very rapidly absorbed, or the residence time is increased, some of the drug will pass into the intestine undissolved. For the foregoing reasons, it is desirable to develop pharmaceutical formulations that ensure the rapid and efficient release of any drug remaining in the formulation as it passes from the stomach to the intestine.

### SUMMARY OF THE INVENTION

The present invention provides novel controlled release formulations, preferably tablets, that, in certain embodiments, have gastro-retentive properties. The present invention provides controlled release formulations that provide rapid release of the drug as they move from the stomach into the higher pH of the intestine. Moreover, the controlled release formulations are adapted for continued drug release and retention of dosage form in the stomach and for rapid release of drug as it moves from the stomach into the higher pH environment of the intestine. The invention also provides methods for efficient delivery of drug for improved bioavailability and convenience of dosing. This invention is distinctly different from an enteric coated controlled release tablet in that an enteric coated tablet provides little, if any, drug release in the acidic pH of the stomach.

In one aspect, the invention provides drug formulations suited for oral administration comprising one or more pH sensitive polymers that extend the time of drug delivery into both the stomach and upper GI tract for purposes of achieving a greater and more prolonged therapeutic effect. The pH sensitive polymers are selected to augment drug dissolution/release at the higher pH of the intestine thereby releasing any drug remaining associated in the formulation as it is expelled from the stomach to reach the small intestine. In a non-limiting embodiment of the invention, such pH sensitive polymers may be further characterized by their ability to imbibe water and expand, thereby, further increasing the likelihood of drug release in the desired gastric-intestinal environment.

Accordingly, the present invention provides a controlled release formulation, preferably having gastro-retentive properties adapted for oral administration comprising one or more pH sensitive polymers and a therapeutic agent, wherein the pH sensitive polymer allows for release of the therapeutic agent in the increased pH of the small intestine. In an embodiment of the invention, the pH sensitive polymer is Carbapol 71 G, hydroxypropylmethylcellulose acetate succinate (a.k.a. hypromellose acetate succinate) (HPMCAS), Eudragit L-100, Eudragit S-100, Eudragit L-30D, Euragit FS 30D, Eudragit L-100-55, polyvinyl acetate phthalate, hydroxypropyl methylcellulose phthalate, hydroxypropyl methylcellulose phthalate 50, hydroxypropyl methylcellulose phthalate 55, cellulose acetate phthalate or cellulose acetate trimellate or a mixture of two or more said polymers..

In another embodiment of the invention, the formulations of the invention further comprise a swellable biocompatible hydrophilic polymer that is not necessarily a pH sensitive polymer, which is capable of volume-expansion in the aqueous environment of the stomach to a size that increases the likelihood that the composition will be retained in the stomach for a prolonged period of time.

Accordingly, the controlled release formulations of the present invention can further comprise a swellable hydrophilic polymer. In a specific embodiment of the invention, the swellable hydrophilic polymer is a synthetic hydrophilic polymer. Synthetic polymers useful herein include, but are not limited to: polyalkylene oxides, particularly poly(ethylene oxide), polyethylene glycol and poly(ethylene oxide)-poly(propylene oxide) copolymers; cellulosic polymers; acrylic acid and methacrylic acid polymers, copolymers and esters thereof, preferably formed from acrylic acid, methacrylic acid, methyl acrylate, ethyl acrylate, methyl methacrylate, ethyl methacrylate, and copolymers thereof, with each other or with additional acrylate species such as aminoethyl acrylate; maleic anhydride copolymers; polymaleic acid;poly(acrylamides) such as polyacrylamide per se, poly(methacrylamide), poly(dimethylacrylamide), and poly(N-isopropyl-acrylamide); poly(olefinic alcohol) such as poly(vinyl alcohol); poly(N-vinyl lactams) such as poly(vinyl pyrrolidone), poly(N-vinyl caprolactam), and copolymers thereof; polyols such as glycerol, polyglycerol (particularly highly branched polyglycerol), propylene glycol and trimethylene glycol substituted with one or more polyalkylene oxides, e.g., mono-, di- and tri-polyoxyethylated glycerol, mono- and di-polyoxyethylated propylene glycol, and mono- and di-polyoxyethylated trimethylene glycol; polyoxyethylated sorbitol and polyoxyethylated glucose; polyoxazolines, including poly(methyloxazoline) and poly(ethyloxazoline); polyvinylamines; polyvinylacetates, including polyvinylacetate per se as well as ethylene-vinyl acetate copolymers, polyvinyl acetate phthalate, and the like; polyimines, such as polyethyleneimine; starch and starch-based polymers; polyurethane hydrogels; chitosan; polysaccharide gums; zein; and shellac, ammoniated shellac, shellac-acetyl alcohol, and shellac n-butyl stearate. In a preferred embodiment of the invention, the swellable polymer is a polyvinyl acetate. In yet another embodiment of the invention, the polyvinyl acetate is Kollidon SR.

As indicated above, the formulations of invention comprise one or more therapeutic agents formulated for controlled release upon ingestion. Such agents are those compounds capable of exerting a therapeutically beneficial effect on a patient and include prodrugs, hydrates, solvates, molecular complexes, co-crystals, co-precipitates, and pharmaceutically acceptable salts and derivatives of the compound (s).

In specific embodiments of the invention, the formulation of the invention comprises an HCV serine protease inhibitor, in particular compound 1, 2 or 3 defined below; metaformin; or Niacin.

The controlled release formulations of the invention may further comprise one or more pharmaceutically acceptable adjuvants, such as a surfactant, pharmaceutically acceptable carrier and an excipients such as a filler, binder, glidant, lubricant, and/or disintegrant. Each excipient must be acceptable in the sense of being compatible with the other ingredients of the formulation and not injurious to the mammal in need of treatment.

In a specific embodiment of the invention, the disintegrant is a super disintegrant. In another embodiment of the invention, the disintegrant is a cross-linked carboxymethyl cellulose sodium, sodium starch glycolate, cross-linked polyvinyl pyrollidone or low substituted hydroxylpropyl cellulose.

In another aspect of the invention, a controlled release formulation adapted for oral administration is provided comprising one or more swellable hydrophilic polymers, one or more pH sensitive polymers, a superdisintegrant and a therapeutic agent.

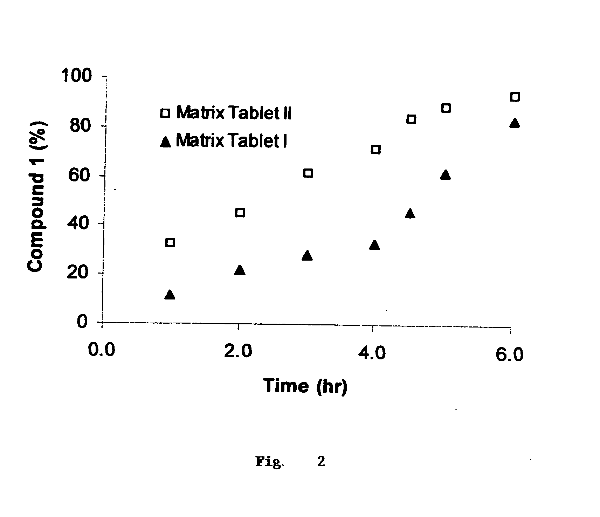

In an embodiment of the invention, a controlled release formulation adapted for oral administration is provided comprising one or more swellable hydrophilic polymers, one or more pH sensitive polymers, a superdisintegrant and a therapeutic agent, having the dissolution profile shown in Figure 2, 3, 6, 7 or 8 when tested in a USP2 apparatus Paddle Stirrer filled with 900 ml pH 1.2 HCl medium, with or without 0.5% Tween 80, for three to four hours, followed by 900 ml phosphate buffer at pH 6.8, with or without 0.5% Tween 80, for 2 hours at 37 °C with 50 rpm stir speed.

In a specific embodiment of the invention, a controlled release formulation for an HCV protease inhibitor (compound 1, as defined below) comprising the formulation shown in Table 1 or Table 2 is provided. In another specific embodiment, a controlled release formulation for an HCV protease inhibitor (compound 2, as defined below) comprising the formulation shown in Table 6 is provided. In another specific embodiment, a controlled release formulation for Metformin comprising the formulation shown in Table 7 or Table 8 is provided.

In yet another specific embodiment, a controlled release formulation for Niacin comprising the formulation shown in Table 9 or Table 10 is provided.

In a further aspect, the invention provides a method of treating a subject in need thereof with a therapeutic agent that comprises administering to the subject a dosage form adapted to be retained in the stomach over a prolonged period and further adapted to rapidly disintegrate at the higher pH of the upper GI tract. Specifically, a method is provided of averting, ameliorating one or more symptoms or treating disease by administering to a patient susceptible to or afflicted with the disease a therapeutically effective amount of a therapeutic agent contained in the gastric controlled release formulations of the present invention.

The method may further comprise administering one or more dosage forms to a subject in the fed state at the start of each dosing period, such as within one hour of the subject consuming food. The presence of food will delay gastric emptying and provide increased retention of the dosage form in the stomach.

Another embodiment of the invention is a process for making a controlled release formulation of the invention comprising the steps of: a) blending one or more swellable hydrophilic polymers, one or more pH sensitive polymers, a superdisintegrant and a therapeutic agent, and b) compressing the combination. Yet another embodiment of the invention is a process for making a controlled release formulation of the invention comprising the steps of: a) wet granulation of the therapeutic agent with one or more swellable hydrophilic polymers, one or more pH sensitive polymers, and a superdisintegrant b) blending one or more swellable hydrophilic polymers, one or more pH sensitive polymers, a superdisintegrant and a therapeutic agent, and c) compressing the combination.

These oral dosage forms are adapted to address problems with current therapies which negatively affect duration of drug absorption and biovailability. The present invention provides formulations or dosage forms with advantageious duration of drug absorption and bioavailability. The present invention therefore further provides methods of treating diseases with such dosage forms, by administering a dosage form of this invention.

### BRIEF DESCRIPTION OF THE FIGURES

Figure 1. Images of Matrix Tablet 1 for HCV protease inhibitor compound 1 (Left: Start; Middle: in pH 1.2 HCl, 15 min.; Right: in pH 1.2 HCl for 1 hour)
Figure 2. Dissolution profile of Matrix Tablets I and II for HCV protease inhibitor compound 1 (0-4 hr in pH 1.2 HCl, 0.5% Tween 80; 4-6 hr in pH 6.8 phosphate buffer, 0.5% Tween 80)
Figure 3. Dissolution profile of Matrix Tablet III and IV for HCV protease compound 1 (0-4 hr in pH 1.2 HCl, 0.5% Tween 80; 4-6 hr in pH 6.8 phosphate buffer, 0.5% Tween 80, 50 rpm)
Figure 4. Comparison of *in vivo* results of a bolus dose to divided doses
Figure 5A. Simulated profiles for input rates of 160 mg/hr for 5 hours and 133 mg/hr for 6 hours
Figure 5B. Simulated steady state profiles for an input at 133 mg/hr dosed at 8 hour intervals.
Figure 6. Dissolution profile of of Formulation #16 (Carbopol based) and #19 (Calcium polycarbophil based) for HCV protease compound 2 (0-4 hr in pH 1.2 HCl, 0.5% Tween 80; 4-6 hr in pH 6.8 phosphate buffer, 0.5% Tween 80; 100 rpm, 37 °C)
Figure 7. Dissolution profile of formulation #22 of Metformin (0-3 hr in pH 1.2 HCl; 3-5 hr in pH 6.8 phosphate buffer; 50 rpm at 37 °C)
Figure 8. Dissolution profile of formulation #5 of Niacin (0-3 hr in pH 1.2 HCl; 3-5 hr in pH 6.8 phosphate buffer; 50 rpm at 37 °C)

### DETAILED DESCRIPTION OF THE INVENTION

The present invention provides novel controlled release drug formulations, preferably with gastroretentive properties. The formulations are adapted for controlled release of the drug in the stomach followed by a pH-enhanced more complete release of any remaining drug from a matrix as it enters the intestinal environment. The purpose is to maximize absorption of drug that is absorbed primarily and only in the upper gastrointestinal tract. The formulations of the invention comprise one or more pH sensitive polymers that accelerate hydration, expansion, and/or disintegration at the higher pH of the intestine thereby ensuring enhanced dissolution of embedded drug as it moves from the stomach to the intestine. The formulations otherwise ensure more complete release of the drug in the upper GI tract in the event of unexpected exit from the stomach thereby resulting in maximized drug absorption.

The present invention is based, in part, on the observation that prolonged drug release in the stomach will significantly prolong duration of drug absorption at the upper GI tract and thus lead to a significant increase in bioavailability or improved PK profile of drugs that have a limited absorption window located at the upper GI tract.

The present invention provides a controlled release formulation, optionally with gastric retention properties, that expands upon contact with the gastric fluid of a patient followed by a rapid volume expansion/disintegration at the intestinal pH to provide enhanced drug release in the upper GI tract after it moves from the stomach to the small intestine. The expanding composition is advantageously used as a gastric retention delivery system in an orally administered pharmaceutical dosage form to increase the likelihood that the dosage form will be retained in a patient's stomach for a prolonged period of time and that any remaining unreleased drug is quickly released as it moves from the stomach to the small intestine.

Controlled-release is a term known in the medicinal art and is typically used interchangeably with sustained release, slow release, controlled availability, slow acting, extended release, and metered release. Controlled-release is generally defined as the release of an agent from a dosage formulation slowly over a period of time, such as over hours or days.

The compositions of the present invention are formulated with one or more controlled-release polymer matrices to provide the rate controlled-release of any one or more of the therapeutic agents to optimize the therapeutic effects. Such controlled release polymeric matrices are impregnated with the therapeutic agents and shaped in tablet form or capsules containing such impregnated or encapsulated porous polymeric matrices.

As used herein, in connection with the ingredients or components of the present formulations, the terms "at least one" and "one or more" mean that 1-3 of the specified components can be included, e.g. 1-3 pH sensitive polymers, 1-3 swellable polymers. Preferably 1-2 of the specified components are used.

The present invention is based at least in part, on the use of at least one water imbibing pH sensitive polymer in the formulations of the invention. The use of such a pH sensitive polymer ensures rapid volume expansion/disintegration and dissolution of any remaining drug in the dosage form that leaves the stomach earlier than anticipated and potentially passes the absorption window before the remaining drug has had the opportunity to dissolve and be absorbed. Such water imbibing pH sensitive polymers include, but are not limited to, Carbopols (polymers of acrylic acid crosslinked with a polyfunctional agent, manufactured by B.F. Goodrich Chemical Group) such as Carbopol 71 G, Carbopol 971 P NF, Carbopol 974 P NF, Carbopol 934 P NF, Cabopol 5984 EP or Carbopol 980 NF. The water imbibing pH sensitive polymer is preferably present at from about 5 to 80 weight percent (wt.%), or more preferably about 8 to 65 weight percent (wt.%), or more preferably about 10 to 50 weight percent (wt.%).

Additionally, the formulations of the invention may comprise one or more pH sensitive polymers, not necessarily strong water imbibing polymers, that function to further augment pH-induced disintegration and dissolution of the formulation as it passes from the stomach to the small intestine. Such pH sensitive polymers include, but are not limited to, hypromellose acetate succinate (HPMCAS), polymethacrylates such as Eudragit L-100, Eudragit S-100, Eudragit L-30D, Euragit FS 30D, Eudragit L-100-55 (Eudragit polymers are manufactured by EVONIK Industries), polyvinyl acetate phthalate, hydroxypropyl methylcellulose phthalate, hydroxypropyl methylcellulose phthalate 50, hydroxypropyl methylcellulose phthalate 55, cellulose acetate phthalate and cellulose acetate trimellate, 971 P NF, Carbopol 974 P NF, Carbopol 934 P NF. The pH sensitive polymer is preferably present at from about 5 to 75 weight percent (wt.%), or about 6 to 65 weight percent (wt.%), or more preferably about 8 to 55 weight percent (wt.%).

In another aspect of the invention, the compositions of the invention may comprise one or more controlled-release carriers that are swellable polymers. The swellable polymer is a biocompatible or bioerodible, hydrophilic polymer, preferably a cellulosic polymer. The term "hydrophilic" is generally defined in terms of a partition coefficient P, which is the ratio of the equilibrium concentration of a compound in an organic phase to that in an aqueous phase. A hydrophilic compound has a P value less than 1.0, typically less than about 0.5, where P is the partition coefficient of the compound between octanol and water. Hydrophilic polymeric carriers are thus compatible with aqueous fluids such as those present in the human body.

The term "polymer" as used herein refers to a molecule containing a plurality of covalently attached monomer units, and includes branched, dendrimeric and star polymers as well as linear polymers. The term also includes both homopolymers and copolymers, e.g., random copolymers, block copolymers and graft copolymers, as well as uncrosslinked polymers and slightly to moderately to substantially crosslinked polymers.

The terms "swellable" and "bioerodible" (or simply "erodible") are used to refer to the preferred polymers herein, with "swellable" polymers being those that are capable of absorbing water and physically swelling as a result, with the extent to which a polymer can swell being determined by the degree of crosslinking, and "bioerodible" or "erodible" polymers referring to polymers that slowly dissolve and/or gradually hydrolyze in an aqueous fluid, and/or that physically erodes as a result of movement within the stomach or gastrointestinal tract.

Polymers suitable for use in certain embodiments of the present invention are those that both swell upon absorption of gastric fluid and gradually erode over a time period of hours. Erosion may initiate simultaneously with the swelling process, upon contact of the surface of the dosage formulation with gastric fluid. Erosion reflects the dissolution of the polymer beyond the polymer gel-solution interface where the polymer has become sufficiently dilute that it can be transported away from the dosage formulation by diffusion or convection. This may also depend on the hydrodynamic and mechanical forces present in the gastrointestinal tract during the digestive process. While swelling and erosion may occur at the same time, it is preferred herein that drug release should be either diffusion-controlled and or erosion-controlled, meaning that the selected polymer should be such that complete drug release occurs primarily as a result of either diffusion and or erosion rather than just swelling and dissolution. However, swelling should take place at a rate that is sufficiently fast to allow the tablet to be retained in the stomach. At minimum, for a volume-expansive gastric retentive dosage formulation, there should be an extended period during which the dosage formulation maintains its size before it is diminished by erosion.

Suitable polymers for use in the present dosage formulations may be linear, branched, dendrimeric, or star polymers, and include synthetic hydrophilic polymers as well as semi-synthetic and naturally occurring hydrophilic polymers. The polymers may be homopolymers or copolymers, if copolymers, either random copolymers, block copolymers or graft copolymers. Synthetic hydrophilic polymers useful herein include, but are not limited to: polyalkylene oxides, particularly poly(ethylene oxide), polyethylene glycol and poly(ethylene oxide)-poly(propylene oxide) copolymers; cellulosic polymers; acrylic acid and methacrylic acid polymers, copolymers and esters thereof, preferably formed from acrylic acid, methacrylic acid, methyl acrylate, ethyl acrylate, methyl methacrylate, ethyl methacrylate, and copolymers thereof, with each other or with additional acrylate species such as aminoethyl acrylate; maleic anhydride copolymers; polymaleic acid; poly(acrylamides) such as polyacrylamide per se, poly(methacrylamide), poly(dimethylacrylamide), and poly(N-isopropylacrylamide); poly(olefinic alcohol) such as poly(vinyl alcohol); poly(N-vinyl lactams) such as poly(vinyl pyrrolidone), poly(N-vinyl caprolactam), and copolymers thereof; polyols such as glycerol, polyglycerol (particularly highly branched polyglycerol), propylene glycol and trimethylene glycol substituted with one or more polyalkylene oxides, e.g., mono-, di- and tri-polyoxyethylated glycerol, mono- and di-polyoxyethylated propylene glycol, and mono- and di-polyoxyethylated trimethylene glycol; polyoxyethylated sorbitol and polyoxyethylated glucose; polyoxazolines, including poly(methyloxazoline) and poly(ethyloxazoline); polyvinylamines; polyvinylacetates, including polyvinylacetate per se as well as ethylene-vinyl acetate copolymers, polyvinyl acetate phthalate, and the like; polyimines, such as polyethyleneimine; starch and starch-based polymers; polyurethane hydrogels; chitosan; polysaccharide gums; zein; and shellac, ammoniated shellac, shellac-acetyl alcohol, and shellac n-butyl stearate.

In a preferred embodiment of the invention, the expandable hydrophilic polymers to be used in the formulations of the invention are polyvinly acetates. In a preferred embodiment of the invention, the polyvinyl acetate is Kollidon SR, available from BASF Corporation.

The term "cellulosic polymer" is used herein to denote a linear polymer of anhydroglucose. Cellulosic polymers that can be used advantageously in the present dosage formulations include, without limitation, hydroxymethylcellulose, hydroxypropylcellulose, hydroxyethylcellulose, hydroxypropyl methylcellulose, methylcellulose, ethylcellulose, cellulose acetate, cellulose acetate phthalate, cellulose acetate trimellitate, hydroxypropyl methylcellulose phthalate, hydroxypropylcellulose phthalate, cellulose hexahydrophthalate, cellulose acetate hexahydrophthalate, carboxymethylcellulose, carboxymethylcellulose sodium, and microcrystalline cellulose. Preferred cellulosic polymers are alkyl-substituted cellulosic polymers that ultimately dissolve in the GI tract in a predictably delayed manner. Preferred alkyl-substituted cellulose derivatives are those substituted with alkyl groups of 1 to 3 carbon atoms each. Examples are methylcellulose, hydroxymethylcellulose, hydroxyethylcellulose, hydroxypropylcellulose, hydroxypropyl methylcellulose, and carboxymethylcellulose and mixtures thereof. In terms of their viscosities, one class of preferred alkyl-substituted celluloses includes those whose viscosity is within the range of about 50 to about 110,000 centipoise as a 2% aqueous solution at 20°C. Another class includes those whose viscosity is within the range of about 800 to about 6,000 centipoise as a 1 % aqueous solution at 20°C. Particularly preferred alkyl-substituted celluloses are hydroxyethylcellulose and hydroxypropylmethylcellulose. A presently preferred hydroxyethylcellulose is NATRASOL® 250HX NF (National Formulary), available from Aqualon Company, Wilmington, Del., USA.

Suitable polymers also include naturally occurring hydrophilic polymers such as, by way of example, proteins such as collagen, fibronectin, albumins, globulins, fibrinogen, fibrin and thrombin; aminated polysaccharides, particularly the glycosaminoglycans, e.g., hyaluronic acid, chitin, chondroitin sulfate A, B, or C, keratin sulfate, keratosulfate and heparin; guar gum; xanthan gum; carageenan; alginates; pectin; and activated polysaccharides such as dextran and starches.

The aforementioned list of polymers is not exhaustive, and a variety of other synthetic hydrophilic polymers may be used, as will be appreciated by those skilled in the art.

The polymer may include biodegradable segments and blocks, either distributed throughout the polymer's molecular structure or present as a single block, as in a block copolymer. Biodegradable segments are those that degrade so as to break covalent bonds. Typically, biodegradable segments are segments that are hydrolyzed in the presence of water. Biodegradable segments may be composed of small molecular segments such as ester linkages, anhydride linkages, ortho ester linkages, ortho carbonate linkages, amide linkages, phosphonate linkages, etc.

Any polymer or polymers of the matrix may also be crosslinked, with the degree of crosslinking directly affecting the rate of polymer swelling as well as the erosion rate. That is, a polymer having a higher degree of crosslinking will exhibit less swelling and slower erosion than a polymer having a lower degree of crosslinking. Crosslinked polymers may be prepared using the above-mentioned exemplary polymers using conventional crosslinking procedures (e.g., chemical crosslinking with an added crosslinking agent, photolytically induced crosslinking, etc.), or the polymers may be obtained commercially in crosslinked form.

The water-swellable polymers can be used individually or in combination. Certain combinations will often provide a more controlled release of the drug than their components when used individually. Examples include, but are not limited to, the following: a cellulosic polymer combined with a gum, such as hydroxyethylcellulose or hydroxypropylcellulose combined with xanthan gum; a polyalkylene oxide combined with a gum, such as poly(ethylene oxide) combined with xanthan gum; and a polyalkylene oxide combined with a cellulosic polymer, such as poly(ethylene oxide) combined with hydroxyethylcellulose or hydroxypropylcellulose.

Combinations of different poly(ethylene oxide)s are also encompassed, with polymers of different molecular weights contributing to different dosage formulation characteristics. For example, a very high molecular weight poly(ethylene oxide) such as Polyox® 303 (with a number average molecular weight of 7 million) or Polyox® Coag (with a number average molecular weight of 5 million) may be used to significantly enhance diffusion relative to disintegration release by providing high swelling as well as tablet integrity. Incorporating a lower molecular weight poly(ethylene oxide) such as Polyox® WSR N-60K (number average molecular weight approximately 2 million) with Polyox® 303 and/or Polyox® Coag increases disintegration rate relative to diffusion rate, as the lower molecular weight polymer reduces swelling and acts as an effective tablet disintegrant. Incorporating an even lower molecular weight poly(ethylene oxide) such as Polyox® WSR N-80 (number average molecular weight approximately 200,000) further increases disintegration rate.

The hydrophilicity and water swellability of these polymers cause the drug-containing matrices to swell in size in the gastric cavity due to ingress of water in order to achieve a size that will be retained in the stomach when introduced during the fed mode. These qualities also cause the matrices to become slippery, which provides resistance to peristalsis and further promotes their retention in the stomach. The release rate of a drug from the matrix is primarily dependent upon the rate of water imbibition and the rate at which the drug dissolves and diffuses from the swollen polymer, which in turn is related to the solubility and dissolution rate of the drug, the drug particle size and the drug concentration in the matrix. The insoluble drug is released from these systems primarily by erosion.

The amount of polymer relative to the drug can vary, depending on the drug release rate desired and on the polymer, its molecular weight, and excipients that may be present in the formulation. The amount of polymer will be sufficient however to retain at least about 40% of the drug within the matrix one hour after ingestion (or immersion in the gastric fluid). Preferably, the amount of polymer is such that at least 50% of the drug remains in the matrix one hour after ingestion. More preferably, at least 60%, and most preferably at least 80%, of the drug remains in the matrix one hour after ingestion. In all cases, however, substantially all of the drug will be released from the matrix within about eight hours, and preferably within about six hours, after ingestion, "substantially all" meaning at least 85%, preferably at least 90%.

Higher molecular weight polymers may be preferred to provide a desired extended release profile using the present dosage formulations. Suitable molecular weights are generally in the range of about 5,000 to about 20,000,000. For sparingly soluble drugs, the polymers have molecular weights preferably in the range of about 5,000 to about 8,000,000, more preferably in the range of about 10,000 to about 5,000,000. For water-soluble drugs, the polymers preferably have molecular weights of at least about 10,000, but the molecular weight used will vary with the selected polymer. For example, for hydroxypropyl methylcellulose, the minimum molecular weight may be as low as 10,000, while for poly(ethylene oxides) the molecular weight may be far higher, on the order of 2,000,000 or more.

The swellable polymer used as the controlled-release dosage formulation carrier is preferably present in an amount to obtain a weight gain level of the dosage formulation from about 1 to 90 percent, or about 2 to 50 percent, or more preferably about 2 to 25 percent. The swellable polymer used as the controlled-release dosage formulation carrier is also preferably present at from about 5 to 85 weight percent (wt.%), or about 15 to 80 weight percent (wt.%), or more preferably about 20 to 75 weight percent (wt.%).

As would be understood, the formulations of the present invention further comprise at least one therapeutic agent. The terms "drug," and "therapeutic agent" are all used interchangeably in this application and mean a compound that exerts a therapeutically beneficial effect on a patient. The term is intended to encompass various forms of the therapeutic agents including but not limited to a therapeutic compound as well as prodrugs, solvates, hydrates, co-crystals, coprecipitate, molecular complexes and pharmaceutically acceptable salts and derivatives of the therapeutic compound.

Therapeutic agents that may be administered in the drug delivery formulations of the present invention include, but are not limited to, HCV protease inhibitors, adrenergic receptor agonists and antagonists; muscarinic receptor agonists and antagonists; anticholinesterase agents; neuromuscular blocking agents; ganglionic blocking and stimulating agents; sympathomimetic drugs; serotonin receptor agonists and antagonists; central nervous system active drugs such as psychotropic drugs, antipsychotic drugs, antianxiety drugs, antidepressants, antimanic drugs, anesthetics, hypnotics, sedatives, hallucinogenic drugs and antihallucinogenic drugs; antiepileptic drugs; antimigraine drugs; drugs for treatment of Parkinson's, Alzheimer's and Huntington's disease; analgesics; antitussive agents; antihistaminic drugs; bradykinin receptor antagonists; antipyretic agents; antiinflammatory agents; NSAIDs; diuretics; inhibitors of NaCl transport; vasopressin receptor agonists and antagonists; ACE inhibitors; angiotensin II receptor antagonists; renin inhibitors; calcium channel blockers; β-adrenergic receptor antagonists; antiplatelet agents; antithrombic agents; antihypertensive agents; vasodialators; phosphodiesterase inhibitors; antiarrhythmic drugs; HMG CoA reductase inhibitors; ATPase inhibitors; prostaglandins and prostaglandin analogs; laxatives; antidiarrheal agents; antiemetic agents; prokinetic agents; antiparasitic agents such as antimalarial agents, antibacterial agents, drugs for treatment of protozoal infections and antihelmintic drugs; antimicrobial drugs such as sulfonamides, quinolones, .beta.-lactam antibiotics, aminoglycosides, tetracyclines, chloramphenicol and erythromycin; drugs for treatment of tuberculosis, drugs for treatment of leprosy; antifungal agents; antiviral agents; immunomodulators; hematopoietic agents; growth factors; vitamins; minerals; anticoagulants; hormones and hormone antagonists such as antithyroid drugs, estrogens, progestins, androgens, adrenocortical steroids and adrenocortical steroid inhibitors; insulin; hypoglycemic agents; calcium resorption inhibitors; glucocorticoids; retinoids and heavy-metal antagonists. The therapeutic agent in the formulations may be in the form of a pharmaceutically acceptable salt, prodrug or derivative of the agent that exerts a therapeutic effect in the patient upon administration.

As described in detail below, in specific purely exemplary illustrative embodiments of the invention the therapeutic agent is an HCV protease inhibitor, Metformin or Niacin. Such HCV protease inhibitor pharmaceutical formulations can be used for treating or ameliorating one or more symptoms of HCV infection or treating a disorder associated with HCV. Metformin is an oral anti-diabetic drug, and Metformin pharmaceutical formulations of the invention can be used for treating or ameliorating one or more symptoms of type-2 diabetes. Niacin is an effective antilipidemic drug, and Niacin pharmaceutical formulations of the invention can be used for treating or ameliorating one or more symptoms of hyperlipidemia or dislipidemia

Typical HCV protease inhibitors for use in formulation of the present invention include those described below in Formulae I to XXVIII:

### Formula I, as disclosed in US Patent 7,012,066:

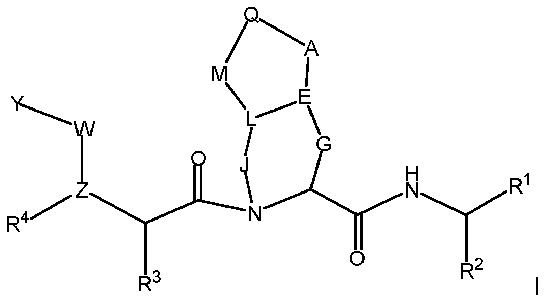

or a pharmaceutically acceptable salt, solvate, or ester thereof;
wherein in Formula I:
Y is selected from the group consisting of the following moieties: alkyl, alkyl-aryl, heteroalkyl, heteroaryl, aryl-heteroaryl, alkyl-heteroaryl, cycloalkyl, alkyloxy, alkyl-aryloxy, aryloxy, heteroaryloxy, heterocycloalkyloxy, cycloalkyloxy, alkylamino, arylamino, alkyl-arylamino, arylamino, heteroarylamino, cycloalkylamino and heterocycloalkylamino, with the proviso that Y maybe optionally substituted with X¹¹ or X¹²;
X¹¹ is alkyl, alkenyl, alkynyl, cycloalkyl, cycloalkyl-alkyl, heterocyclyl, heterocyclylalkyl, aryl, alkylaryl, arylalkyl, heteroaryl, alkylheteroaryl, or heteroarylalkyl, with the proviso that X¹¹ may be additionally optionally substituted with X¹²;
X¹² is hydroxy, alkoxy, aryloxy, thio, alkylthio, arylthio, amino, alkylamino, arylamino, alkylsulfonyl, arylsulfonyl, alkylsulfonamido, arylsulfonamido, carboxy, carbalkoxy, carboxamido, alkoxycarbonylamino, alkoxycarbonyloxy, alkylureido, arylureido, halogen, cyano, or nitro, with the proviso that said alkyl, alkoxy, and aryl may be additionally optionally substituted with moieties independently selected from X¹²;
R¹ is COR⁵, wherein R⁵ is COR⁷ wherein R⁷ is NHR⁹, wherein R⁹ is selected from the group consisting of H, alkyl, aryl, heteroalkyl, heteroaryl, cycloalkyl, cycloalkyl, arylalkyl, heteroarylalkyl, [CH(R^{1'})]ₚCOOR¹¹,[CH(R^{1'})]ₚCONR¹²R¹³,[CH(R^{1'})]ₚSO₂R¹¹,[CH(R^{1'})]ₚCOR¹¹,[CH( R^{1'})]pCH(OH)R¹¹, CH(R^{1'})CONHCH(R²)COOR¹¹, CH(R^{1'})CONHCH(R^{2'})CONR¹²R^{1 3}, CH(R^{1'})CONHCH(R²)R', CH(R^{1'})CONHCH(R^{2'})CONHCH(R^{3'})COOR¹¹, CH(R^{1'})C ONHCH(R²)CONHCH(R³)CONR¹²R¹³, CH(R^{1'})CONHCH(R^{2'})CONHCH(R^{3'})CON HCH(R^{4'})COOR¹¹, CH(R^{1'})CONHCH(R^{2'})CONHCH(R^{3'})CONHCH(R^{4'})CONR¹²R¹³, CH(R^{1'})CONHCH(R^{2'})CONHCH(R^{3'})CONHCH(R^{4'})CONHCH(R^{5'})COOR¹¹andCH( R^{1'})CONHCH(R^{2'})CONHCH(R^{3'})CONHCH(R^{4'})CONHCH(R^{5'}) CONR¹²R¹³, wherein R^{1'}, R^{2'}, R^{3'}, R^{4'}, R^{5'}, R¹¹, R¹², R¹³, and R' are independently selected from the group consisting of H, alkyl, aryl, heteroalkyl, heteroaryl, cycloalkyl, alkyl-aryl, alkyl-heteroaryl, aryl-alkyl and heteroaralkyl;
Z is selected from O, N, CH or CR;
W maybe present or absent, and if W is present, W is selected from C=O, C=S, C(=N-CN), or SO₂;
Q maybe present or absent, and when Q is present, Q is CH, N, P, (CH₂)ₚ, (CHR)ₚ, (CRR')ₚ, O, NR, S, or SO₂; and when Q is absent, M may be present or absent; when Q and M are absent, A is directly linked to L;
A is O, CH₂, (CHR) ₚ , (CHR-CHR') ₚ , (CRR') ₚ, NR, S, SO₂ or a bond;
E is CH, N, CR, or a double bond towards A, L or G;
G may be present or absent, and when G is present, G is (CH₂)ₚ, (CHR) ₚ, or (CRR')ₚ; and when G is absent, J is present and E is directly connected to the carbon atom in Formula I as G is linked to;
J may be present or absent, and when J is present, J is (CH2)ₚ, (CHR)ₚ, or (CRR')ₚ, SO₂, NH, NR or O; and when J is absent, G is present and E is directly linked to N shown in Formula I as linked to J;
L may be present or absent, and when L is present, L is CH, CR, O, S or NR; and when L is absent, then M may be present or absent; and if M is present with L being absent, then M is directly and independently linked to E, and J is directly and independently linked to E;
M may be present or absent, and when M is present, M is O, NR, S, SO₂, (CH2)ₚ, (CHR)ₚ (CHR-CHR')ₚ, or (CRR')ₚ;
p is a number from 0 to 6; and
R, R', R², R³ and R⁴ are independently selected from the group consisting of H; C1-C10 alkyl; C2-C10 alkenyl; C3-C8 cycloalkyl; C3-C8 heterocycloalkyl, alkoxy, aryloxy, alkylthio, arylthio, amino, amido, ester, carboxylic acid, carbamate, urea, ketone, aldehyde, cyano, nitro, halogen; (cycloalkyl)alkyl and (heterocycloalkyl)alkyl, wherein said cycloalkyl is made of three to eight carbon atoms, and zero to six oxygen, nitrogen, sulfur, or phosphorus atoms, and said alkyl is of one to six carbon atoms; aryl; heteroaryl; alkyl-aryl; and alkyl-heteroaryl;
wherein said alkyl, heteroalkyl, alkenyl, heteroalkenyl, aryl, heteroaryl, cycloalkyl and heterocycloalkyl moieties may be optionally and chemically-suitably substituted, with said term "substituted" referring to optional and chemically-suitable substitution with one or more moieties selected from the group consisting of alkyl, alkenyl, alkynyl, aryl, aralkyl, cycloalkyl, heterocyclic, halogen, hydroxy, thio, alkoxy, aryloxy, alkylthio, arylthio, amino, amido, ester, carboxylic acid, carbamate, urea, ketone, aldehyde, cyano, nitro, sulfonamido, sulfoxide, sulfone, sulfonyl urea, hydrazide, and hydroxamate;
further wherein said unit N-C-G-E-L-J-N represents a five-membered or six-membered cyclic ring structure with the proviso that when said unit N-C-G-EL-J-N represents a five-membered cyclic ring structure, or when the bicyclic ring structure in Formula I comprising N, C, G, E, L, J, N, A, Q, and M represents a five-membered cyclic ring structure, then said five-membered cyclic ring structure lacks a carbonyl group as part of the cyclic ring;

### Formula II, as disclosed in U.S. Patent No. 6,800,434:

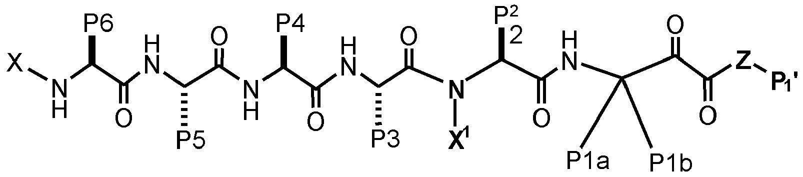

or a pharmaceutically acceptable salt, solvate, or ester thereof; wherein in Formula II:
Z is NH;
X is alkylsulfonyl, heterocyclylsulfonyl, heterocyclylalkylsulfonyl, arylsulfonyl, heteroarylsulfonyl, alkylcarbonyl, heterocyclylcarbonyl, heterocyclylalkylcarbonyl, arylcarbonyl, heteroarylcarbonyl, alkoxycarbonyl, heterocyclyloxycarbonyl, aryloxycarbonyl, heteroaryloxycarbonyl, alkyaminocarbonyl, heterocyclylaminocarbonyl, arylaminocarbonyl, or heteroarylaminocarbonyl moiety, with the proviso that X may be additionally optionally substituted with R¹² or R¹³;
X¹ is H; C₁-C₄ straight chain alkyl; C₁-C₄ branched alkyl or ; CH₂-aryl (substituted or unsubstituted);
R¹² is alkyl, alkenyl, alkynyl, cycloalkyl, cycloalkyl-alkyl, heterocyclyl, heterocyclylalkyl, aryl, alkylaryl, arylalkyl, heteroaryl, alkylheteroaryl, or heteroarylalkyl moiety, with the proviso that R¹² may be additionally optionally substituted with R¹³.
R¹³ is hydroxy, alkoxy, aryloxy, thio, alkylthio, arylthio, amino, alkylamino, arylamino, alkylsulfonyl, arylsulfonyl, alkylsulfonamido, arylsulfonamido, carboxy, carbalkoxy, carboxamido, alkoxycarbonylamino, alkoxycarbonyloxy, alkylureido, arylureido, halogen, cyano, or nitro moiety, with the proviso that the alkyl, alkoxy, and aryl may be additionally optionally substituted with moieties independently selected from R¹³.
P1 a, P1b, P2, P3, P4, P5, and P6 are independently: H; C1-C10 straight or branched chain alkyl; C2-C10 straight or branched chain alkenyl; C3-C8 cycloalkyl, C3-C8 heterocyclic; (cycloalkyl)alkyl or (heterocyclyl)alkyl , wherein said cycloalkyl is made up of 3 to 8 carbon atoms, and zero to 6 oxygen, nitrogen, sulfur, or phosphorus atoms, and said alkyl is of 1 to 6 carbon atoms; aryl, heteroaryl, arylalkyl, or heteroarylalkyl, wherein said alkyl is of 1 to 6 carbon atoms;
wherein said alkyl, alkenyl, cycloalkyl, heterocyclyl; (cycloalkyl)alkyl and (heterocyclyl)alkyl moieties may be optionally substituted with R¹³, and further wherein said P1 a and P1 b may optionally be joined to each other to form a spirocyclic or spiroheterocyclic ring, with said spirocyclic or spiroheterocyclic ring containing zero to six oxygen, nitrogen, sulfur, or phosphorus atoms, and may be additionally optionally substituted with R¹³; and
P1' is H, alkyl, alkenyl, alkynyl, cycloalkyl, cycloalkyl-alkyl, heterocyclyl, heterocyclyl-alkyl, aryl, aryl-alkyl, heteroaryl, or heteroaryl-alkyl; with the proviso that said P1' may be additionally optionally substituted with R¹³;

### Formula III, as disclosed in US Patent Publication 2002/0160962:

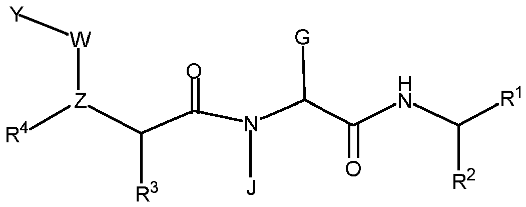

or a pharmaceutically acceptable salt, solvate, or ester thereof; wherein in Formula III:
G is carbonyl;
J and Y may be the same or different and are independently selected from the group consisting of the moieties: H, alkyl, alkyl-aryl, heteroalkyl, heteroaryl, aryl-heteroaryl, alkyl-heteroaryl, cycloalkyl, alkyloxy, alkyl-aryloxy, aryloxy, heteroaryloxy, heterocycloalkyloxy, cycloalkyloxy, alkylamino, arylamino, alkyl-arylamino, arylamino, heteroarylamino, cycloalkylamino and heterocycloalkylamino, with the proviso that Y maybe additionally optionally substituted with X¹¹ or X¹²;
X¹¹ is selected from the group consisting of alkyl, alkenyl, alkynyl, cycloalkyl, cycloalkyl-alkyl, heterocyclyl, heterocyclylalkyl, aryl, alkylaryl, arylalkyl, heteroaryl, alkylheteroaryl, or heteroarylalkyl moiety, with the proviso that X¹¹ may be additionally optionally substituted with X¹²;
X¹² is hydroxy, alkoxy, aryloxy, thio, alkylthio, arylthio, amino, alkylamino, arylamino, alkylsulfonyl, arylsulfonyl, alkylsulfonamido, arylsulfonamido, carboxy, carbalkoxy, carboxamido, alkoxycarbonylamino, alkoxycarbonyloxy, alkylureido, arylureido, halogen, cyano, or nitro, with the proviso that said alkyl, alkoxy, and aryl may be additionally optionally substituted with moieties independently selected from X¹²;
R¹ is COR⁵ or C(OR)₂, wherein R⁵ is selected from the group consisting of H, OH, OR⁸, NR⁹R¹⁰, CF₃, C₂F₅, C₃F₇, CF₂R⁶, R⁶ and COR⁷ wherein R⁷ is selected from the group consisting of H, OH, OR⁸, CHR⁹R¹⁰, and NR⁹R¹⁰, wherein R⁶, R⁸, R⁹ and R¹⁰ may be the same or different and are independently selected from the group consisting of H, alkyl, aryl, heteroalkyl, heteroaryl, cycloalkyl, cycloalkyl, arylalkyl, heteroarylalkyl,
CH(R^{1'})COOR¹¹,CH(R^{1'})CONR¹²R¹³,CH(R^{1'})CONHCH(R^{2'})COOR¹¹, CH(R^{1'})CONHCH(R^{2'})CONR¹²R¹³,CH(R^{1'})CONHCH(R^{2'})R',CH(R^{1'})CONHCH(R^{2'} )CONHCH(R^{3'})COOR¹¹,CH(R^{1'})CONHCH(R^{2'})CONHCH(R^{3'})CONR¹²R¹³, CH(R^{1'})CONHCH(R^{2'})CONHCH(R^{3'})CONHCH(R^{4'})COOR¹¹,CH(R¹)CONHCH(R² )CONHCH(R^{3'})CONHCH(R^{4'})CONR¹²R¹³, CH(R^{1'})CONHCH(R²)CONHCH(R^{3'})C ONHCH(R^{4'})CONHCH(R⁵)COOR¹¹,andCH(R^{1'})CONHCH(R²)CONHCH(R^{3'})CO NHCH(R^{4'})CONHCH(R^{5'}) CONR¹²R¹³, wherein R^{1'}, R^{2'}, R^{3'}, R^{4'}, R^{5'}, R¹¹, R¹², R¹³ , and R' may be the same or different and are independently selected from a group consisting of H, alkyl, aryl, heteroalkyl, heteroaryl, cycloalkyl, alkyl-aryl, alkyl-heteroaryl, aryl-alkyl and heteroaralkyl;
Z is selected from O, N, or CH;
W maybe present or absent, and if W is present, W is selected from C=O, C=S, or SO₂; and
R, R', R², R³ and R⁴ are independently selected from the group consisting of H; C1-C10 alkyl; C2-C10 alkenyl; C3-C8 cycloalkyl; C3-C8 heterocycloalkyl, alkoxy, aryloxy, alkylthio, arylthio, amino, amido, ester, carboxylic acid, carbamate, urea, ketone, aldehyde, cyano, nitro; oxygen, nitrogen, sulfur, or phosphorus atoms (with said oxygen, nitrogen, sulfur, or phosphorus atoms numbering zero to six); (cycloalkyl)alkyl and (heterocycloalkyl)alkyl, wherein said cycloalkyl is made of three to eight carbon atoms, and zero to six oxygen, nitrogen, sulfur, or phosphorus atoms, and said alkyl is of one to six carbon atoms; aryl; heteroaryl; alkyl-aryl; and alkyl-heteroaryl;
wherein said alkyl, heteroalkyl, alkenyl, heteroalkenyl, aryl, heteroaryl, cycloalkyl and heterocycloalkyl moieties may be optionally substituted, with said term "substituted" referring to optional and chemically-suitable substitution with one or more moieties selected from the group consisting of alkyl, alkenyl, alkynyl, aryl, aralkyl, cycloalkyl, heterocyclic, halogen, hydroxy, thio, alkoxy, aryloxy, alkylthio, arylthio, amino, amido, ester, carboxylic acid, carbamate, urea, ketone, aldehyde, cyano, nitro, sulfonamide, sulfoxide, sulfone, sulfonylurea, hydrazide, and hydroxamate;

### Formula IV, as disclosed in US Patent 6,894,072 :

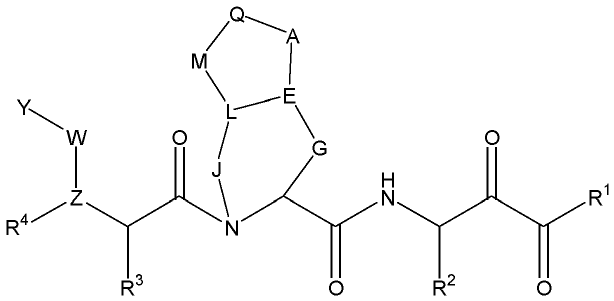

or a pharmaceutically acceptable salt, solvate, or ester thereof; wherein in Formula IV:
Y is selected from the group consisting of the following moieties: alkyl, alkyl-aryl, heteroalkyl, heteroaryl, aryl-heteroaryl, alkyl-heteroaryl, cycloalkyl, alkyloxy, alkyl-aryloxy, aryloxy, heteroaryloxy, heterocycloalkyloxy, cycloalkyloxy, alkylamino, arylamino, alkyl-arylamino, arylamino, heteroarylamino, cycloalkylamino and heterocycloalkylamino, with the proviso that Y maybe optionally substituted with X¹¹ or X¹²;
X¹¹ is alkyl, alkenyl, alkynyl, cycloalkyl, cycloalkyl-alkyl, heterocyclyl, heterocyclylalkyl, aryl, alkylaryl, arylalkyl, heteroaryl, alkylheteroaryl, or heteroarylalkyl, with the proviso that X¹¹ may be additionally optionally substituted with X¹²;
X¹² is hydroxy, alkoxy, aryloxy, thio, alkylthio, arylthio, amino, alkylamino, arylamino, alkylsulfonyl, arylsulfonyl, alkylsulfonamido, arylsulfonamido, carboxyl, carbalkoxy, carboxamido, alkoxycarbonylamino, alkoxycarbonyloxy, alkylureido, arylureido, halogen, cyano, or nitro, with the proviso that said alkyl, alkoxy, and aryl may be additionally optionally substituted with moieties independently selected from X¹²;
R¹ is selected from the following structures: 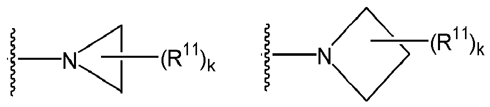 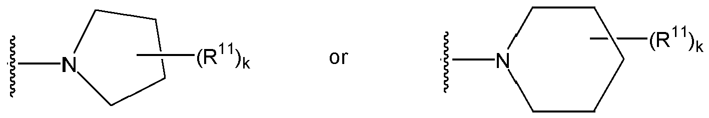
wherein k is a number from 0 to 5, which can be the same or different, R¹¹ denotes optional substituents, with each of said substituents being independently selected from the group consisting of alkyl, alkenyl, alkynyl, aryl, cycloalkyl, alkyl-aryl, heteroalkyl, heteroaryl, aryl-heteroaryl, alkyl-heteroaryl, alkyloxy, alkyl-aryloxy, aryloxy, heteroaryloxy, heterocycloalkyloxy, cycloalkyloxy, alkylamino, arylamino, alkyl-arylamino, arylamino, heteroarylamino, cycloalkylamino, heterocycloalkylamino, hydroxy, thio, alkylthio, arylthio, amino, alkylsulfonyl, arylsulfonyl, alkylsulfonamido, arylsulfonamido, carboxyl, carbalkoxy, carboxamido, alkoxycarbonylamino, alkoxycarbonyloxy, alkylureido, arylureido, halogen, cyano, and nitro, with the proviso that R¹¹ (when R¹¹ ≠ H) maybe optionally substituted with X¹¹ or X¹²;
Z is selected from O, N, CH or CR;
W may be present or absent, and if W is present, W is selected from C=O, C=S, C(=N-CN), or S(O₂);
Q may be present or absent, and when Q is present, Q is CH, N, P, (CH₂)ₚ, (CHR)ₚ, (CRR')ₚ, O, N(R), S, or S(O₂); and when Q is absent, M may be present or absent; when Q and M are absent, A is directly linked to L; A is O, CH₂, (CHR) ₚ , (CHR-CHR') ₚ , (CRR') ₚ, N(R), S, S(O₂) or a bond;
E is CH, N, CR, or a double bond towards A, L or G;
G may be present or absent, and when G is present, G is (CH₂)ₚ, (CHR) ₚ, or (CRR')ₚ; and when G is absent, J is present and E is directly connected to the carbon atom in Formula I as G is linked to;
J may be present or absent, and when J is present, J is (CH₂)ₚ, (CHR) ₚ, or (CRR')ₚ, S(O₂), NH, N(R) or O; and when J is absent, G is present and E is directly linked to N shown in Formula I as linked to J;
L may be present or absent, and when L is present, L is CH, C(R), O, S or N(R); and when L is absent, then M may be present or absent; and if M is present with L being absent, then M is directly and independently linked to E, and J is directly and independently linked to E;
M may be present or absent, and when M is present, M is O, N(R), S, S(O₂), (CH₂)ₚ, (CHR)ₚ (CHR-CHR')ₚ, or (CRR')ₚ;
p is a number from 0 to 6; and
R, R', R², R³ and R⁴ can be the same or different, each being independently selected from the group consisting of H; C₁-C₁₀ alkyl; C₂-C₁₀ alkenyl; C₃-C₈ cycloalkyl; C₃-C₈ heterocycloalkyl, alkoxy, aryloxy, alkylthio, arylthio, amino, amido, ester, carboxylic acid, carbamate, urea, ketone, aldehyde, cyano, nitro, halogen, (cycloalkyl)alkyl and (heterocycloalkyl)alkyl, wherein said cycloalkyl is made of three to eight carbon atoms, and zero to six oxygen, nitrogen, sulfur, or phosphorus atoms, and said alkyl is of one to six carbon atoms; aryl; heteroaryl; alkyl-aryl; and alkyl-heteroaryl;
wherein said alkyl, heteroalkyl, alkenyl, heteroalkenyl, aryl, heteroaryl, cycloalkyl and heterocycloalkyl moieties may be optionally substituted, with said term "substituted" referring to substitution with one or more moieties which can be the same or different, each being independently selected from the group consisting of alkyl, alkenyl, alkynyl, aryl, aralkyl, cycloalkyl, heterocyclic, halogen, hydroxy, thio, alkoxy, aryloxy, alkylthio, arylthio, amino, amido, ester, carboxylic acid, carbamate, urea, ketone, aldehyde, cyano, nitro, sulfonamido, sulfoxide, sulfone, sulfonyl urea, hydrazide, and hydroxamate;
further wherein said unit N-C-G-E-L-J-N represents a five-membered cyclic ring structure or six-membered cyclic ring structure with the proviso that when said unit N-C-G-E-L-J-N represents a five-membered cyclic ring structure, or when the bicyclic ring structure in Formula I comprising N, C, G, E, L, J, N, A, Q, and M represents a five-membered cyclic ring structure, then said five-membered cyclic ring structure lacks a carbonyl group as part of said five-membered cyclic ring;

### Formula V, as disclosed in U.S. Patent Publication 2005/0119168:

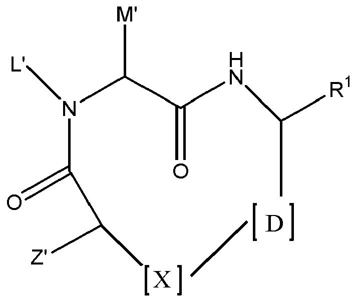

or a pharmaceutically acceptable salt, solvate, or ester thereof; wherein in Formula V:
(1) R¹ is -C(O)R⁵ or -B(OR)₂;
(2) R⁵ is H, -OH, -OR⁸, -NR⁹R¹⁰, -C(O)OR⁸, -C(O)NR⁹R¹⁰ , -CF₃, -C₂F₅, C₃F₇, -CF₂R⁶, -R⁶, -C(O)R⁷ or NR⁷SO₂R⁸;
(3) R⁷ is H, -OH, -OR⁸, or -CHR⁹R¹⁰;
(4) R⁶, R⁸, R⁹ and R¹⁰ are independently selected from the group consisting of H: alkyl, alkenyl, aryl, heteroalkyl, heteroaryl, cycloalkyl, arylalkyl, heteroarylalkyl, R¹⁴, -CH(R^{1'})CH(R^{1'})C(O)OR¹¹,[CH(R^{1'})]ₚC(O)OR¹¹,-[CH(R^{1'})]ₚC(O)NR¹²R¹³,-[CH(R^{1'})] ₚS(O₂)R¹¹,-[CH(R^{1'})]ₚC(O)R¹¹,-[CH(R^{1'})]ₚS(O₂)NR¹²R¹³, OCH(R^{1'})C(O)N(H)CH(R^{2'})(R'), CH(R^{1'})CH(R^{1'})C(O)NR¹²R¹³, -CH(R^{1'})CH(R^{1'})S(O₂)R¹¹, - CH(R^{1'})CH(R^{1'})S(O₂)NR¹²R¹³, -CH(R^{1'})CH(R^{1'})C(O)R¹¹, -[CH(R^{1'})]ₚCH(OH)R¹¹, - CH(R^{1'})C(O)N(H)CH(R^{2'})C(O)OR¹¹, C(O)N(H)CH(R^{2'})C(O)OR¹¹,-C(O)N(H)CH(R^{2'})C(O)R¹¹, CH(R¹)C(O)N(H)CH(R^{2'}) C(O)NR¹²R¹³,-CH(R^{1'})C(O)N(H)CH(R^{2'})R', CH(R^{1'})C(O)N(H)CH(R^{2'})C(O)N(H) CH(R^{3'})C(O)OR¹¹,CH(R^{1'})C(O)N(H)CH(R^{2'})C(O)CH(R^{3'})NR¹²R¹³,CH(R^{1'})C(O)N(H )CH(R^{2'})C(O)N(H)CH(R^{3'})C(O)NR¹²R¹³,CH(R^{1'})C(O)N(H)CH(R^{2'})C(O)N(H)CH(R^{3'} )C(O)N(H)CH(R^{4'})C(O)OR¹¹, H(R^{1'})C(O)N(H)CH(R^{2'})C(O)N(H)CH(R^{3'})C(O)N(H)CH(R^{4'})C(O)NR¹²R¹³, CH(R^{1'})C(O)N(H)CH(R^{2'} )C(O)N(H)CH(R^{3'})C(O)N(H)CH(R^{4'})C(O)N(H)CH(R^{5'})C(O)OR¹¹, andCH(R^{1'})C(O)N(H)CH(R^{2'})C(O)N(H)CH(R^{3'})C(O)N(H)CH(R^{4'})C(O)N(H)CH(R^{5'}) C(O)NR¹²R¹³;
   wherein R^{1'}, R^{2'}, R^{3'}, R^{4'}, R^{5'}, R¹¹, R¹² and R¹³ can be the same or different, each being independently selected from the group consisting of: H, halogen, alkyl, aryl, heteroalkyl, heteroaryl, cycloalkyl, alkoxy, aryloxy, alkenyl, alkynyl, alkyl-aryl, alkyl-heteroaryl, heterocycloalkyl, aryl-alkyl and heteroaralkyl; or
   R¹² and R¹³ are linked together wherein the combination is cycloalkyl, heterocycloalkyl, ary or heteroaryl;
   R¹⁴ is present or not and if present is selected from the group consisting of: H, alkyl, aryl, heteroalkyl, heteroaryl, cycloalkyl, alkyl-aryl, allyl, alkyl-heteroaryl, alkoxy, aryl-alkyl, alkenyl, alkynyl and heteroaralkyl;
(5) R and R' are present or not and if present can be the same or different, each being independently selected from the group consisting of: H, OH, C₁-C₁₀ alkyl, C₂-C₁₀ alkenyl, C₃-C₈ cycloalkyl, C₃-C₈ heterocycloalkyl, alkoxy, aryloxy, alkylthio, arylthio, alkylamino, arylamino, amino, amido, arylthioamino, arylcarbonylamino, arylaminocarboxy, alkylaminocarboxy, heteroalkyl, alkenyl, alkynyl, (aryl)alkyl, heteroarylalkyl, ester, carboxylic acid, carbamate, urea, ketone, aldehyde, cyano, nitro, halogen, (cycloalkyl)alkyl, aryl, heteroaryl, (alkyl)aryl, alkylheteroaryl, alkyl-heteroaryl and (heterocycloalkyl)alkyl, wherein said cycloalkyl is made of three to eight carbon atoms, and zero to six oxygen, nitrogen, sulfur, or phosphorus atoms, and said alkyl is of one to six carbon atoms;
(6) L' is H, OH, alkyl, heteroalkyl, aryl, heteroaryl, cycloalkyl, or heterocyclyl;
(7) M' is H, alkyl, heteroalkyl, aryl, heteroaryl, cycloalkyl, arylalkyl, heterocyclyl or an amino acid side chain;
   or L' and M' are linked together to form a ring structure wherein the portion of structural Formula 1 represented by: 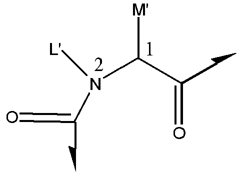 and wherein structural Formula 2 is represented by: 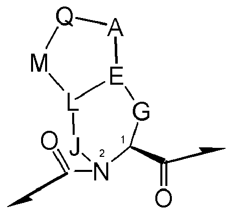 wherein in Formula 2:
   E is present or absent and if present is C, CH, N or C(R);
   J is present or absent, and when J is present, J is (CH₂)ₚ, (CHR-CHR')ₚ, (CHR)ₚ, (CRR')ₚ, S(O₂), N(H), N(R) or O; when J is absent and G is present, L is directly linked to the nitrogen atom marked position 2;
   p is a number from 0 to 6;
   L is present or absent, and when L is present, L is C(H) or C(R); when L is absent, M is present or absent; if M is present with L being absent, then M is directly and independently linked to E, and J is directly and independently linked to E;
   G is present or absent, and when G is present, G is (CH₂)ₚ, (CHR)ₚ, (CHR-CHR')ₚ or (CRR')ₚ; when G is absent, J is present and E is directly connected to the carbon atom marked position 1;
   Q is present or absent, and when Q is present, Q is NR, PR, (CR=CR), (CH₂)ₚ,
   (CHR)ₚ , (CRR')ₚ, (CHR-CHR')ₚ, O, NR, S, SO, or SO₂; when Q is absent, M is (i) either directly linked to A or (ii) an independent substituent on L, said independent substituent bing selected from -OR, -CH(R)(R'), S(O)₀₋₂R or -NRR' or (iii) absent; when both Q and M are absent, A is either directly linked to L, or A is an independent substituent on E, said independent substituent bing selected from -OR, -CH(R)(R'), S(O)₀₋₂R or -NRR' or A is absent;
   A is present or absent and if present A is O, O(R), (CH₂)ₚ, (CHR)ₚ, (CHR-CHR')ₚ , (CRR')ₚ, N(R), NRR', S, S(O₂), -OR, CH(R)(R') or NRR'; or A is linked to M to form an alicyclic, aliphatic or heteroalicyclic bridge;
   M is present or absent, and when M is present, M is halogen, O, OR, N(R), S, S(O₂), (CH₂)ₚ, (CHR)ₚ (CHR-CHR')ₚ, or (CRR')ₚ; or M is linked to A to form an alicyclic, aliphatic or heteroalicyclic bridge;
(8) Z' is represented by the structural Formula 3: 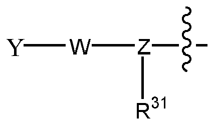 wherein in Formula 3:
   Y is selected from the group consisting of: H, aryl, alkyl, alkyl-aryl, heteroalkyl, heteroaryl, aryl-heteroaryl, alkyl-heteroaryl, cycloalkyl, alkyloxy, alkyl-aryloxy, aryloxy, heteroaryloxy, heterocycloalkyloxy, heteroalkyl-heteroaryl, heteroalkyl-heterocycloalkyl, cycloalkyloxy, alkylamino, arylamino, alkyl-arylamino, arylamino, heteroarylamino, cycloalkylamino and heterocycloalkylamino, and Y is unsubstituted or optionally substituted with one or two substituents which are the same or different and are independently selected from X¹¹ or X¹²;
   X¹¹ is alkyl, alkenyl, alkynyl, cycloalkyl, cycloalkyl-alkyl, heterocyclyl, heterocyclylalkyl, aryl, alkylaryl, arylalkyl, heteroaryl, alkylheteroaryl, or heteroarylalkyl, and X¹¹ is unsubstituted or optionally substituted with one or more of X¹² moieties which are the same or different and are independently selected;
   X¹² is hydroxy, alkoxy, alkyl, alkenyl, alkynyl, aryl, aryloxy, thio, alkylthio, arylthio, amino, alkylamino, arylamino, alkylsulfonyl, arylsulfonyl, alkylsulfonamido, arylsulfonamido, carboxy, carbalkoxy, carboxamido, alkylcarbonyl, arylcarbonyl, heteroalkylcarbonyl, heteroarylcarbonyl, sulfonylurea, cycloalkylsulfonamido, heteroaryl-cycloalkylsulfonamido, heteroaryl-sulfonamido, alkoxycarbonylamino, alkoxycarbonyloxy, alkylureido, arylureido, halogen, cyano, or nitro, and said alkyl, alkoxy, and aryl are unsubstituted or optionally independently substituted with one or more moieties which are the same or different and are independently selected from alkyl, alkenyl, alkynyl, cycloalkyl, cycloalkyl-alkyl, heterocyclyl, heterocyclylalkyl, aryl, alkylaryl, arylalkyl, heteroaryl, alkylheteroaryl, or heteroarylalkyl;
   Z is O, N, C(H) or C(R);
   R³¹ is H, hydroxyl, aryl, alkyl, alkyl-aryl, heteroalkyl, heteroaryl, aryl-heteroaryl, alkyl-heteroaryl, cycloalkyl, alkyloxy, alkyl-aryloxy, aryloxy, heteroaryloxy, heterocycloalkyloxy, heteroalkyl-heteroaryl, cycloalkyloxy, alkylamino, arylamino, alkyl-arylamino, arylamino, heteroarylamino, cycloalkylamino or heterocycloalkylamino, and R³¹ is unsubstituted or optionally substituted with one or two substituents which are the same or different and are independently selected from X¹³ or X¹⁴;
   X¹³ is alkyl, alkenyl, alkynyl, cycloalkyl, cycloalkyl-alkyl, heterocyclyl, heterocyclylalkyl, aryl, alkylaryl, arylalkyl, heteroaryl, alkylheteroaryl, or heteroarylalkyl, and X¹³ is unsubstituted or optionally substituted with one or more of X¹⁴ moieties which are the same or different and are independently selected;
   X¹⁴ is hydroxy, alkoxy, alkyl, alkenyl, alkynyl, aryl, aryloxy, thio, alkylthio, arylthio, amino, alkylamino, arylamino, alkylsulfonyl, arylsulfonyl, alkylsulfonamido, arylsulfonamido, carboxy, carbalkoxy, carboxamido, alkylcarbonyl, arylcarbonyl, heteroalkylcarbonyl, heteroarylcarbonyl, cycloalkylsulfonamido, heteroaryl-cycloalkylsulfonamido, heteroarylsulfonamido, alkoxycarbonylamino, alkoxycarbonyloxy, alkylureido, arylureido, halogen, cyano, or nitro, and said alkyl, alkoxy, and aryl are unsubstiuted or optionally independently substituted with one or more moieties which are the same or different and are independently selected from alkyl, alkenyl, alkynyl, cycloalkyl, cycloalkyl-alkyl, heterocyclyl, heterocyclylalkyl, aryl, alkylaryl, arylalkyl, heteroaryl, alkylheteroaryl, or heteroarylalkyl;
   W may be present or absent, and if W is present, W is C(=O), C(=S), C(=N-CN), or S(O₂);
(9) X is represented by structural Formula 4: 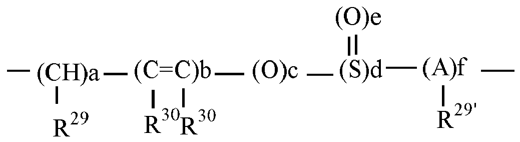 wherein in Formula 4:
   a is 2, 3, 4, 5, 6, 7, 8 or 9;
   b, c, d, e and f are 0, 1, 2, 3, 4 or 5;
   A is C, N, S or O;
   R²⁹ and R^{29'} are independently present or absent and if present can be the same or different, each being independently one or two substituents independently selected from the group consisting of: H, halo, alkyl, aryl, cycloalkyl, cycloalkylamino, cycloalkylaminocarbonyl, cyano, hydroxy, alkoxy, alkylthio, amino, -NH(alkyl), -NH(cycloalkyl), -N(alkyl)₂, carboxyl, C(O)O-alkyl, heteroaryl, aralkyl, alkylaryl, aralkenyl, heteroaralkyl, alkylheteroaryl, heteroaralkenyl, hydroxyalkyl, aryloxy, aralkoxy, acyl, aroyl, nitro, aryloxycarbonyl, aralkoxycarbonyl, alkylsulfonyl, arylsulfonyl, heteroarylsulfonyl, alkylsulfinyl, arylsulfinyl, heteroarylsulfinyl, arylthio, heteroarylthio, aralkylthio, heteroaralkylthio, cycloalkenyl, heterocyclyl, heterocyclenyl, Y₁Y₂N-alkyl-, Y₁Y₂NC(O)- and Y₁Y₂NSO₂-, wherein Y₁ and Y₂ can be the same or different and are independently selected from the group consisting of hydrogen, alkyl, aryl, and aralkyl; or
   R²⁹ and R^{29'} are linked together such that the combination is an aliphatic or heteroaliphatic chain of 0 to 6 carbons;
   R³⁰ is present or absent and if present is one or two substituents independently selected from the group consisting of: H, alkyl, aryl, heteroaryl and cylcoalkyl;
(10) D is represented by structural Formula 5: 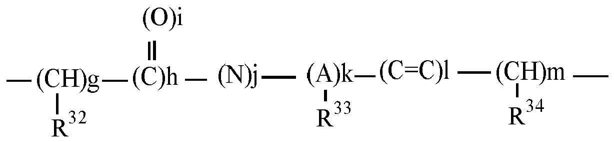 wherein in Formula 5:
   R³², R³³ and R³⁴ are present or absent and if present are independently one or two substituents independently selected from the group consisting of: H, halo, alkyl, aryl, cycloalkyl, cycloalkylamino, spiroalkyl, cycloalkylaminocarbonyl, cyano, hydroxy, alkoxy, alkylthio, amino, -NH(alkyl), -NH(cycloalkyl), -N(alkyl)₂, carboxyl, -C(O)O-alkyl, heteroaryl, aralkyl, alkylaryl, aralkenyl, heteroaralkyl, alkylheteroaryl, heteroaralkenyl, hydroxyalkyl, aryloxy, aralkoxy, acyl, aroyl, nitro, aryloxycarbonyl, aralkoxycarbonyl, alkylsulfonyl, arylsulfonyl, heteroarylsulfonyl, alkylsulfinyl, arylsulfinyl, heteroarylsulfinyl, arylthio, heteroarylthio, aralkylthio, heteroaralkylthio, cycloalkenyl, heterocyclyl, heterocyclenyl, Y₁Y₂N-alkyl-,
   Y₁Y₂NC(O)- and Y₁Y₂NSO₂-, wherein Y₁ and Y₂ can be the same or different and are independently selected from the group consisting of hydrogen, alkyl, aryl, and aralkyl; or
   R³² and R³⁴ are linked together such that the combination forms a portion of a cycloalkyl group;
   g is 1, 2, 3, 4, 5, 6, 7, 8 or 9;
   h, i, j, k, l and m are 0, 1, 2, 3, 4 or 5; and
   A is C, N, S or O,
(11) provided that when structural Formula 2:

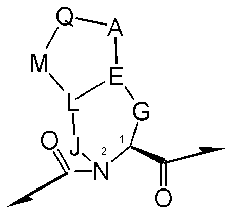

### Formula 2 is

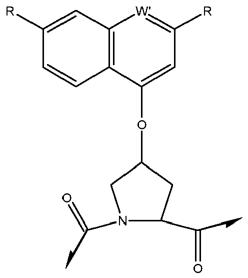

and
W' is CH or N, both the following conditional exclusions (i) and (ii) apply: conditional exclusion (i): Z' is not -NH-R³⁶, wherein R³⁶ is H, C_{6 or 10} aryl, heteroaryl, -C(O)-R³⁷, -C(O)-OR³⁷ or -C(O)-NHR³⁷, wherein R³⁷ is C₁₋₆ alkyl or C₃₋₆ cycloalkyl;
and
conditional exclusion (ii): R¹ is not -C(O)OH, a pharmaceutically acceptable salt of -C(O)OH, an ester of -C(O)OH or -C(O)NHR³⁸ wherein R³⁸ is selected from the group consisting of C₁₋₈ alkyl, C₃₋₆ cycloalkyl, C_{6 to 10} aryl or C₇₋₁₆ aralkyl;

### Formula VI, as disclosed in U.S. Patent Publication Ser. No. 2005/0085425:

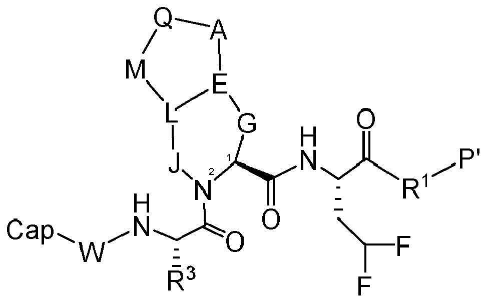

or a pharmaceutically acceptable salt, solvate, or ester thereof; wherein in Formula VI:
Cap is H, alkyl, alkyl-aryl, heteroalkyl, heteroaryl, aryl-heteroaryl, alkyl-heteroaryl, cycloalkyl, alkyloxy, alkyl-aryloxy, aryloxy, heteroaryloxy, heterocyclyloxy, cycloalkyloxy, amino, alkylamino, arylamino, alkyl-arylamino, arylamino, heteroarylamino, cycloalkylamino, carboxyalkylamino, arlylalkyloxy or heterocyclylamino, wherein each of said alkyl, alkyl-aryl, heteroalkyl, heteroaryl, aryl-heteroaryl, alkyl-heteroaryl, cycloalkyl, alkyloxy, alkyl-aryloxy, aryloxy, heteroaryloxy, heterocyclyloxy, cycloalkyloxy, amino, alkylamino, arylamino, alkyl-arylamino, arylamino, heteroarylamino, cycloalkylamino, carboxyalkylamino, arlylalkyloxy or heterocyclylamino can be unsubstituted or optionally independently substituted with one or two substituents which can be the same or different and are independently selected from X¹ and X² ;
P' is -NHR;
X¹ is alkyl, alkenyl, alkynyl, cycloalkyl, cycloalkyl-alkyl, heterocyclyl, heterocyclylalkyl, aryl, alkylaryl, arylalkyl, arylheteroaryl, heteroaryl, heterocyclylamino, alkylheteroaryl, or heteroarylalkyl, and X¹ can be unsubstituted or optionally independently substituted with one or more of X² moieties which can be the same or different and are independently selected; X² is hydroxy, alkyl, aryl, alkoxy, aryloxy, thio, alkylthio, arylthio, amino, alkylamino, arylamino, alkylsulfonyl, arylsulfonyl, alkylsulfonamido, arylsulfonamido, carboxy, carbalkoxy, carboxamido, alkoxycarbonylamino, alkoxycarbonyloxy, alkylureido, arylureido, halogen, cyano, keto, ester or nitro, wherein each of said alkyl, alkoxy, and aryl can be unsubstituted or optionally independently substituted with one or more moieties which can be the same or different and are independently selected from alkyl, alkenyl, alkynyl, cycloalkyl, cycloalkyl-alkyl, heterocyclyl, heterocyclylalkyl, aryl, alkylaryl, arylalkyl, arylheteroaryl, heteroaryl, heterocyclylamino, alkylheteroaryl and heteroarylalkyl;
W may be present or absent, and when W is present W is C(=O), C(=S), C(=NH), C(=N-OH), C(=N-CN), S(O) or S(O₂);
Q maybe present or absent, and when Q is present, Q is N(R), P(R), CR=CR', (CH₂)ₚ, (CHR)ₚ , (CRR')ₚ, (CHR-CHR')ₚ, O, S, S(O) or S(O₂); when Q is absent, M is (i) either directly linked to A or (ii) M is an independent substituent on L and A is an independent substituent on E, with said independent substituent being selected from -OR, -CH(R') , S(O)₀₋₂R or -NRR'; when both Q and M are absent, A is either directly linked to L, or A is an independent substituent on E, selected from -OR, CH(R)(R'), -S(O)₀₋₂R or -NRR';
A is present or absent and if present A is -O-, -O(R) CH₂-, -(CHR)ₚ-, - (CHR-CHR')ₚ-, (CRR')ₚ, N(R), NRR', S, or S(O₂), and when Q is absent, A is - OR, -CH(R)(R') or -NRR' ; and when A is absent, either Q and E are connected by a bond or Q is an independent substituent on M;
E is present or absent and if present E is CH, N, C(R);
G may be present or absent, and when G is present, G is (CH₂)ₚ, (CHR)ₚ, or (CRR')ₚ; when G is absent, J is present and E is directly connected to the carbon atom marked position 1;
J may be present or absent, and when J is present, J is (CH₂)ₚ, (CHR-CHR')ₚ, (CHR)ₚ, (CRR')ₚ, S(O₂), N(H), N(R) or O; when J is absent and G is present, L is directly linked to the nitrogen atom marked position 2;
L may be present or absent, and when L is present, L is CH, N, or CR; when L is absent, M is present or absent; if M is present with L being absent, then M is directly and independently linked to E, and J is directly and independently linked to E;
M may be present or absent, and when M is present, M is O, N(R), S, S(O₂), (CH₂)ₚ, (CHR)ₚ, (CHR-CHR')ₚ, or (CRR')ₚ;
p is a number from 0 to 6;
R, R' and R³ can be the same or different, each being independently selected from the group consisting of: H, C₁-C₁₀ alkyl, C₂-C₁₀ alkenyl, C₃-C₈ cycloalkyl, C₃-C₈ heterocyclyl, alkoxy, aryloxy, alkylthio, arylthio, amino, amido, arylthioamino, arylcarbonylamino, arylaminocarboxy, alkylaminocarboxy, heteroalkyl, heteroalkenyl, alkenyl, alkynyl, aryl-alkyl, heteroarylalkyl, ester, carboxylic acid, carbamate, urea, ketone, aldehyde, cyano, nitro, halogen, (cycloalkyl)alkyl, aryl, heteroaryl, alkyl-aryl, alkylheteroaryl, alkyl-heteroaryl and (heterocyclyl)alkyl;
R and R' in (CRR') can be linked together such that the combination forms a cycloalkyl or heterocyclyl moiety; and
R¹ is carbonyl;

### Formula VII, as disclosed in US Patent 7,253,160:

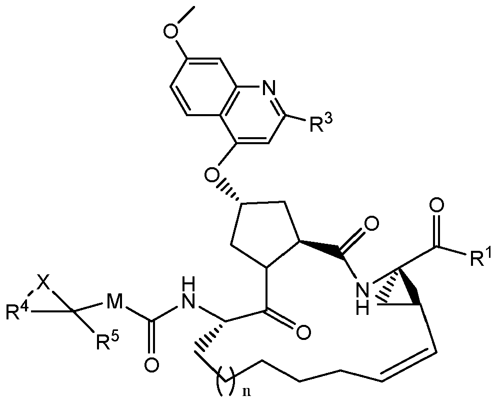

or a pharmaceutically acceptable salt, solvate, or ester thereof; wherein in Formula VII:
M is O, N(H), or CH₂;
n is 0-4;
R¹ is -OR⁶, -NR⁶R⁷ or 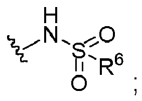 where R⁶ and R⁷ can be the same or different, each being independently selected from the group consisting of hydrogen, alkyl, alkenyl, alkynyl, heteroalkyl, cycloalkyl, cycloalkylalkyl, aryl, arylalkyl, heteroaryl, heteroarylalkyl, heterocyclyl, heterocyclylalkyl, hydroxyl, amino, arylamino and alkylamino;
   R⁴ and R⁵ can be the same or different, each being independently selected from the group consisting of H, alkyl, aryl and cycloalkyl; or alternatively R⁴ and R⁵ together form part of a cyclic 5- to 7- membered ring such that the moiety 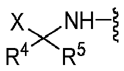 is represented by 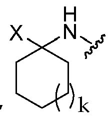 where k is 0 to 2;
X is selected from the group consisting of: 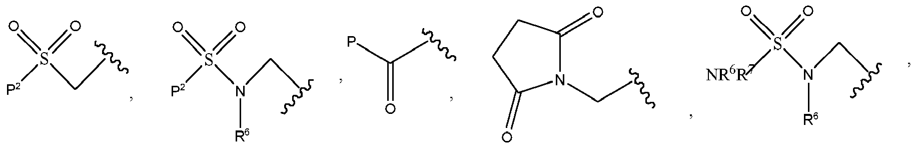 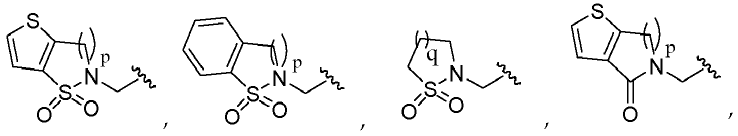 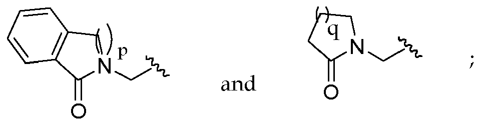 where p is 1 to 2, q is 1-3 and P² is alkyl, aryl, heteroaryl, heteroalkyl, cycloalkyl, dialkylamino, alkylamino, arylamino or cycloalkylamino; and
R³ is selected from the group consisting of: aryl, heterocyclyl, heteroaryl, 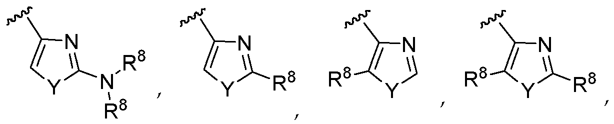 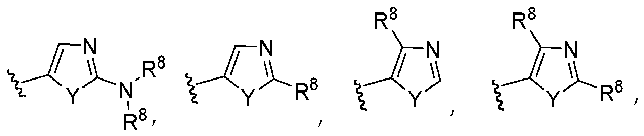 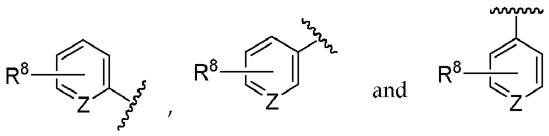 where Y is O, S or NH, and Z is CH or N, and the R⁸ moieties can be the same or different, each R⁸ being independently selected from the group consisting of hydrogen, alkyl, heteroalkyl, cycloalkyl, aryl, heteroaryl, heterocyclyl, hydroxyl, amino, arylamino, alkylamino, dialkylamino, halo, alkylthio, arylthio and alkyloxy;

### Formula VIII, as disclosed in US Patent 7,253,160:

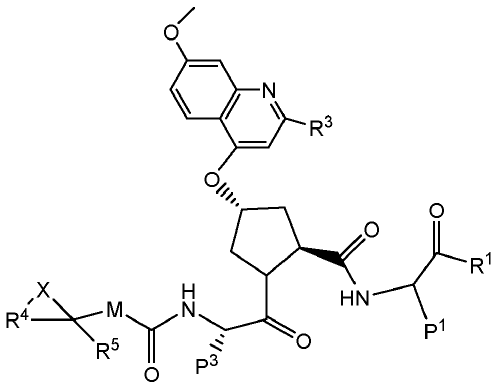

or a pharmaceutically acceptable salt, solvate, or ester thereof; wherein in Formula VIII:
M is O, N(H), or CH₂;
R¹ is -C(O)NHR⁶, where R⁶ is hydrogen, alkyl, alkenyl, alkynyl, heteroalkyl, cycloalkyl, cycloalkylalkyl, aryl, arylalkyl, heteroaryl, heteroarylalkyl, heterocyclyl, heterocyclylalkyl, hydroxyl, amino, arylamino or alkylamino;
P₁ is selected from the group consisting of alkyl, alkenyl, alkynyl, cycloalkyl haloalkyl;
P₃ is selected from the group consisting of alkyl, cycloalkyl, aryl and cycloalkyl fused with aryl;
R⁴ and R⁵ can be the same or different, each being independently selected from the group consisting of H, alkyl, aryl and cycloalkyl; or alternatively R⁴ and R⁵ together form part of a cyclic 5- to 7- membered ring such that the
   moiety 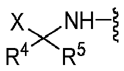 is represented by 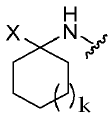 where k is 0 to 2;
X is selected from the group consisting of: 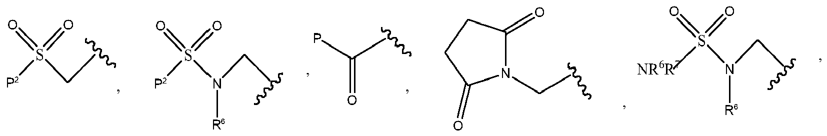 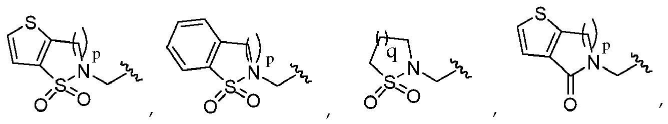 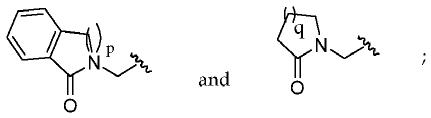 where p is 1 to 2, q is 1 to 3 and P² is alkyl, aryl, heteroaryl, heteroalkyl, cycloalkyl, dialkylamino, alkylamino, arylamino or cycloalkylamino; and
R³ is selected from the group consisting of: aryl, heterocyclyl, heteroaryl, 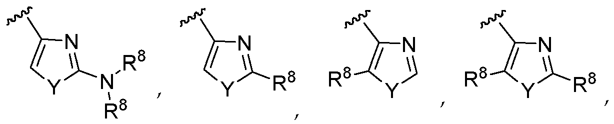 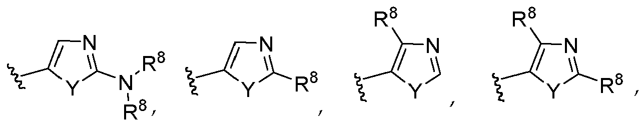 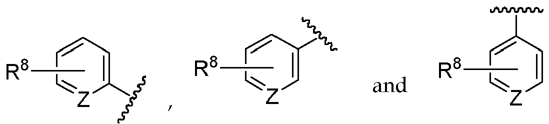 where Y is O, S or NH, and Z is CH or N, and the R⁸ moieties can be the same or different, each R⁸ being independently selected from the group consisting of hydrogen, alkyl, heteroalkyl, cycloalkyl, aryl, heteroaryl, heterocyclyl, hydroxyl, amino, arylamino, alkylamino, dialkylamino, halo, alkylthio, arylthio and alkyloxy;

### Formula IX, as disclosed in US Patent 7,253,160:

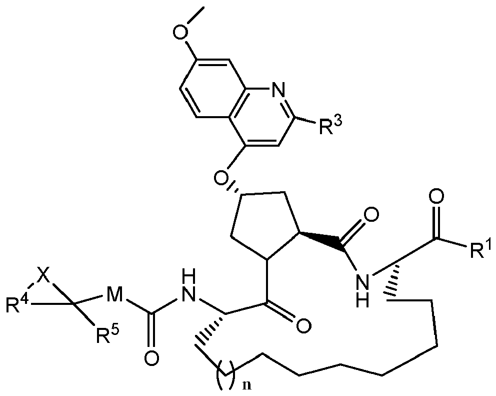

or a pharmaceutically acceptable salt, solvate, or ester thereof; wherein in Formula IX:
M is O, N(H), or CH₂;
n is 0-4;
R¹ is -OR⁶, -NR⁶R⁷ or 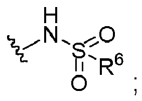 where R⁶ and R⁷ can be the same or different, each being independently selected from the group consisting of hydrogen, alkyl, alkenyl, alkynyl, heteroalkyl, cycloalkyl, cycloalkylalkyl, aryl, arylalkyl, heteroaryl, heteroarylalkyl, heterocyclyl, heterocyclylalkyl, hydroxyl, amino, arylamino and alkylamino;
   R⁴ and R⁵ can be the same or different, each being independently selected from the group consisting of H, alkyl, aryl and cycloalkyl; or alternatively R⁴ and R⁵ together form part of a cyclic 5- to 7- membered ring such that the moiety 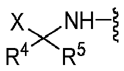 is represented by 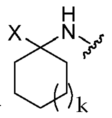 where k is 0 to 2;
X is selected from the group consisting of: 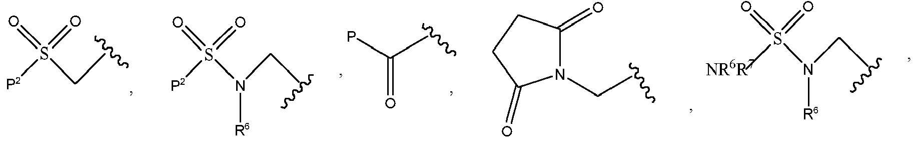 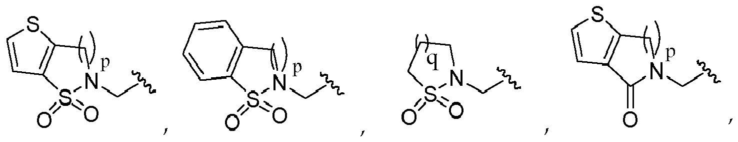 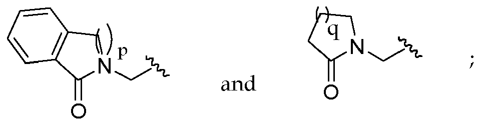
where p is 1 to 2, q is 1 to 3 and P² is alkyl, aryl, heteroaryl, heteroalkyl, cycloalkyl, dialkylamino, alkylamino, arylamino or cycloalkylamino; and
R³ is selected from the group consisting of: aryl, heterocyclyl, heteroaryl, 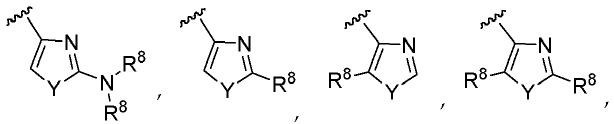 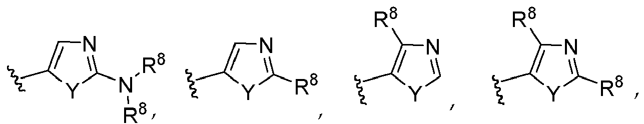 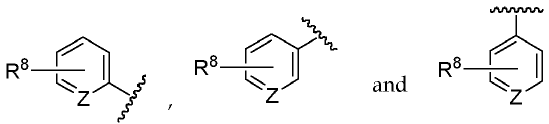 where Y is O, S or NH, and Z is CH or N, and the R⁸ moieties can be the same or different, each R⁸ being independently selected from the group consisting of hydrogen, alkyl, heteroalkyl, cycloalkyl, aryl, heteroaryl, heterocyclyl, hydroxyl, amino, arylamino, alkylamino, dialkylamino, halo, alkylthio, arylthio and alkyloxy;

### Formula X, as disclosed in US PAtent 7,205,330:

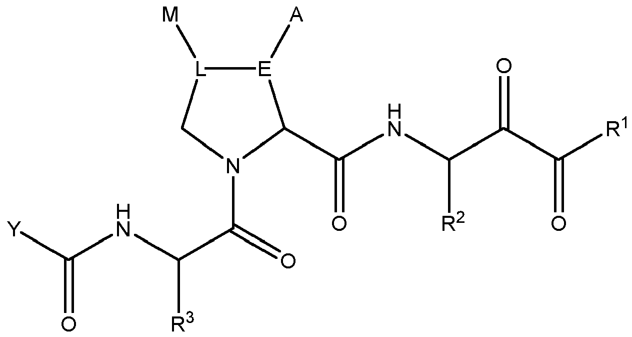

or a pharmaceutically acceptable salt, solvate, or ester thereof; wherein in Formula X:
R¹ is NHR⁹, wherein R⁹ is H, alkyl-, alkenyl-, alkynyl-, aryl-, heteroalkyl-, heteroaryl-, cycloalkyl-, heterocyclyl-, arylalkyl-, or heteroarylalkyl;
A and M can be the same or different, each being independently selected from R, OR, NHR, NRR', SR, SO₂R, and halo; or A and M are connected to each other such that the moiety: 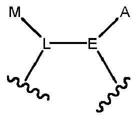 shown above in Formula I forms either a three, four, six, seven or eight-membered cycloalkyl, a four to eight-membered heterocyclyl, a six to ten-membered aryl, or a five to ten-membered heteroaryl;
E is C(H) or C(R);
L is C(H), C(R), CH₂C(R), or C(R)CH₂;
R, R', R², and R³ can be the same or different, each being independently selected from the group consisting of H, alkyl-, alkenyl-, alkynyl-, cycloalkyl-, heteroalkyl-, heterocyclyl-, aryl-, heteroaryl-, (cycloalkyl)alkyl-, (heterocyclyl)alkyl-, aryl-alkyl-, and heteroaryl-alkyl-; or alternately R and R' in NRR' are connected to each other such that NRR' forms a four to eight-membered heterocyclyl;
and Y is selected from the following moieties: 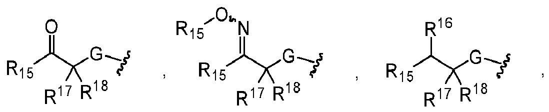 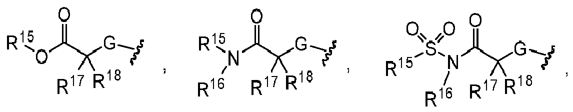 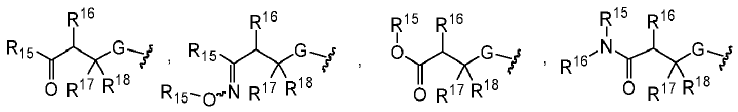 wherein G is NH or O; and R¹⁵ , R¹⁶, R¹⁷ and R¹⁸ can be the same or different, each being independently selected from the group consisting of H, alkyl, heteroalkyl, alkenyl, heteroalkenyl, alkynyl, heteroalkynyl, cycloalkyl, heterocyclyl, aryl, arylalkyl, heteroaryl, and heteroarylalkyl, or alternately, R¹⁵ and R¹⁶ are connected to each other to form a four to eight-membered cycloalkyl, heteroaryl or heterocyclyl structure, and likewise, independently R¹⁷ and R¹⁸ are connected to each other to form a three to eight-membered cycloalkyl or heterocyclyl;
   wherein each of said alkyl, aryl, heteroaryl, cycloalkyl or heterocyclyl can be unsubstituted or optionally independently substituted with one or more moieties selected from the group consisting of: hydroxy, alkoxy, aryloxy, thio, alkylthio, arylthio, amino, amido, alkylamino, arylamino, alkylsulfonyl, arylsulfonyl, sulfonamido, alkyl, aryl, heteroaryl, alkylsulfonamido, arylsulfonamido, keto, carboxy, carbalkoxy, carboxamido, alkoxycarbonylamino, alkoxycarbonyloxy, alkylureido, arylureido, halo, cyano, and nitro;

### Formula XI, as disclosed in US Patent 7,192,957:

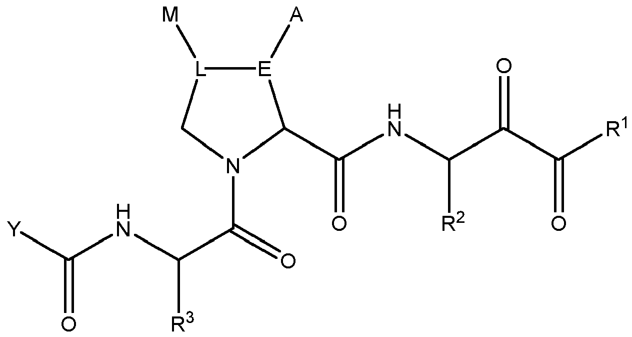

or a pharmaceutically acceptable salt, solvate, or ester thereof; wherein in Formula XI:
R¹ is NHR⁹, wherein R⁹ is H, alkyl-, alkenyl-, alkynyl-, aryl-, heteroalkyl-, heteroaryl-, cycloalkyl-, heterocyclyl-, arylalkyl-, or heteroarylalkyl;
A and M can be the same or different, each being independently selected from R, NR⁹R¹⁰, SR, SO₂R, and halo; or A and M are connected to each other (in other words, A-E-L-M taken together) such that the moiety: 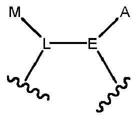
shown above in Formula I forms either a three, four, six, seven or eight-membered cycloalkyl, a four to eight-membered heterocyclyl, a six to ten-membered aryl, or a five to ten-membered heteroaryl;
E is C(H) or C(R);
L is C(H), C(R), CH₂C(R), or C(R)CH₂;
R, R', R², and R³ can be the same or different, each being independently selected from the group consisting of H, alkyl-, alkenyl-, alkynyl-, cycloalkyl-, heteroalkyl-, heterocyclyl-, aryl-, heteroaryl-, (cycloalkyl)alkyl-, (heterocyclyl)alkyl-, aryl-alkyl-, and heteroaryl-alkyl-; or alternately R and R' in NRR' are connected to each other such that NR⁹R¹⁰ forms a four to eight-membered heterocyclyl;
Y is selected from the following moieties: 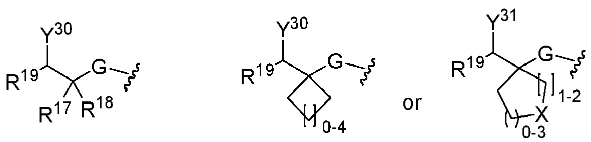 wherein Y³⁰ and Y³¹ are selected from 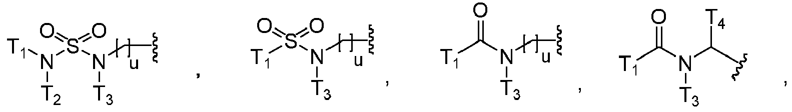 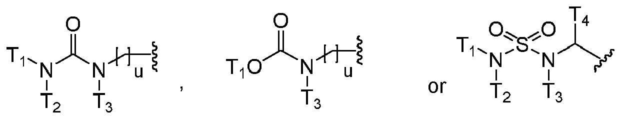 where u is a number 0-6;
X is selected from O, NR¹⁵, NC(O)R¹⁶, S, S(O) and SO₂;
G is NH or O; and
R¹⁵, R¹⁶, R¹⁷, R¹⁸, R¹⁹, T₁, T₂, T₃ and T₄ can be the same or different, each being independently selected from the group consisting of H, alkyl, heteroalkyl, alkenyl, heteroalkenyl, alkynyl, heteroalkynyl, cycloalkyl, heterocyclyl, aryl, arylalkyl, heteroaryl, and heteroarylalkyl, or alternately, R¹⁷ and R¹⁸ are connected to each other to form a three to eight-membered cycloalkyl or heterocyclyl;
   wherein each of said alkyl, aryl, heteroaryl, cycloalkyl or heterocyclyl can be unsubstituted or optionally independently substituted with one or more moieties selected from the group consisting of: hydroxy, alkoxy, aryloxy, thio, alkylthio, arylthio, amino, amido, alkylamino, arylamino, alkylsulfonyl, arylsulfonyl, sulfonamido, alkyl, aryl, heteroaryl, alkylsulfonamido, arylsulfonamido, keto, carboxy, carbalkoxy, carboxamido, alkoxycarbonylamino, alkoxycarbonyloxy, alkylureido, arylureido, halo, cyano, and nitro;

### Formula XII, as disclosed in US Patent 7,186,747:

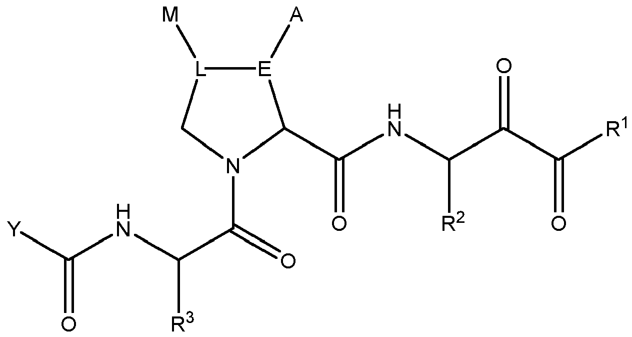

or a pharmaceutically acceptable salt, solvate, or ester thereof; wherein in Formula XII:
R¹ is NHR⁹, wherein R⁹ is H, alkyl-, alkenyl-, alkynyl-, aryl-, heteroalkyl-, heteroaryl-, cycloalkyl-, heterocyclyl-, arylalkyl-, or heteroarylalkyl;
A and M can be the same or different, each being independently selected from R, OR, NHR, NRR', SR, SO₂R, and halo; or A and M are connected to each other such that the moiety: 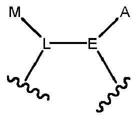 shown above in Formula I forms either a three, four, six, seven or eight-membered cycloalkyl, a four to eight-membered heterocyclyl, a six to ten-membered aryl, or a five to ten-membered heteroaryl;
E is C(H) or C(R);
L is C(H), C(R), CH₂C(R), or C(R)CH₂;
R, R', R², and R³ can be the same or different, each being independently selected from the group consisting of H, alkyl-, alkenyl-, alkynyl-, cycloalkyl-, heteroalkyl-, heterocyclyl-, aryl-, heteroaryl-, (cycloalkyl)alkyl-, (heterocyclyl)alkyl-, aryl-alkyl-, and heteroaryl-alkyl-; or alternately R and R' in NRR' are connected to each other such that NRR' forms a four to eight-membered heterocyclyl;
   and Y is selected from the following moieties: 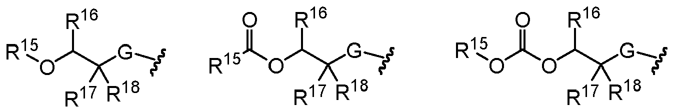 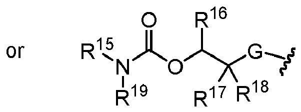 wherein G is NH or O; and R¹⁵ R¹⁶, R¹⁷, R¹⁸, and R¹⁹ can be the same or different, each being independently selected from the group consisting of H, alkyl, heteroalkyl, alkenyl, heteroalkenyl, alkynyl, heteroalkynyl, cycloalkyl, heterocyclyl, aryl, arylalkyl, heteroaryl, and heteroarylalkyl, or alternately, (i) either R¹⁵ and R¹⁶ are connected to each other to form a four to eight-membered cyclic structure, or R¹⁵ and R¹⁹ are connected to each other to form a four to eight-membered cyclic structure, and (ii) likewise, independently, R¹⁷ and R¹⁸ are connected to each other to form a three to eight-membered cycloalkyl or heterocyclyl;
   wherein each of said alkyl, aryl, heteroaryl, cycloalkyl or heterocyclyl can be unsubstituted or optionally independently substituted with one or more moieties selected from the group consisting of: hydroxy, alkoxy, aryloxy, thio, alkylthio, arylthio, amino, amido, alkylamino, arylamino, alkylsulfonyl, arylsulfonyl, sulfonamido, alkylsulfonamido, arylsulfonamido, alkyl, aryl, heteroaryl, keto, carboxy, carbalkoxy, carboxamido, alkoxycarbonylamino, alkoxycarbonyloxy, alkylureido, arylureido, halo, cyano, and nitro;

### Formula XIII, as disclosed in US Patent 7,173,057:

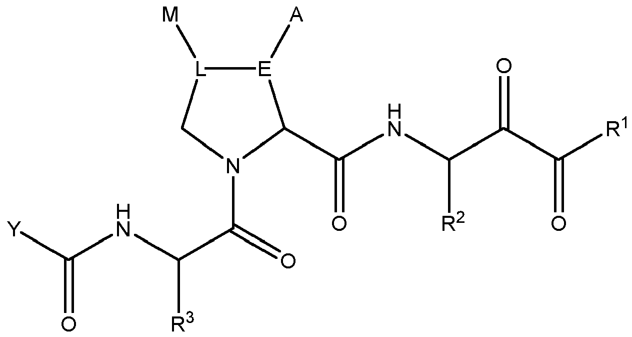

or a pharmaceutically acceptable salt, solvate, or ester thereof;
wherein in Formula XIII:
R¹ is NHR⁹, wherein R⁹ is H, alkyl-, alkenyl-, alkynyl-, aryl-, heteroalkyl-, heteroaryl-, cycloalkyl-, heterocyclyl-, arylalkyl-, or heteroarylalkyl;
A and M can be the same or different, each being independently selected from R, OR, NHR, NRR', SR, SO₂R, and halo; or A and M are connected to each other (in other words, A-E-L-M taken together) such that the moiety: 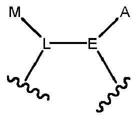 shown above in Formula I forms either a three, four, six, seven or eight-membered cycloalkyl, a four to eight-membered heterocyclyl, a six to ten-membered aryl, or a five to ten-membered heteroaryl;
E is C(H) or C(R);
L is C(H), C(R), CH₂C(R), or C(R)CH₂;
R, R', R², and R³ can be the same or different, each being independently selected from the group consisting of H, alkyl-, alkenyl-, alkynyl-, cycloalkyl-, heteroalkyl-, heterocyclyl-, aryl-, heteroaryl-, (cycloalkyl)alkyl-, (heterocyclyl)alkyl-, aryl-alkyl-, and heteroaryl-alkyl-; or alternately R and R' in NRR' are connected to each other such that NRR' forms a four to eight-membered heterocyclyl;
   and Y is selected from the following moieties: 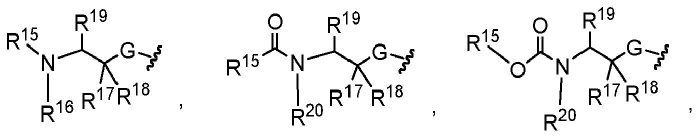 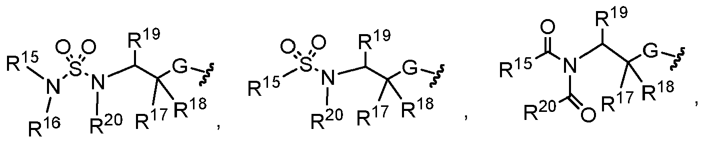 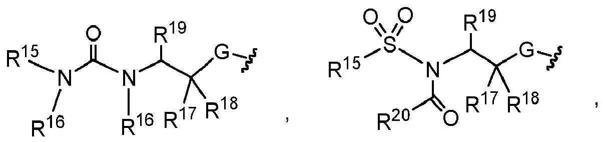 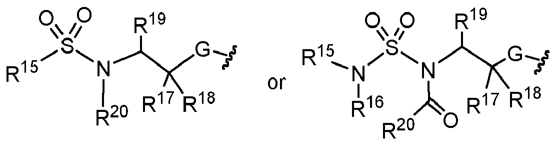 wherein G is NH or O, and R¹⁵ R¹⁶, R¹⁷, R¹⁸, R¹⁹ and R²⁰ can be the same or different, each being independently selected from the group consisting of H, C₁-C₁₀ alkyl, C₁-C₁₀ heteroalkyl, C₂-C₁₀ alkenyl, C₂-C₁₀ heteroalkenyl, C₂-C₁₀ alkynyl, C₂-C₁₀ heteroalkynyl, C₃-C₈ cycloalkyl, C₃-C₈ heterocyclyl, aryl, heteroaryl, or alternately: (i) either R¹⁵ and R¹⁶ can be connected to each other to form a four to eight-membered cycloalkyl or heterocyclyl, or R¹⁵ and R¹⁹ are connected to each other to form a five to eight-membered cycloalkyl or heterocyclyl, or R¹⁵ and R²⁰ are connected to each other to form a five to eight-membered cycloalkyl or heterocyclyl, and (ii) likewise, independently, R¹⁷ and R¹⁸ are connected to each other to form a three to eight-membered cycloalkyl or heterocyclyl,
   wherein each of said alkyl, aryl, heteroaryl, cycloalkyl or heterocyclyl can be unsubstituted or optionally independently substituted with one or more moieties selected from the group consisting of: hydroxy, alkoxy, aryloxy, thio, alkylthio, arylthio, amino, amido, alkylamino, arylamino, alkylsulfonyl, arylsulfonyl, sulfonamido, alkylsulfonamido, arylsulfonamido, keto, carboxy, carbalkoxy, carboxamido, alkoxycarbonylamino, alkoxycarbonyloxy, alkylureido, arylureido, halo, cyano, and nitro;

### Formula XIV, as disclosed in US Patent Publication 2007/0042968:

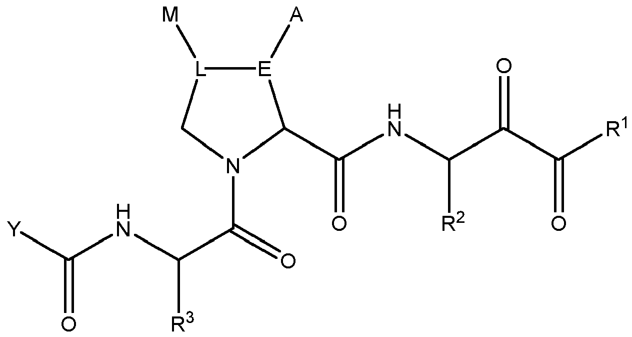

or a pharmaceutically acceptable salt, solvate, or ester thereof; wherein in Formula XIV:
R¹ is NHR⁹, wherein R⁹ is H, alkyl-, alkenyl-, alkynyl-, aryl-, heteroalkyl-, heteroaryl-, cycloalkyl-, heterocyclyl-, arylalkyl-, or heteroarylalkyl;
A and M can be the same or different, each being independently selected from R, OR, NHR, NRR', SR, SO₂R, and halo;
   or A and M are connected to each other such that the moiety: 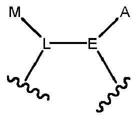 shown above in Formula I forms either a three, four, six, seven or eight-membered cycloalkyl, a four to eight-membered heterocyclyl, a six to ten-membered aryl, or a five to ten-membered heteroaryl;
E is C(H) or C=;
L is C(H), C=, CH₂C=, or C=CH₂;
R, R', R², and R³ can be the same or different, each being independently selected from the group consisting of H, alkyl, heteroalkyl, alkenyl, heteroalkenyl, alkynyl, heteroalkynyl, cycloalkyl, heterocyclyl, aryl, arylalkyl, heteroaryl, and heteroarylalkyl, or alternately R and R' in NRR' are connected to each other such that NRR' forms a four to eight-membered heterocyclyl;
   and Y is selected from the following moieties: 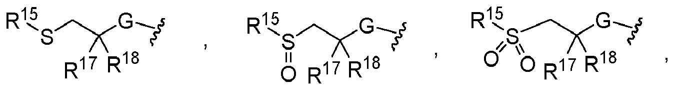 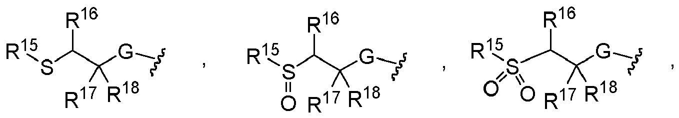 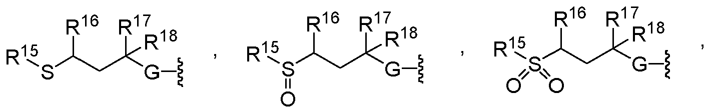 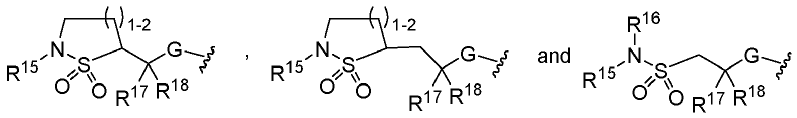 wherein G is NH or O; and R¹⁵, R¹⁶, R¹⁷ and R¹⁸ can be the same or different, each being independently selected from the group consisting of H, alkyl, heteroalkyl, alkenyl, heteroalkenyl, alkynyl, heteroalkynyl, cycloalkyl, heterocyclyl, aryl, and heteroaryl, or alternately, (i) R¹⁵ and R¹⁶ are connected to each other to form a four to eight-membered cyclic structure, and (ii) likewise, independently R¹⁷ and R¹⁸ are connected to each other to form a three to eight-membered cycloalkyl or heterocyclyl;
   wherein each of said alkyl, aryl, heteroaryl, cycloalkyl or heterocyclyl can be unsubstituted or optionally independently substituted with one or more moieties selected from the group consisting of: hydroxy, alkoxy, aryloxy, thio, alkylthio, arylthio, amino, amido, alkylamino, arylamino, alkylsulfonyl, arylsulfonyl, sulfonamido, alkylsulfonamido, arylsulfonamido, alkyl, aryl, heteroaryl, keto, carboxy, carbalkoxy, carboxamido, alkoxycarbonylamino, alkoxycarbonyloxy, alkylureido, arylureido, halo, cyano, and nitro;

### Formula XV, as dcisclosed in U.S. Patent Publication 2005/0153900:

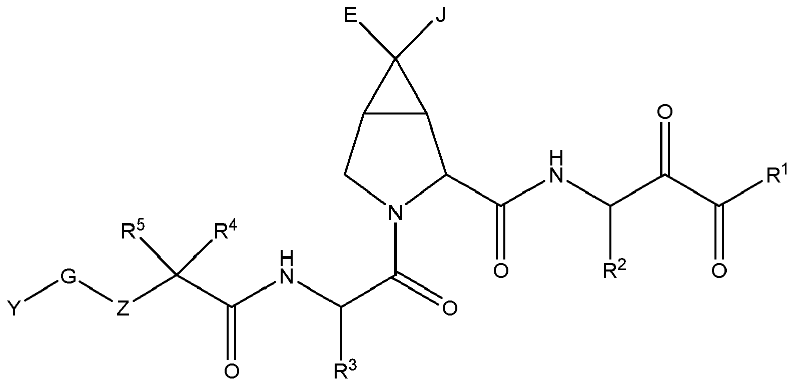

or a pharmaceutically acceptable salt, solvate, or ester thereof; wherein in Formula XV:
R¹ is NHR⁹, wherein R⁹ is H, alkyl-, aryl-, heteroalkyl-, heteroaryl-, cycloalkyl-, cycloalkyl-, arylalkyl-, or heteroarylalkyl;
E and J can be the same or different, each being independently selected from the group consisting of R, OR, NHR, NRR⁷, SR, halo, and S(O₂)R, or E and J can be directly connected to each other to form either a three to eight-membered cycloalkyl, or a three to eight-membered heterocyclyl moiety;
Z is N(H), N®, or O, with the proviso that when Z is O, G is present or absent and if G is present with Z being O, then G is C(=O);
G maybe present or absent, and if G is present, G is C(=O) or S(O₂), and when G is absent, Z is directly connected to Y;
Y is selected from the group consisting of: 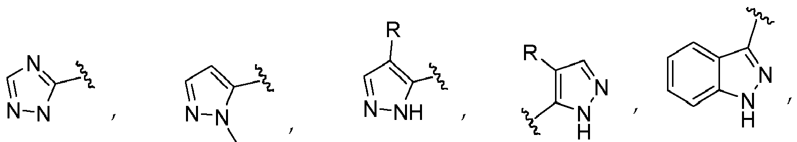 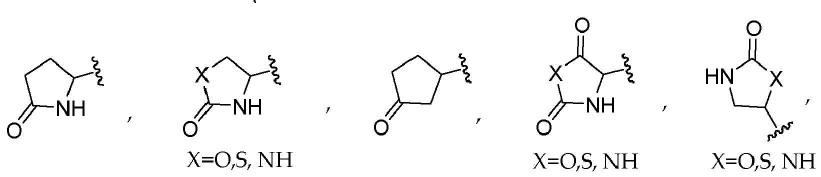 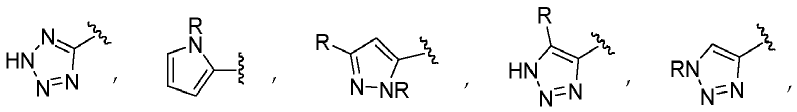 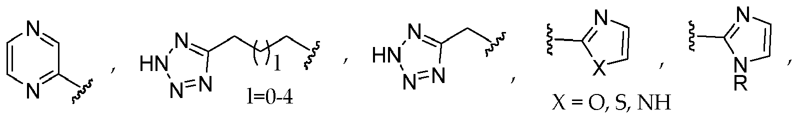 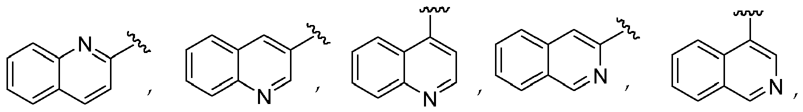 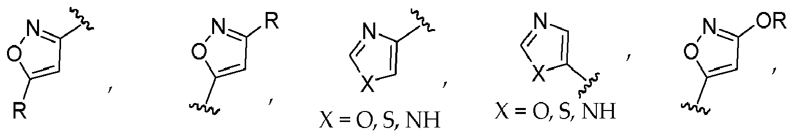 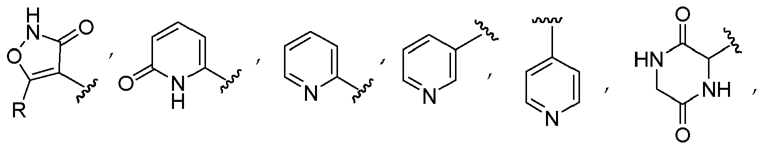 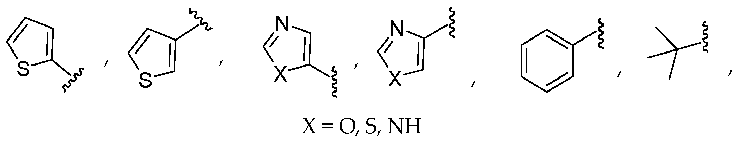 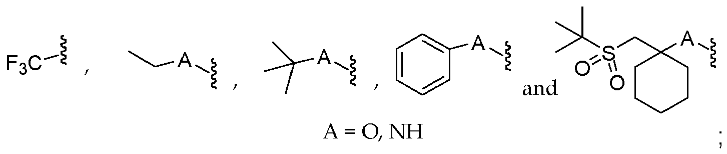
R, R⁷, R², R³, R⁴ and R⁵ can be the same or different, each being independently selected from the group consisting of H, alkyl-, alkenyl-, alkynyl-, cycloalkyl-, heteroalkyl-, heterocyclyl-, aryl-, heteroaryl-, (cycloalkyl)alkyl-, (heterocyclyl)alkyl-, aryl-alkyl-, and heteroaryl-alkyl-, wherein each of said heteroalkyl, heteroaryl and heterocyclyl independently has one to six oxygen, nitrogen, sulfur, or phosphorus atoms;
wherein each of said alkyl, heteroalkyl, alkenyl, alkynyl, aryl, heteroaryl, cycloalkyl and heterocyclyl moieties can be unsubstituted or optionally independently substituted with one or more moieties selected from the group consisting of alkyl, alkenyl, alkynyl, aryl, aralkyl, cycloalkyl, heterocyclyl, halo, hydroxy, thio, alkoxy, aryloxy, alkylthio, arylthio, amino, amido, ester, carboxylic acid, carbamate, urea, ketone, aldehyde, cyano, nitro, sulfonamido, sulfoxide, sulfone, sulfonyl urea, hydrazide, and hydroxamate;

### Formula XVI, as disclosed in U.S. Patent Publication 2005/0197301:

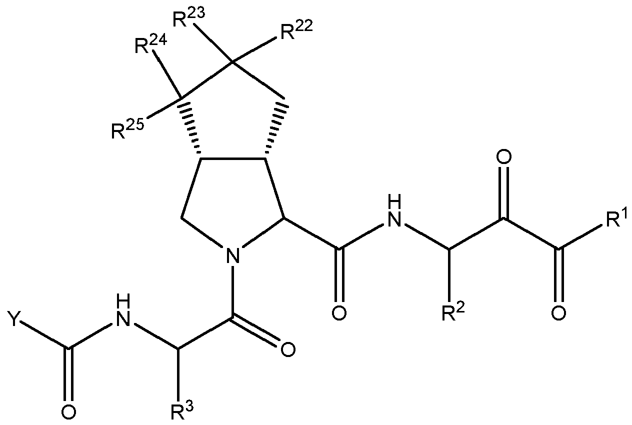

or a pharmaceutically acceptable salt, solvate, or ester thereof;
wherein in Formula XVI:
R¹ is NHR⁹, wherein R⁹ is H, alkyl-, alkenyl-, alkynyl-, aryl-, heteroalkyl-, heteroaryl-, cycloalkyl-, heterocyclyl-, arylalkyl-, or heteroarylalkyl;
R² and R³ can be the same or different, each being independently selected from the group consisting of H, alkyl, heteroalkyl, alkenyl, heteroalkenyl, alkynyl, heteroalkynyl, cycloalkyl, heterocyclyl, aryl, arylalkyl, heteroaryl, and heteroarylalkyl;
Y is selected from the following moieties: 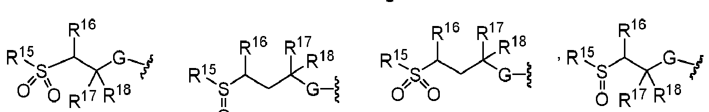 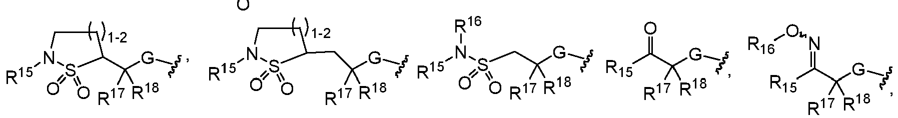 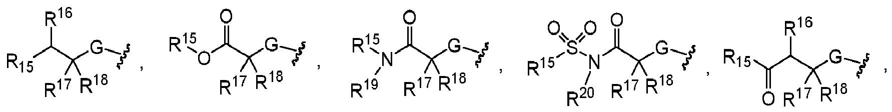 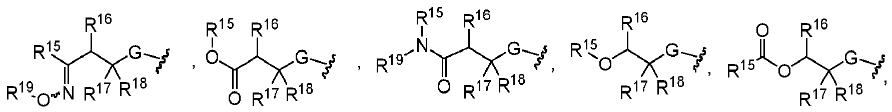 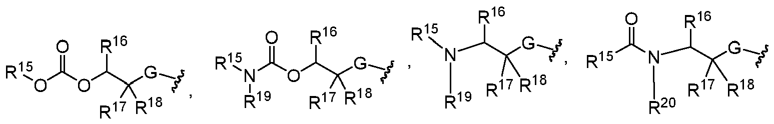 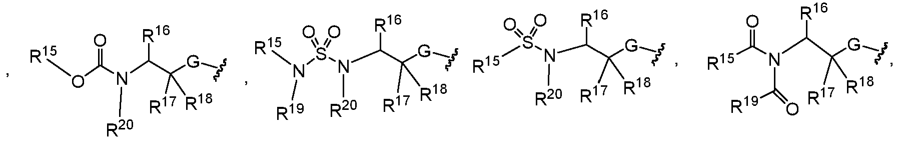 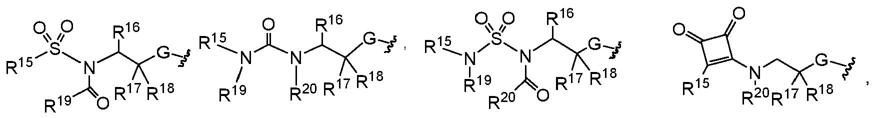 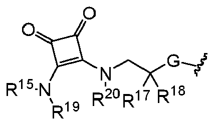 wherein G is NH or O; and R¹⁵ R¹⁶, R¹⁷, R¹⁸, R¹⁹, R²⁰, R²¹, R²², R²³, R²⁴ and R²⁵ can be the same or different, each being independently selected from the group consisting of H, alkyl, heteroalkyl, alkenyl, heteroalkenyl, alkynyl, heteroalkynyl, cycloalkyl, heterocyclyl, aryl, arylalkyl, heteroaryl, and heteroarylalkyl, or alternately (i) R¹⁷ and R¹⁸ are independently connected to each other to form a three to eight-membered cycloalkyl or heterocyclyl; (ii) likewise independently R¹⁵ and R¹⁹ are connected to each other to form a four to eight-membered heterocyclyl; (iii) likewise independently R¹⁵ and R¹⁶ are connected to each other to form a four to eight-membered heterocyclyl; (iv) likewise independently R¹⁵ and R²⁰ are connected to each other to form a four to eight-membered heterocyclyl; (v) likewise independently R²² and R²³ are connected to each other to form a three to eight-membered cycloalkyl or a four to eight-membered heterocyclyl; and (vi) likewise independently R²⁴ and R²⁵ are connected to each other to form a three to eight-membered cycloalkyl or a four to eight-membered heterocyclyl;
wherein each of said alkyl, aryl, heteroaryl, cycloalkyl or heterocyclyl can be unsubstituted or optionally independently substituted with one or more moieties selected from the group consisting of hydroxy, alkoxy, aryloxy, thio, alkylthio, arylthio, amino, amido, alkylamino, arylamino, alkylsulfonyl, arylsulfonyl, sulfonamido, alkyl, aryl, heteroaryl, alkylsulfonamido, arylsulfonamido, keto, carboxy, carbalkoxy, carboxamido, alkoxycarbonylamino, alkoxycarbonyloxy, alkylureido, arylureido, halo, cyano, and nitro;

### Formula XVII, as disclosed in U.S. Patent Publication 2005/0209164:

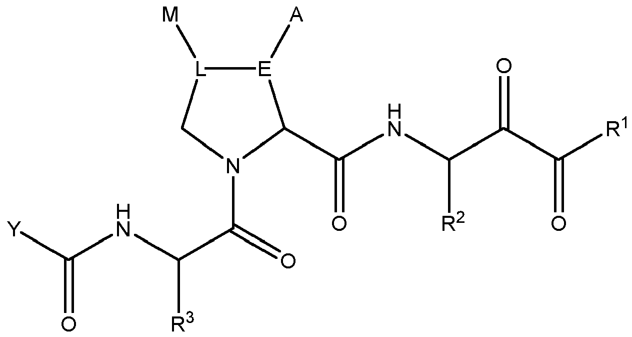

or a pharmaceutically acceptable salt, solvate, or ester thereof; wherein in Formula XVII:
R¹ is NHR⁹, wherein R⁹ is H, alkyl-, alkenyl-, alkynyl-, aryl-, heteroalkyl-, heteroaryl-, cycloalkyl-, heterocyclyl-, arylalkyl-, or heteroarylalkyl;
A and M can be the same or different, each being independently selected from R, OR, NHR, NRR', SR, SO₂R, and halo; or A and M are connected to each other such that the moiety: 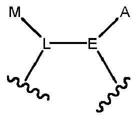
shown above in Formula I forms either a three, four, six, seven or eight-membered cycloalkyl, a four to eight-membered heterocyclyl, a six to ten-membered aryl, or a five to ten-membered heteroaryl;
E is C(H) or C=;
L is C(H), C=, CH₂C=, or C=CH₂;
R, R', R², and R³ can be the same or different, each being independently selected from the group consisting of H, alkyl-, alkenyl-, alkynyl-, cycloalkyl-, heteroalkyl-, heterocyclyl-, aryl-, heteroaryl-, (cycloalkyl)alkyl-, (heterocyclyl)alkyl-, aryl-alkyl-, and heteroaryl-alkyl-; or alternately R and R' in NRR' are connected to each other such that NRR' forms a four to eight-membered heterocyclyl;
Y is selected from the following moieties: 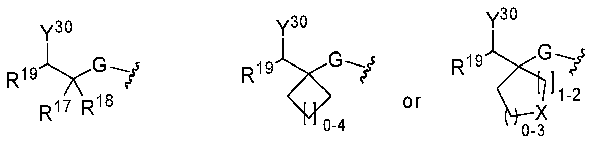 wherein Y³⁰ is selected from 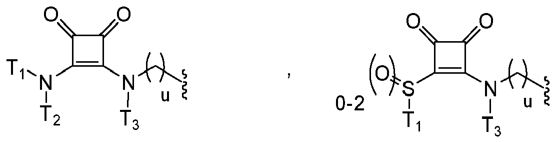 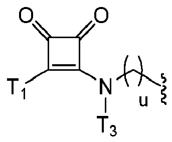 where u is a number 0-1;
X is selected from O, NR¹⁵, NC(O)R¹⁶, S, S(O) and SO₂; G is NH or O; and
R¹⁵, R¹⁶, R¹⁷, R¹⁸, R¹⁹, T₁, T₂, and T₃ can be the same or different, each being independently selected from the group consisting of H, alkyl, heteroalkyl, alkenyl, heteroalkenyl, alkynyl, heteroalkynyl, cycloalkyl, heterocyclyl, aryl, arylalkyl, heteroaryl, and heteroarylalkyl, or alternately, R¹⁷ and R¹⁸ are connected to each other to form a three to eight-membered cycloalkyl or heterocyclyl;
wherein each of said alkyl, aryl, heteroaryl, cycloalkyl or heterocyclyl can be unsubstituted or optionally independently substituted with one or more moieties selected from the group consisting of: hydroxy, alkoxy, aryloxy, thio, alkylthio, arylthio, amino, amido, alkylamino, arylamino, alkylsulfonyl, arylsulfonyl, sulfonamido, alkyl, aryl, heteroaryl, alkylsulfonamido, arylsulfonamido, keto, carboxy, carbalkoxy, carboxamido, alkoxycarbonylamino, alkoxycarbonyloxy, alkylureido, arylureido, halo, cyano, and nitro;

### Formula XVIII, as disclosed in U.S. Patent Publication 2006/0046956:

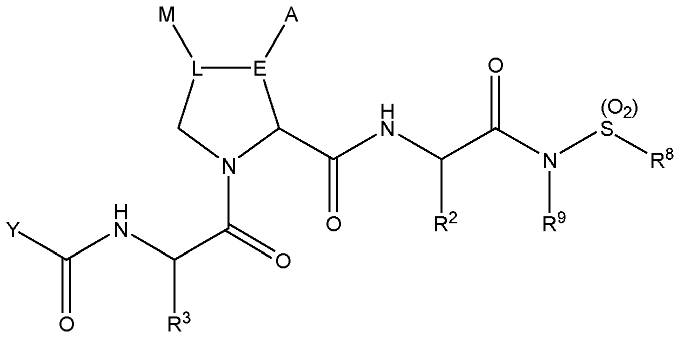

or a pharmaceutically acceptable salt, solvate, or ester thereof; wherein in Formula XVIII:
R⁸ is selected from the group consisting of alkyl-, aryl-, heteroalkyl-, heteroaryl-, cycloalkyl-, heterocyclyl-, arylalkyl-, heteroarylalkyl- , and heterocyclylalkyl;
R⁹ is selected from the group consisting of H, alkyl, alkenyl, alkynyl, aryl and cycloalkyl;
A and M can be the same or different, each being independently selected from R, OR, N(H)R, N(RR'), SR, S(O₂)R, and halo; or A and M are connected to each other (in other words, A-E-L-M taken together) such that the moiety: 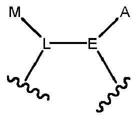 shown above in Formula I forms either a three, four, five, six, seven or eight-membered cycloalkyl, a four to eight-membered heterocyclyl, a six to ten-membered aryl, or a five to ten-membered heteroaryl;
E is C(H) or C(R);
L is C(H), C(R), CH₂C(R), or C(R)CH₂;
R and R' can be the same or different, each being independently selected from the group consisting of H, alkyl-, alkenyl-, alkynyl-, cycloalkyl-, heteroalkyl-, heterocyclyl-, aryl-, heteroaryl-, (cycloalkyl)alkyl-, (heterocyclyl)alkyl-, aryl-alkyl-, and heteroaryl-alkyl-; or alternately R and R' in N(RR') are connected to each other such that N(RR') forms a four to eight-membered heterocyclyl; R² and R³ can be the same or different, each being independently selected from the group consisting of H, alkyl, heteroalkyl, alkenyl, heteroalkenyl, alkynyl, heteroalkynyl, cycloalkyl, spiro-linked cycloalkyl, heterocyclyl, aryl, arylalkyl, heteroaryl, and heteroarylalkyl;
Y is selected from the following moieties: 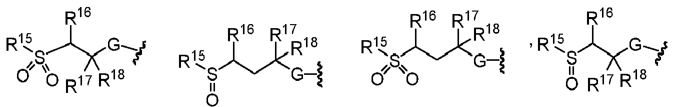 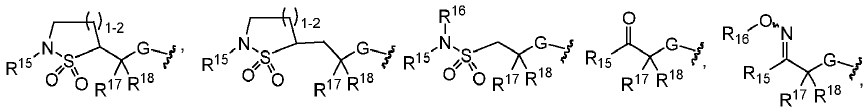 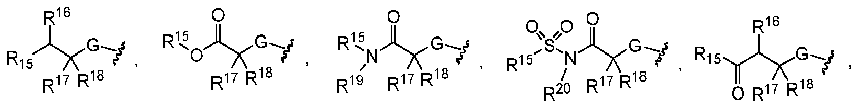 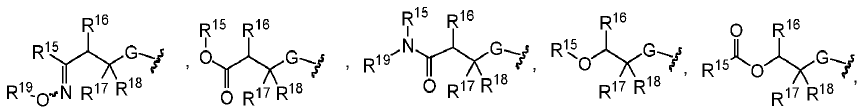 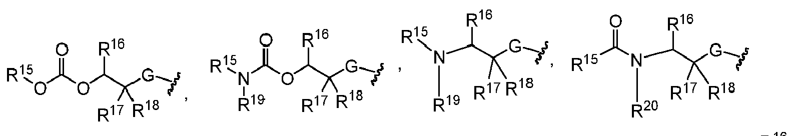 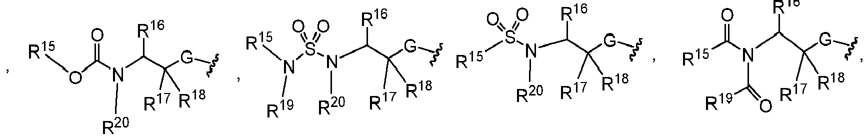 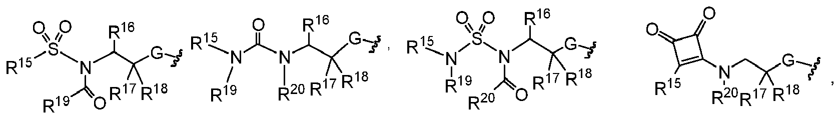 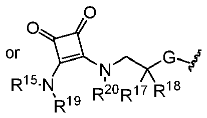 wherein G is NH or O; and R¹⁵, R¹⁶, R¹⁷, R¹⁸, R¹⁹ and R²⁰ can be the same or different, each being independently selected from the group consisting of H, alkyl, heteroalkyl, alkenyl, heteroalkenyl, alkynyl, heteroalkynyl, cycloalkyl, heterocyclyl, aryl, arylalkyl, heteroaryl, and heteroarylalkyl, or alternately (i) R¹⁷ and R¹⁸ are independently connected to each other to form a three to eight-membered cycloalkyl or heterocyclyl; (ii) likewise independently R¹⁵ and R¹⁹ are connected to each other to form a four to eight-membered heterocyclyl; (iii) likewise independently R¹⁵ and R¹⁶ are connected to each other to form a four to eight-membered heterocyclyl; and (iv) likewise independently R¹⁵ and R²⁰ are connected to each other to form a four to eight-membered heterocyclyl;
   wherein each of said alkyl, aryl, heteroaryl, cycloalkyl, spiro-linked cycloalkyl, and heterocyclyl can be unsubstituted or optionally independently substituted with one or more moieties selected from the group consisting of hydroxy, alkoxy, aryloxy, thio, alkylthio, arylthio, amino, amido, alkylamino, arylamino, alkylsulfonyl, arylsulfonyl, sulfonamido, alkyl, alkenyl, aryl, heteroaryl, alkylsulfonamido, arylsulfonamido, keto, carboxy, carbalkoxy, carboxamido, alkoxycarbonylamino, alkoxycarbonyloxy, alkylureido, arylureido, halo, cyano, and nitro;

### Formula XIX, as disclosed in U.S. Patent Publication 2005/0272663:

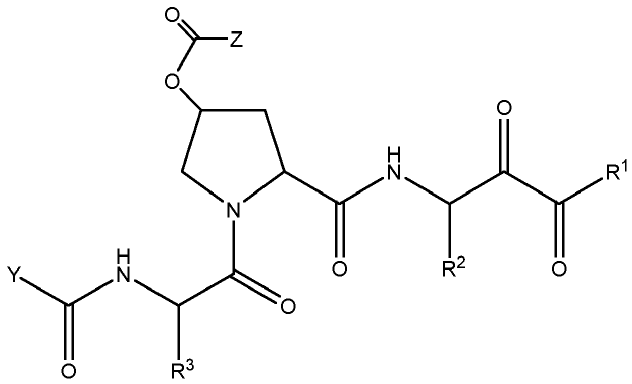

or a pharmaceutically acceptable salt, solvate, or ester thereof; wherein in Formula XIX:
Z is selected from the group consisting of a heterocyclyl moiety, N(H)(alkyl), -N(alkyl)₂, -N(H)(cycloalkyl), -N(cycloalkyl)₂, -N(H)(aryl, -N(aryl)₂, - N(H)(heterocyclyl), -N(heterocyclyl)₂, -N(H)(heteroaryl), and -N(heteroaryl)₂;
R¹ is NHR⁹, wherein R⁹ is H, alkyl-, alkenyl-, alkynyl-, aryl-, heteroalkyl-, heteroaryl-, cycloalkyl-, heterocyclyl-, arylalkyl-, or heteroarylalkyl;
R² and R³ can be the same or different, each being independently selected from the group consisting of H, alkyl, heteroalkyl, alkenyl, heteroalkenyl, alkynyl, heteroalkynyl, cycloalkyl, heterocyclyl, aryl, arylalkyl, heteroaryl, and heteroarylalkyl;
Y is selected from the following moieties: 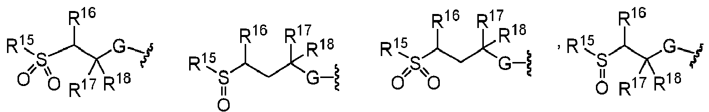 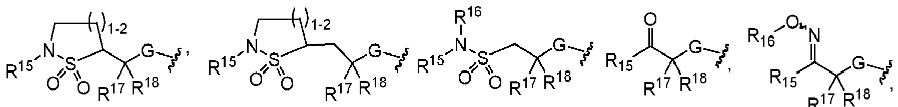 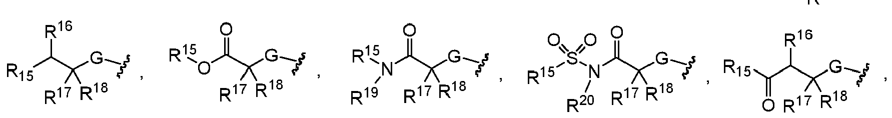 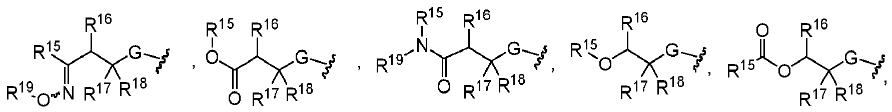 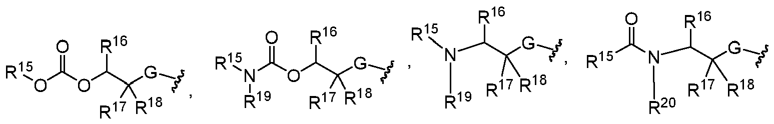 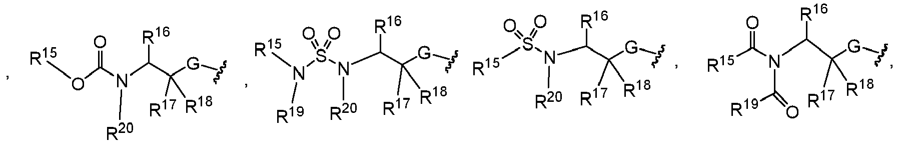 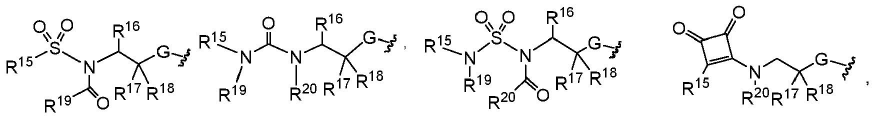 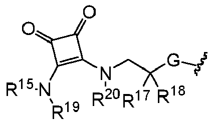
wherein G is NH or O; and R¹⁵, R¹⁶, R¹⁷, R¹⁸, R¹⁹, R²⁰ and R²¹ can be the same or different, each being independently selected from the group consisting of H, alkyl, heteroalkyl, alkenyl, heteroalkenyl, alkynyl, heteroalkynyl, cycloalkyl, heterocyclyl, aryl, arylalkyl, heteroaryl, and heteroarylalkyl, or alternately (i) R¹⁷ and R¹⁸ are independently connected to each other to form a three to eight-membered cycloalkyl or heterocyclyl; (ii) likewise independently R¹⁵ and R¹⁹ are connected to each other to form a four to eight-membered heterocyclyl; (iii) likewise independently R¹⁵ and R¹⁶ are connected to each other to form a four to eight-membered heterocyclyl; and (iv) likewise independently R¹⁵ and R²⁰ are connected to each other to form a four to eight-membered heterocyclyl;
wherein each of said alkyl, aryl, heteroaryl, cycloalkyl or heterocyclyl can be unsubstituted or optionally independently substituted with one or more moieties selected from the group consisting of hydroxy, alkoxy, aryloxy, thio, alkylthio, arylthio, amino, amido, alkylamino, arylamino, alkylsulfonyl, arylsulfonyl, sulfonamido, alkyl, aryl, heteroaryl, alkylsulfonamido, arylsulfonamido, keto, carboxy, carbalkoxy, carboxamido, alkoxycarbonylamino, alkoxycarbonyloxy, alkylureido, arylureido, halo, cyano, and nitro;

### Formula XX, as disclosed in US Patent 6,767,991:

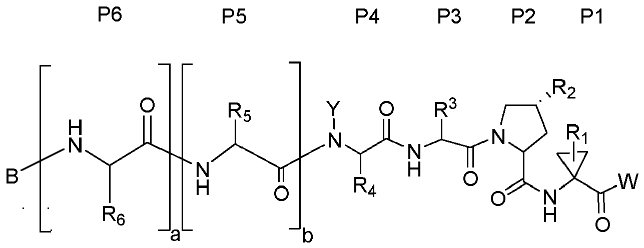

or a pharmaceutically acceptable salt, solvate, or ester thereof; wherein in Formula XX:
a is 0 or 1; b is 0 or 1; Y is H or C₁₋₆ alkyl;
B is H, an acyl derivative of formula R₇-C(O)- or a sulfonyl of formula R₇-SO2 wherein
R7 is (i) C_{I-10} alkyl optionally substituted with carboxyl, C₁₋₆ alkanoyloxy or C₁₋₆ alkoxy,;
   (ii) C₃₋₇ cycloalkyl optionally substituted with carboxyl, (C₁₋₆ alkoxy)carbonyl or phenylmethoxycarbonyl;
   (iii) C₆ or C₁₀ aryl or C₇₋₁₆ aralkyl optionally substituted with C₁₋₆ alkyl, hydroxy, or amino optionally substituted with C₁₋₆ alkyl; or
   (iv) Het optionally substituted with C₁₋₆ alkyl, hydroxy, amino optionally substituted with C₁₋₆ alkyl, or amido optionally substituted with C₁₋₆ alkyl;
R₆, when present, is C₁₋₆ alkyl substituted with carboxyl;
R₅, when present, is C₁₋₆ alkyl optionally substituted with carboxyl;
R₄ is C₁₋₁₀ alkyl, C₃₋₇cycloalkyl or C₄₋₁₀ (alkylcycloalkyl);
R₃ is C₁₋₁₀ alkyl, C₃₋₇ cycloalkyl or C₄₋₁₀ (alkylcycloalkyl);
R₂ is CH₂-R₂₀, NH-R₂₀, 0-R₂₀ or S-R₂₀, wherein R₂₀ is a saturated or unsaturated C₃₋₇ cycloalkyl or C₄₋₁₀ (alkyl cycloalkyl) being optionally mono-, di- or tri-substituted with R₂₁, or R₂₀ is a C₆ or C₁₀ aryl or C₇₋₁₆ aralkyl optionally mono-, di-or tri- substituted with R₂₁,
or R₂₀ is Het or (lower alkyl)-Het optionally mono-, di- or tri- substituted with R₂₁, wherein each R₂₁ is independently C₁₋₆ alkyl; C₁₋₆alkoxy; amino optionally mono-or di-substituted with C₁₋₆ alkyl; sulfonyl; NO₂; OH; SH; halo; haloalkyl; amido optionally mono-substituted with C₁₋₆ alkyl, C₆ or C₁₀ aryl, C₇₋₁₆ aralkyl, Het or (lower alkyl)-Het; carboxyl; carboxy(lower alkyl); C₆ or C₁₀ aryl, C₇₋₁₆ aralkyl or Het, said aryl, aralkyl or Het being optionally substituted with R₂₂; wherein R₂₂ is C₁₋₆alkyl; C₁₋₆ alkoxy; amino optionally mono- or di- substituted with C₁₋₆ alkyl; sulfonyl; NO₂; OH; SH; halo; haloalkyl; carboxyl; amide or (lower alkyl)amide;
R₁ is C₁₋₆ alkyl or C₂₋₆ alkenyl optionally substituted with halogen; and W is hydroxy or a N-substituted amino.

In the above-shown structure of the compound of Formula XX, the terms P6, P5, P4, P3, P2 and P1 denote the respective amino acid moieties as is conventionally known to those skilled in the art;

### Formula XXI, as disclosed in US Patent 6,323,180:

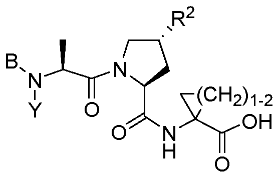

or a pharmaceutically acceptable salt, solvate, or ester thereof; wherein in Formula XXI:
B is H, a C₆ or C₁₀ aryl, C₇₋₁₆ aralkyl; Het or (lower alkyl)- Het, all of which optionally substituted with C₁₋₆ alkyl; C₁₋₆ alkoxy; C₁₋₆ alkanoyl; hydroxy;
hydroxyalkyl; halo; haloalkyl; nitro; cyano; cyanoalkyl; amino optionally substituted with C₁₋₆ alkyl; amido; or (lower alkyl)amide;
   or B is an acyl derivative of formula R₄-C(O)-; a carboxyl of formula R₄-O-C(O)-; an amide of formula R₄-N(R₅)-C(O)-; a thioamide of formula R₄-N(R₅)-C(S)-; or a sulfonyl of formula R₄-SO2 wherein
R₄ is (i) C₁₋₁₀ alkyl optionally substituted with carboxyl, C₁₋₆ alkanoyl, hydroxy, C₁₋₆ alkoxy, amino optionally mono- or di-substituted with C₁₋₆ alkyl, amido, or (lower alkyl) amide;
   (ii) C₃₋₇ cycloalkyl, C₃₋₇ cycloalkoxy, or C₄₋₁₀ alkylcycloalkyl, all optionally substituted with hydroxy, carboxyl, (C₁₋₆ alkoxy)carbonyl, amino optionally mono-or di-substituted with C₁₋₆ alkyl, amido, or (lower alkyl) amide;
   (iii) amino optionally mono- or di-substituted with C₁₋₆ alkyl; amido; or (lower alkyl)amide;
   (iv) C₆ or C₁₀ aryl or C₇₋₁₆ aralkyl, all optionally substituted with C₁₋₆ 6 alkyl, hydroxy, amido, (lower alkyl)amide, or amino optionally mono- or di- substituted with C₁₋₆ alkyl; or
   (v) Het or (lower alkyl)-Het, both optionally substituted with C₁₋₆ alkyl, hydroxy, amido, (lower alkyl) amide, or amino optionally mono- or di-substituted with C₁₋₆ alkyl;
R₅ is H or C₁₋₆ alkyl;
with the proviso that when R₄ is an amide or a thioamide, R₄ is not (ii) a cycloalkoxy;
Y is H or C₁₋₆ alkyl;
R₃ is C₁₋₈ alkyl, C₃₋₇ cycloalkyl, or C₄₋₁₀ alkylcycloalkyl, all optionally substituted with hydroxy, C₁₋₆ alkoxy, C₁₋₆ thioalkyl, amido, (lower alkyl)amido, C₆ or C₁₀ aryl, or C₇₋₁₆ aralkyl;
R₂ is CH₂-R₂₀, NH-R₂₀, O-R₂₀ or S-R₂₀, wherein R₂₀ is a saturated or unsaturated C₃₋₇ cycloalkyl or C₄₋₁₀ (alkylcycloalkyl), all of which being optionally mono-, di- or tri- substituted with R₂₁, or R₂₀ is a C₆ or C₁₀ aryl or C₇₋₁₄ aralkyl, all optionally mono-, di- or tri-substituted with R₂₁,
or R₂₀ is Het or (lower alkyl)-Het, both optionally mono-, di- or tri- substituted with R₂₁,
wherein each R₂₁ is independently C₁₋₆alkyl; C₁₋₆ alkoxy; lower thioalkyl; sulfonyl; NO₂; OH; SH; halo; haloalkyl; amino optionally mono- or di- substituted with C₁₋₆ alkyl, C₆ or C₁₀ aryl, C₇₋₁₄ aralkyl, Het or (lower alkyl)-Het; amido optionally mono-substituted with C₁₋₆ alkyl, C₆ or C₁₀ aryl, C₇₋₁₄ aralkyl, Het or (lower alkyl)-Het; carboxyl; carboxy(lower alkyl); C₆ or C₁₀ aryl, C₇₋₁₄ aralkyl or Het, said aryl, aralkyl or Het being optionally substituted with R₂₂;
wherein R₂₂ is C₁₋₆ alkyl; C₃₋₇ cycloalkyl; C₁₋₆ alkoxy; amino optionally mono- or di-substituted with C₁₋₆ alkyl; sulfonyl; (lower alkyl)sulfonyl; N0₂; OH; SH; halo; haloalkyl; carboxyl; amide; (lower alkyl)amide; or Het optionally substituted with C₁₋₆ alkyl;
R1 is H; C₁₋₆ alkyl, C₃₋₇ cycloalkyl, C₂₋₆ alkenyl, or C₂₋₆ alkynyl, all optionally substituted with halogen;

### Formula XXII, as disclosed in U.S. Patent No. 6,608,027:

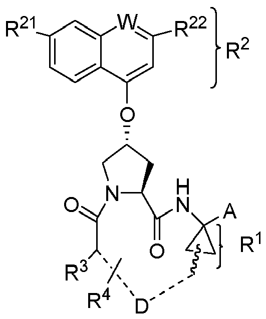

or a pharmaceutically acceptable salt, solvate, or ester thereof; wherein in Formula XXII:
W is CH or N,
R²¹ is H, halo, C₁₋₆ alkyl, C₃₋₆ cycloalkyl, C₁₋₆ haloalkyl, C₁₋₆ alkoxy, C₃₋₆ cycloalkoxy, hydroxy, or N(R²³)₂ , wherein each R²³ is independently H, C₁₋₆ alkyl or C₃₋₆ cycloalkyl;
R²² is H, halo, C₁₋₆ alkyl, C₃₋₆ cycloalkyl, C₁₋₆ haloalkyl, C₁₋₆ thioalkyl, C₁₋₆ alkoxy, C₃₋₆ cycloalkoxy, C₂₋₇ alkoxyalkyl, C₃₋₆cycloalkyl, C_{6 or 10} aryl or Het, wherein Het is a five-, six-, or seven-membered saturated or unsaturated heterocycle containing from one to four heteroatoms selected from nitrogen, oxygen and sulfur;
said cycloalkyl, aryl or Het being substituted with R²⁴, wherein R²⁴ is H, halo, C₁₋₆ alkyl, C₃₋₆ cycloalkyl, C₁₋₆ alkoxy, C₃₋₆ cycloalkoxy, NO₂ , N(R²⁵)₂ , NH-C(O)-R²⁵ or NH-C(O)-NH-R²⁵, wherein each R²⁵ is independently: H, C₁₋₆ alkyl or C₃₋₆ cycloalkyl;
or R²⁴ is NH-C(O)-OR²⁶ wherein R²⁶ is C₁₋₆ alkyl or C₃₋₆ cycloalkyl; R³ is hydroxy, NH₂ , or a group of formula -NH-R³¹, wherein R³¹ is C_{6 or 10} aryl, heteroaryl, -C(O)-R³², -C(O)-NHR³² or -C(O)-OR³² , wherein R³² is C₁₋₆ alkyl or C₃₋₆ cycloalkyl;
D is a 5 to 10-atom saturated or unsaturated alkylene chain optionally containing one to three heteroatoms independently selected from: O, S, or N-R⁴¹ , wherein R⁴¹ is H, C₁₋₆ alkyl, C₃₋₆ cycloalkyl or -C(O)-R⁴², wherein R⁴² is C₁₋₆ alkyl, C₃₋₆ cycloalkyl or C_{6 or 10} aryl; R⁴ is H or from one to three substituents at any carbon atom of said chain D, said substituent independently selected from the group consisting of: C₁₋₆ alkyl, C₁₋₆ haloalkyl, C₁₋₆ alkoxy, hydroxy, halo, amino, oxo, thio and C 1-6 thioalkyl, and
A is an amide of formula -C(O)-NH-R⁵, wherein R⁵ is selected from the group consisting of: C₁₋₈ alkyl, C₃₋₆ cycloalkyl, C₆ or ₁₀ aryl and C₇₋₁₆ aralkyl; or A is a carboxylic acid;

### Formula XXIII, as disclosed in International Patent Publication WO02/18369:

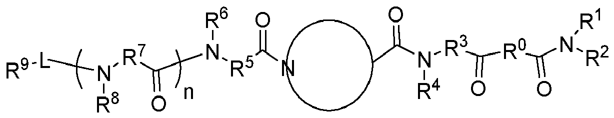

or a pharmaceutically acceptable salt, solvate, or ester thereof; wherein in Formula XXIII:
R⁰ is a bond or difluoromethylene;
R¹ is hydrogen;
R² and R⁹ are each independently optionally substituted aliphatic group, optionally substituted cyclic group or optionally substituted aromatic group; R3, R5 and R7 are each independently:
   optionally substituted (1,1- or 1,2-)cycloalkylene; or
   optionally substituted (1,1- or 1,2-) heterocyclylene; or
   methylene or ethylene), substituted with one substituent selected from the group consisting of an optionally substituted aliphatic group, an optionally substituted cyclic group or an optionally substituted aromatic group, and wherein the methylene or ethylene is further optionally substituted with an aliphatic group substituent; or;
R4, R6, R8 and R¹⁰ are each independently hydrogen or optionally substituted aliphatic group; 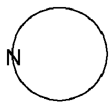 is substituted monocyclic azaheterocyclyl or optionally substituted multicyclic azaheterocyclyl, or optionally substituted multicyclic azaheterocyclenyl wherein the unsaturatation is in the ring distal to the ring bearing the R⁹-L-(N(R⁸)-R⁷-C(O)-)ₙN(R⁶)-R⁵-C(O)-N moiety and to which the - C(O)-N(R⁴)-R³-C(O)C(O)NR²R¹ moiety is attached; L is -C(O)-, -OC(O)-, - NR¹⁰C(O)-, -S(0)₂-, or - NR¹⁰S(0)₂-; and n is 0 or 1, provided
   when 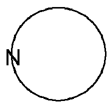 is substituted 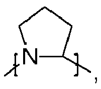 then L is -OC(O)- and R⁹ is optionally substituted aliphatic; or at least one of R³, R⁵ and R⁷ is ethylene, substituted with one substituent selected from the group consisting of an optionally substituted aliphatic group, an optionally substituted cyclic group or an optionally substituted aromatic group and wherein the ethylene is further optionally substituted with an aliphatic group substituent; or R⁴ is optionally substituted aliphatic;

### Formula XXIV, as disclosed in U.S. Patent No. 6,265,380:

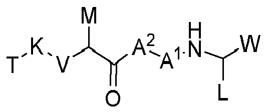

or a pharmaceutically acceptable salt, solvate, or ester thereof; wherein in Formula XXIV:
W is: 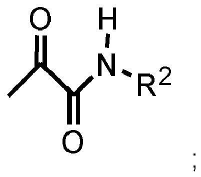
m is 0 or 1;
R² is hydrogen, alkyl, alkenyl, aryl, aralkyl, aralkenyl, cycloalkyl, cycloalkylalkyl, cycloalkenyl, cycloalkenylalkyl, heterocyclyl, heterocyclylalkyl, heterocyclylalkenyl, heteroaryl, or heteroaralkyl; wherein any R² carbon atom is optionally substituted with J;
J is alkyl, aryl, aralkyl, alkoxy, aryloxy, aralkoxy, cycloalkyl, cycloalkoxy, heterocyclyl, heterocyclyloxy, heterocyclylalkyl, keto, hydroxy, amino, alkylamino, alkanoylamino, aroylamino, aralkanoylamino, carboxy, carboxyalkyl, carboxamidoalkyl, halo, cyano, nitro, formyl, acyl, sulfonyl, or sulfonamido and is optionally substituted with 1-3 J¹ groups;
J¹ is alkyl, aryl, aralkyl, alkoxy, aryloxy, heterocyclyl, heterocyclyloxy, keto, hydroxy, amino, alkanoylamino, aroylamino, carboxy, carboxyalkyl, carboxamidoaikyl, halo, cyano, nitro, formyl, sulfonyl, or sulfonamido;
L is alkyl, alkenyl, or alkynyl, wherein any hydrogen is optionally substituted with halogen, and wherein any hydrogen or halogen atom bound to any terminal carbon atom is optionally substituted with sulfhydryl or hydroxy;
A¹ is a bond;
R⁴ is alkyl, cycloalkyl, aryl, aralkyl, heterocyclyl, heterocyclylalkyl, heteroaryl, heteroaralkyl, carboxyalkyl, or carboxamidoalkyl, and is optionally substituted with 1-3 J groups;
R⁵ and R⁶ are independently hydrogen, alkyl, alkenyl, aryl, aralkyl, aralkenyl, cycloalkyl, cycloalkylalkyl, cycloalkenyl, heterocyclyl, heterocyclylalkyl, heteroaryl, or heteroaralkyl, and is optionally substituted with 1-3 J groups;
X is a bond, -C(H)(R7)-, -0-, - S-, or -N(R8)-;
R⁷ is hydrogen, alkyl, alkenyl, aryl, aralkyl, heterocyclyl, heterocyclylalkyl, heteroaryl, or heteroaralkyl, and is optionally substititued with 1-3 J groups;
R⁸ is hydrogen alkyl, aryl, aralkyl, heterocyclyl, heterocyclylalkyl, heteroaryl, heteroaralkyl, aralkanoyl, heterocyclanoyl, heteroaralkanoyl, - C(O)R¹⁴, -SO₂R¹⁴, or carboxamido, and is optionally substititued with 1-3 J groups; or R⁸ and Z, together with the atoms to which they are bound, form a nitrogen containing mono- or bicyclic ring system optionally substituted with 1-3 J groups;
R¹⁴ is alkyl, aryl, aralkyl, heterocyclyl, heterocyclyalkyl, heteroaryl, or heteroaralkyl;
Y is a bond, -CH₂-, -C(O)-, -C(O)C(O)-, - S(O)-, -S(0)₂-, or -S(O)(NR⁷)-, wherein R⁷ is as defined above;
Z is alkyl, aryl, aralkyl, cycloalkyl, cycloalkylalkyl, heterocyclyl, heterocyclylalkyl, heteroaryl, heteroaralkyl, -OR², or -N(R²)₂, wherein any carbon atom is optionally substituted with J, wherein R² is as defined above;
A² is a bond or 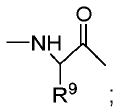
R⁹ is alkyl, cycloalkyl, aryl, aralkyl, heterocyclyl, heterocyclylalkyl, heteroaryl, heteroaralkyl, carboxyalkyl, or carboxamidoalkyl, and is optionally substituted with 1-3 J groups;
M is alkyl, cycloalkyl, aryl, aralkyl, heterocyclyl, heterocyclylalkyl, heteroaryl, or heteroaralkyl, optionally substituted by 1-3 J groups, wherein any alkyl carbon atom may be replaced by a heteroatom;
V is a bond, -CH₂-, -C(H)(R¹¹)-, -0-, -S-, or -N(R¹¹)-;
R¹¹ is hydrogen or C₁₋₃alkyl;
K is a bond, -0-, -S-, -C(O)-, -S(O)-, -S(0)₂-, or -S(O)(NR¹¹)-, wherein R¹¹ is as defined above;
T is -R¹², -alkyl-R¹², -alkenyl-R¹², - alkynyl-R¹², -OR¹², -N(R¹²)₂, -C(O)R¹², -C(=NOalkyl)R¹², or 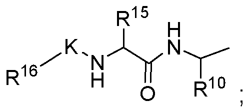
R¹² is hydrogen, aryl, heteroaryl, cycloalkyl, heterocyclyl, cycloalkylidenyl, or heterocycloalkylidenyl, and is optionally substituted with 1-3 J groups, or a first R¹² and a second R¹², together with the nitrogen to which they are bound, form a mono- or bicyclic ring system optionally substituted by 1-3 J groups;
R¹⁰ is alkyl, cycloalkyl, aryl, aralkyl, heterocyclyl, heterocyclylalkyl, heteroaryl, heteroaralkyl, carboxyalkyl, or carboxamidoalkyl, and is optionally substituted with 1-3 hydrogens J groups;
R¹⁵ is alkyl, cycloalkyl, aryl, aralkyl, heterocyclyl, heterocyclylalkyl, heteroaryl, heteroaralkyl, carboxyalkyl, or carboxamidoalkyl, and is optionally substituted with 1-3 J groups; and
R¹⁶ is hydrogen, alkyl, aryl, heteroaryl, cycloalkyl, or heterocyclyl;

### Formula XXV, as disclosed in International Patent Publication WO1998/22496:

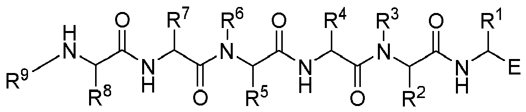

or a pharmaceutically acceptable salt, solvate, or ester thereof; wherein in Formula XXV:
E represents CHO or B(OH)₂;
R¹ represents lower alkyl, halo-lower alkyl, cyano-lower alkyl, lower alkylthio-lower alkyl, aryl-lower alkylthio-lower alkyl, aryl-lower alkyl, heteroaryllower alkyl, lower alkenyl or lower alkynyl;
R² represents lower alkyl, hydroxy-lower alkyl, carboxylower alkyl, aryl-lower alkyl, aminocarbonyl-lower alkyl or lower cycloalkyl-lower alkyl; and
R³ represents hydrogen or lower alkyl;
or R² and R³ together represent di- or trimethylene optionally substituted by hydroxy;
R⁴ represents lower alkyl, hydroxy-lower alkyl, lower cycloalkyl-lower alkyl, carboxy-lower alkyl, aryllower alkyl, lower alkylthio-lower alkyl, cyano-lower alkylthio-lower alkyl, aryl-lower alkylthio-lower alkyl, lower alkenyl, aryl or lower cycloalkyl;
R⁵ represents lower alkyl, hydroxy-lower alkyl, lower alkylthio-lower alkyl, aryl-lower alkyl, aryl-lower alkylthio-lower alkyl, cyano-lower alkylthio-lower alkyl or lower cycloalkyl;
R⁶ represents hydrogen or lower alkyl;
R⁷ represent lower alkyl, hydroxydower alkyl, carboxylower alkyl, aryl-iower alkyl, lower cycloalkyl-lower alkyl or lower cycloalkyl;
R⁸ represents lower alkyl, hydroxy-lower alkyl, carboxylower alkyl or aryl-lower alkyl; and
R⁹ represents lower alkylcarbonyl, carboxy-lower alkylcarbonyl, arylcarbonyl, lower alkylsulphonyl, arylsulphonyl, lower alkoxycarbonyl or aryl-lower alkoxycarbonyl;

### Formula XXVI, as disclosed in U.S. Patent No. 6,143,715:

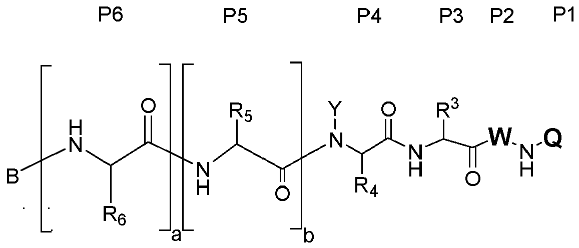

or a pharmaceutically acceptable salt, solvate, or ester thereof; wherein in Formula XXVI:
B is an acyl derivative of formula R₁₁-C(O)- wherein R₁₁ is Cl-10 alkyl optionally substituted with carboxyl; or R₁₁ is C₆ or C₁₀ aryl or C₇₋₁₆ aralkyl optionally substituted with a C₁₋₆ alkyl;
a is 0 or 1;
R₆, when present, is carboxy(lower)alkyl;
b is 0 or 1;
R₅, when present, is C₁₋₆ alkyl, or carboxy(lower)alkyl;
Y is H or C₁₋₆ alkyl;
R₄ is C₁₋₁₀ alkyl; C₃₋₁₀ cycloalkyl;
R₃ is C1-10 alkyl; C₃₋₁₀ cycloalkyl;
W is a group of formula: 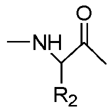
wherein R₂ is C₁₋₁₀ alkyl or C₃₋₇ cycloalkyl optionally substituted with carboxyl; C₆ or C₁₀ aryl; or C₇₋₁₆ aralkyl; or
W is a group of formula: 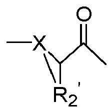 wherein X is CH or N; and
R₂' is C₃₋₄ alkylene that joins X to form a 5- or 6-membered ring, said ring optionally substituted with OH; SH; NH2; carboxyl; R₁₂; OR₁₂, SR₁₂, NHR₁₂ or NR₁₂R₁₂' wherein R₁₂ and R₁₂' are independently:
cyclic C₃₋₁₆ alkyl or acyclic C₁₋₁₆ alkyl or cyclic C₃₋₁₆ alkenyl or acyclic C₂₋₁₆ alkenyl, said alkyl or alkenyl optionally substituted with NH₂, OH, SH, halo, or carboxyl; said alkyl or alkenyl optionally containing at least one heteroatom selected independently from the group consisting of: 0, S, and N; or
R₁₂ and R₁₂' are independently C₆ or C₁₀ aryl or C₇₋₁₆ aralkyl optionally substituted with C₁₋₆ alkyl, NH₂, OH, SH, halo, carboxyl or carboxy(lower)alkyl; said aryl or aralkyl optionally containing at least one heteroatom selected independently from the group consisting of: 0, S, and N;
said cyclic alkyl, cyclic alkenyl, aryl or aralkyl being optionally fused with a second 5-, 6-, or 7-membered ring to form a cyclic system or heterocycle, said second ring being optionally substituted with NH₂. OH, SH, halo, carboxyl or carboxy(lower)alkyl; C₆ or C₁₀ aryl, or heterocycle; said second ring optionally containing at least one heteroatom selected independently from the group consisting of: 0, S, and N;
Q is a group of the formula: 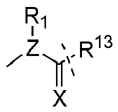 wherein Z is CH;
X is 0 or S;
R₁ is H, C₁₋₆ alkyl or C₁₋₆ alkenyl both optionally substituted with thio or halo;
and
R₁₃ is C0-NH-R₁₄ wherein R₁₄ is hydrogen, cyclic C₃₋₁₀ alkyl or acyclic C₁₋₁₀ alkyl or cyclic C₃₋₁₀ alkenyl or acyclic C₂₋₁₀ alkenyl, said alkyl or alkenyl optionally substituted with NH₂, OH, SH, halo or carboxyl; said alkyl or alkenyl optionally containing at least one heteroatom selected independently from the group consisting of: 0, S, and N; or
R₁₄ is C₆ or C₁₀ aryl or C₇₋₁₆ aralkyl optionally substituted with C₁₋₆ alkyl, NH₂, OH, SH, halo, carboxyl or carboxy(lower)alkyl or substituted with a further C₃₋₇ cycloalkyl, C₆ or C₁₀ aryl, or heterocycle; said aryl or aralkyl optionally containing at least one heteroatom selected independently from the group consisting of: 0, S, and N;
said cyclic alkyl, cyclic alkenyl, aryl or aralkyl being optionally fused with a second 5-, 6-, or 7-membered ring to form a cyclic system or heterocycle, said second ring being optionally substituted with NH₂, OH, SH, halo, carboxyl or carboxy(lower)alkyl or substituted with a further C₃₋₇ cycloalkyl, C₆ or C₁₀ aryl, or heterocycle; said second ring optionally containing at least one heteroatom selected independently from the group consisting of: 0, S, and N;
with the proviso that when Z is CH, then R₁₃ is not an α-amino acid or an ester thereof;
Q is a phosphonate group of the formula: 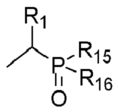 wherein R₁₅ and R₁₆ are independently C₆₋₂₀ aryloxy; and R₁ is as defined above;

In the above-shown structure of the compound of Formula XXVI, the terms P6, P5, P4, P3, P2 and P1 denote the respective amino acid moieties as is conventionally known to those skilled in the art. Thus, the actual structure of the compound of Formula XXVI is:

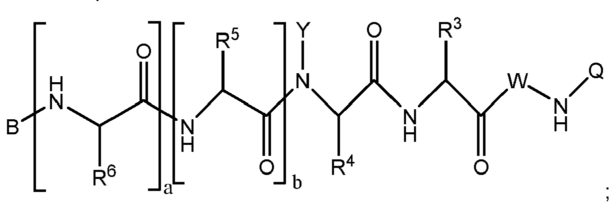

### Formula XXVII, as disclosed in International Patent Publication WO02/18369:

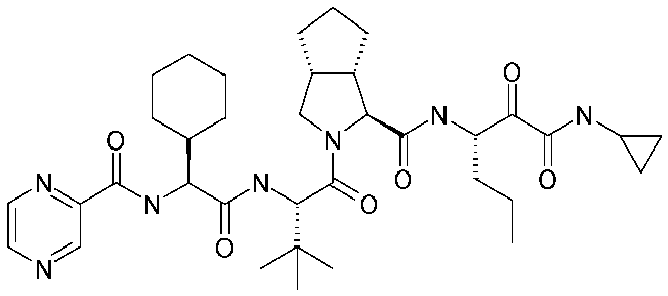

or a pharmaceutically acceptable salt, solvate, or ester thereof; and

### Formula XXVIII, as disclosed in International Patent Publication WO02/18369

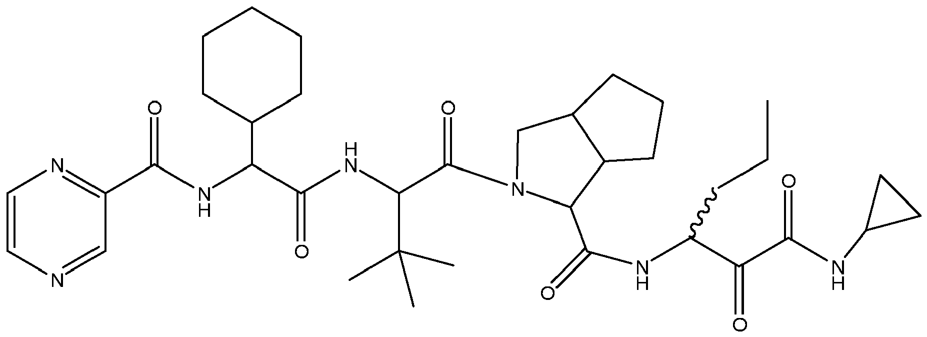

,
or a pharmaceutically acceptable salt, solvate, or ester thereof.

The disclosures of the publications cited above for compounds of Formulae I to XXVIII are incorporated herein by reference in their entirety.

Specific embodimenrts of compounds of Formula I are compounds 1 and 2: 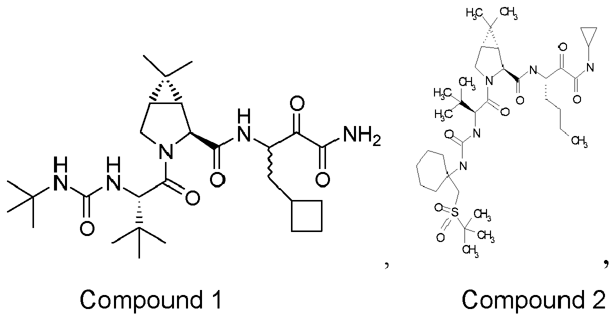 or a pharmaceutically acceptable isomer, salt, solvate or ester therof.

A specific embodiment of the compound of structural Formula XXVIII is compound 3: 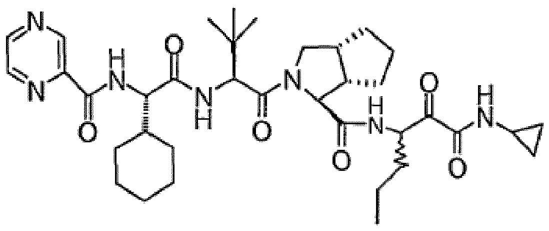 or a pharmaceutically acceptable salt, solvate, or ester thereof.

Isomers of the various therapeutic agents used in the formulations of the present invention (where they exist), including enantiomers, stereoisomers, rotamers, tautomers and racemates are also envisaged as being useful for the formulations of this invention. Polymorphous forms of the therapeutic agents used in the formulations of the present invention, whether crystalline or amorphous, also are useful for the formulations of this invention.

Unless otherwise stated, structures depicted herein are also meant to include compounds which differ only in the presence of one or more isotopically enriched atoms. For example, therapeutic agents having the present structures except for the replacement of a hydrogen by a deuterium or tritium, or the replacement of a carbon by a ¹³C- or ¹⁴C-enriched carbon are also within the scope of this invention.

Forms of the therapeutic agencs including prodrugs and solvates are useful in the formulations of the invention. A discussion of prodrugs is provided in T. Higuchi and V. Stella, Pro-drugs as Novel Delivery Systems (1987) 14 of the A.C.S. Symposium Series, and in Bioreversible Carriers in Drug Design, (1987) Edward B. Roche, ed., American Pharmaceutical Association and Pergamon Press. The term "prodrug" means a compound (e.g, a drug precursor) that is transformed *in vivo* to yield a compound that is therapeutically active.. The transformation may occur by various mechanisms (e.g., by metabolic or chemical processes), such as, for example, through hydrolysis in blood.

"Solvate", in connection with a therapeutic agent means a physical association of a therapeutic agent with one or more solvent molecules. This physical association may involve a varying degree of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolatable solvates. Non-limiting examples of suitable solvates include ethanolates, methanolates, and the like. "Hydrate" is a solvate wherein the solvent molecule is H₂O.

The therapeutic agents used in the formulations of the present invention can form salts that are also useful for formulations within the scope of this invention. The term "salt(s)", as employed herein, denotes acidic salts formed with inorganic and/or organic acids, as well as basic salts formed with inorganic and/or organic bases. In addition, when a therapeutic agent contains both a basic moiety, such as, but not limited to a pyridine or imidazole, and an acidic moiety, such as, but not limited to a carboxylic acid, zwitterions ("inner salts") may be formed and are included within the term "salt(s)" as used herein. Pharmaceutically acceptable (i.e., non-toxic, physiologically acceptable) salts are preferred, although other salts are also useful. Salts of a therapeutic agent may be formed, for example, by reacting the therapeutic agent with an amount of acid or base, such as an equivalent amount, in a medium such as one in which the salt precipitates or in an aqueous medium followed by lyophilization. Acids (and bases) which are generally considered suitable for the formation of pharmaceutically useful salts from basic (or acidic) pharmaceutical compounds are discussed, for example, by S. Berge et al, Journal of Pharmaceutical Sciences (1977) 66(1) 1-19; P. Gould, International J. of Pharmaceutics (1986) - 33 201-217; Anderson et al, The Practice of Medicinal Chemistry (1996), Academic Press, New York; in The Orange Book (Food & Drug Administration, Washington, D.C. on their website); and P. Heinrich Stahl, Camille G. Wermuth (Eds.), Handbook of Pharmaceutical Salts: Properties, Selection, and Use, (2002) Int'l. Union of Pure and Applied Chemistry, pp. 330-331. These disclosures are incorporated herein by reference..

Exemplary acid addition salts include acetates, adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides, hydrobromides, hydroiodides, 2-hydroxyethanesulfonates, lactates, maleates, methanesulfonates, methyl sulfates, 2-naphthalenesulfonates, nicotinates, nitrates, oxalates, pamoates, pectinates, persulfates, 3-phenylpropionates, phosphates, picrates, pivalates, propionates, salicylates, succinates, sulfates, sulfonates (such as those mentioned herein), tartarates, thiocyanates, toluenesulfonates (also known as tosylates,) undecanoates, and the like.

Exemplary basic salts include ammonium salts, alkali metal salts such as sodium, lithium, and potassium salts, alkaline earth metal salts such as calcium and magnesium salts, aluminum salts, zinc salts, salts with organic bases (for example, organic amines) such as benzathines, diethylamine, dicyclohexylamines, hydrabamines (formed with N,N-bis(dehydroabietyl) ethylenediamine), N-methyl-D-glucamines, N-methyl-D-glucamides, t-butyl amines, piperazine, phenylcyclohexylamine, choline, tromethamine, and salts with amino acids such as arginine, lysine and the like. Basic nitrogen-containing groups may be quarternized with agents such as lower alkyl halides (e.g. methyl, ethyl, propyl, and butyl chlorides, bromides and iodides), dialkyl sulfates (e.g. dimethyl, diethyl, dibutyl, and diamyl sulfates), long chain halides (e.g. decyl, lauryl, myristyl and stearyl chlorides, bromides and iodides), aralkyl halides (e.g. benzyl and phenethyl bromides), and others.

All such acid salts and base salts are intended to be useful pharmaceutically acceptable salts within the scope of the invention.

Pharmaceutically acceptable esters of the therapeutic agents used in the formulations of the invention include the following groups: (1) carboxylic acid esters obtained by esterification of the hydroxy groups, in which the non-carbonyl moiety of the carboxylic acid portion of the ester grouping is selected from straight or branched chain alkyl (for example, acetyl, n-propyl, t-butyl, or n-butyl), alkoxyalkyl (for example, methoxymethyl), aralkyl (for example, benzyl), aryloxyalkyl (for example, phenoxymethyl), aryl (for example, phenyl optionally substituted with, for example, halogen, C₁₋₄alkyl, or C₁₋₄alkoxy or amino); (2) sulfonate esters, such as alkyl- or aralkylsulfonyl (for example, methanesulfonyl); (3) amino acid esters (for example, L-valyl or L-isoleucyl); (4) phosphonate esters and (5) mono-, di- or triphosphate esters. The phosphate esters may be further esterified by, for example, a C₁₋₂₀ alcohol or reactive derivative thereof, or by a 2,3-di (C₆₋₂₄)acyl glycerol.

In such esters, unless otherwise specified, any alkyl moiety present preferably contains from 1 to 18 carbon atoms, particularly from 1 to 6 carbon atoms, more particularly from 1 to 4 carbon atoms. Any cycloalkyl moiety present in such esters preferably contains from 3 to 6 carbon atoms. Any aryl moiety present in such esters preferably comprises a phenyl group.

In one embodiment, wherein the therapeutic agent is the HCV protease inhibitor of compound 1 or 2 or a pharmaceutically acceptable salt, solvate, or ester thereof, or a mixture of two or more thereof, the HCV protease inhibitor is administered at a dosage range of about 1920 mg to about 4000 mg per day, preferably about 1920 mg to about 3000 mg per day or about 2560 mg to about 4000 mg per day.

In one embodiment, wherein the therapeutic agent is a HCV protease inhibitor of compound 3, or a pharmaceutically acceptable salt, solvate, or ester thereof, or a mixture of two or more thereof, the HCV protease inhibitor is administered at a dosage range of about 1080 mg to about 3125 mg per day, preferably about 1800 to about 2813 mg per day.

The formulations of the invention may further comprise one or more pharmaceutically acceptable adjuvants and pharmaceutically acceptable carriers and excipients. Each excipient must be acceptable in the sense of being compatible with the other ingredients of the formulation and not injurious to the mammal in need of treatment.

In one embodiment, the adjuvant is at least one pharmaceutically acceptable surfactant. When desired or needed, suitable carriers and other excipients (such as fillers, binders, wetting agents, glidants, lubricants, and disintegrants) may also be incorporated in the formulation. These adjuvants, carriers and excipients as well as others are described hereinafter.

The expanding compositions of the invention may also include one or more distintegrants. In a preffered embodiment of the invention the disintegrant is a superdisintegrant, which is a disintegrant that expands upon contact with water. Preferred superdisintegrants of the present invention expand to at least double their non-hydrated volume on contact with water. Exemplary of these disintegrants are cross-linked carboxymethyl cellulose sodium (a.k.a. croscarmellose sodium), sodium starch glycolate and cross-linked polyvinyl pyrollidone (a.k.a. crospovidone) and low substituted hydroxypropyl cellulose (e.g., L-HPC manufactured by Shin-Etsu Chemical Co., Ltd.). Croscarmellose sodium is commercially available from FMC Corp. under the tradename Ac-Di-Sol® and from Avebe Corp. under the tradename Primellose®. Sodium starch glycolate is commercially available from Penwest Pharmaceuticals Co. under the tradename Explotab® and from Avebe Corp. under the tradename Primojel®. Crospovidone is commercially available from BASF Corp. under the tradename Kollidon® CL and from International Specialty Chemicals Corp. under the tradename Polyplasdone®. Low substituted hydroxypropyl cellulose (L-HPC) is available from Shin Etsu Chemical Company (LH-B1 and LH 21).

These superdisintegrants may be used alone or in combination in the pharmaceutical formulations of the present invention in a total amount of about 1 to about 60% by weight, preferably about 3 to about 50%, more preferably about 10 to about 35% by weight.

Surfactant refers to an adjuvant material that reduces the contact angle of the therapeutic agent and may also be referred to as a wetting agent. The surfactant in the pharmaceutical formulations of the present invention enhances wetting of the therapeutic agent and improves the dissolution rate of the formulation to render a greater quantity of the therapeutic agent available more quickly for absorption than is available in a formulation of the present therapeutic agent that does not include a surfactant. Any pharmaceutically acceptable surfactant that improves wetting of the present compounds may be used. Particularly suitable surfactants include sodium lauryl sulfate, stearic acid, monoethanolamine, docusate sodium, sorbitan fatty acid esters, polyoxyethylene sorbitan fatty acid esters, ethoxylated aliphatic alcohols, propylene glycol monocaprylate, glycerol monostearate, medium chain triglycerides, polyoxyethylene alkyl ethers, and polyoxyethylene stearates. In one embodiment, the surfactant is sodium lauryl sulfate. In another embodiment, the surfactant is a polyoxyethylene sorbitan fatty acid ester. In yet another embodiment, the surfactant is PEG-1-PEG-9-lauryl glycol ether.

The surfactants may be used alone in or combination in the pharmaceutical formulations of the present invention in a total amount of about 0.1 to about 0.5% by weight.

Binders refers to substances that bind or "glue" powders together and make them cohesive by forming granules, thus serving as an "adhesive" in the formulation. Suitable binders include sugars such as lactose, sucrose and corn sweeteners; starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropylmethylcellulose; polyvinylpyrrolidone; polyethylene glycol; waxes and inorganics such as magnesium aluminum silicate.

The amount of binder if present in the formulation can range from about 0.5 to about 35% by weight of the total formulation, or preferably at about 0.5 to about 25% by weight, or more preferably at about 1 to about 10% by weight, or at about 1 to about 5% by weight.

Glidants refer to material that prevents caking and improves the flow characteristics of granulations, so that flow is smooth and uniform. Suitable glidants include silicon dioxide and talc. The amount of glident if present in the formulation can range from about 0.1% to about 5% by weight of the total formulation, or from about 0.5 to about 3% by weight.

Lubricants are substances added to the formulation to enable the tablet, granules, etc. after it has been compressed, to release from the mold or die.

The rate of dissolution of the formulations of the invention can range suitably to generally allow the dissolution of from about 25 % of the drug in the first 5-6 hours to about 90% of the drug in the first 5-6 hours, preferably from about 30 % of the drug in the first 5-6 hours to about 90% of the drug in the first 5-6 hours following ingestion. In a preferred embodiment of the invention, the formulations of the invention have a rate of dissolution depicted in any of Figures 2, 3, 6, 7 and 8 of the present specification.

Dissolution can be determined according to standard USP procedures well known to those skilled in the art. A non-limiting example of a suitable procedure for determining dissolution is described in the following table:

**Table P**

| Dissolution Procedure | |
|---|---|
| Apparatus | USP Apparatus 2 (Paddles):50 ror 100 rpm paddle speed |
| Dissolution | 900 ml of pH 1.2 HCl media, with or without 0.5 % |
| Medium | Tween 80, for 3-4 hours, followed by 900 ml of phosphate buffer at pH 6.8, with or without 0.5% Tween 80, for 2 hours |
| Temperature | 37°C |
| Detection | HPLC with UV detector or UV spectrometer |

The novel expanding formulations of the invention can be prepared by dry blending, dry granulation or wet granulation or other manufacturing process known to those of skill in the art.

In dry granulation, the ingredients are blended dry and then compacted into a slug or a sheet and then comminuted into compacted granules. It will be appreciated that the processes of slugging or roller compaction, followed by comminution and recompression render the swellable polymer, optional disintegrant and the therapeutic agent intragranular in the final dosage form. The therapeutic agent may also be provided intragranularly by blending it with the swellable polymer prior to compaction. The granulate may be used to prepare a dosage form by any of the means known to those of skill in the art.

In wet granulation, the ingredients may be granulated using water, a water:alcohol mixture or an alcohol as a granulation solvent with or without binder in it by standard granulation techniques known in the art. The granulate may then be dried and optionally milled and sieved. After drying, the granulate prepared by wet granulation may be used to prepare a dosage form by any of the means known to those of skill in the art.

The formulation may be compacted following conventional compression and/or direct compression techniques. Direct compression produces a more uniform tablet without granulation. Thus the polymers, a therapeutic agent and other desired excipients are blended prior to direct compression tableting. Optional excipients that are particularly well suited for direct compression tableting include microcrystalline cellulose, spray dried lactose, dicalcium phosphate dihydrate and colloidal silica.

In certain embodiments of the inventions, additional control of release of the therapeutic agent may be provided by further applying a coating. In certain embodiments, the coating is applied to the therapeutic agent; in other embodiments, the coating is applied to the formulation containing the therapeutic agent; and in yet other embodiments, the coating is applied to both the therapeutic agent and to the formulation.

One gastric retention dosage form embodiment is a tablet which may be prepared by compacting the swellable polymer, therapeutic agent, and, optionally, other excipients, as a powder blend or granulate in any type of tableting equipment known to the pharmaceutical arts. Dosage forms of the present invention may be made in any shape desired. Ovoid or elliptical shaped dosage forms are well retained after expanding to their full extent.

In a further aspect, the invention provides a method of treating a subject in need thereof with a theraeutic agent that comprises administering to the subject a dosage form adapted to be retained in the stomach over a prolonged period and further adapted to rapidly disintegrate at the higher pH environment of the upper GI tract. In certain embodiments, the method comprises administering one or more dosage forms to a subject in the fed state at the start of each dosing period, such as within one hour of the the subject's consuming food. Pharmaceutical dosage forms of this invention can be retained in the stomach for two hours or more, more preferably about five hours or more.

In a specific embodiment, the present invention provides methods for treating or ameliorating one or more symptoms of HCV, or disorders associated with HCV comprising administering an effective amount of the aforementioned controlled-release dosage formulation(s) comprising compound 1, compound 2 or compound 3, or a mixture of two or more thereof, to a subject in need of such treatment. In yet another specific embodiment, the present invention provides methods for treating or ameliorating one or more symptoms of type-2 diabetes comprising administering an effective amount of of the aforementioned controlled-release dosage formulation comprising Metformin. In yet a further specific embodiment, the present invention provides methods for treating or ameliorating one or more symptoms of hyperlipidemia or dislipedemia comprising administering an effective amount of of the aforementioned controlled-release dosage formulation comprising Niacin.

The following non-limiting examples illustrate various features of emodiments of the present invention. These illustrative examples are in no way intended to be limiting on any embodiments.

### Example 1

Matrix Tablets I and II, containing the HCV serine protease inhibitor compound 1 as the therapeutic agent and which are targeted to give about 6 hour and about 4 hour drug release profiles, respectively are prepared as follows.

### Preparation of Matrix Tablet I

As listed in Table 1, each of the ingredients for Tablet I are weighed to an accuracy of 0.02 g into a 500 cc amber glass bottle which is then closed (total:108 g for 120 tablets). The bottle is subject to tumble mixing for 10 min using a Turbula Shaker-Mixer (vendor: Glen Mills Inc). The blend is then passed in small portions through a 20 mesh sieve (U.S. Standard Testing Sieve, ATM, and No. L3-30) with a spatula. The entire blend that passed through the screen is pooled together. The passing of the blend through the 20 mesh sieve is repeated until the entire blend has passed through the sieve three times. 900 mg of the final blend is weighed and pressed into a capsule-shape tablet using a Carver press under the following conditions: (a) two lower punches : size 0.750 x 0.328 capsule shape; (b) die size : 0.750 x 0.3281 capsule shape; and (c) pressure setting: 1000 psi. The tablet is collected after compression. The formation of tablets is repeated to make 100 tablets, which are stored in amber glass bottles at 4°C until use or shipping.

**TABLE I**

| Ingredients: | % w/w | For 1 tablet (mg) | For 120 tablets (g) |
|---|---|---|---|
| Kollidon SR | 10 | 90 | 10.80 |
| Crospovidone | 27.56 | 248 | 29.80 |
| Carbopol 71G | 10 | 90 | 10.80 |
| HPMCAS | 7 | 63 | 7.56 |
| LG | | | |
| Mg Stearate | 1 | 9 | 1.08 |
| Therapeutic Agent | 44.44 | 400 | 48.00 |
| Total | 100 | 900 | 108.00 |

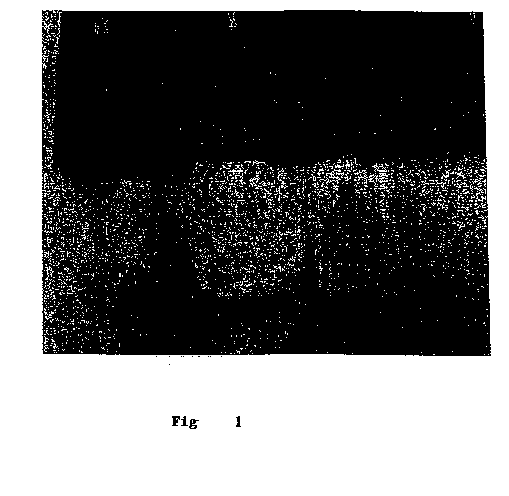

Matrix tablet I is rapidly expanded in simulated gastric fluid with volume expansion more than 3-fold as shown in Figure 1.

The dissolution profile for Matrix Tablet I was obtained using the procedure generally described above herein in Table P, Dissolution Procedure.

The dissolution profile for Matrix Tablet I is shown in Figure 2. A significant burst release was observed for Matrix Tablet I after switching the dissolution media from pH 1.2 to pH 6.8 due to the pH sensitive polymers such as Carbopol and hydroxypropyl methylcellulose acetate succinate (HPMCAS, available as Hypromellose acetate succinate) in the formulation of the invention.

### Preparation of Matrix Tablet II

As listed in Table 2, each of the ingredients in Matrix Tablet II are weighed to an accuracy of 0.02 g into a 500 cc amber glass bottle which is then closed (total:102 g for 120 tablets). The bottle is subject to tumble mixing for 10 min using a Turbula Shaker-Mixer (vendor: Glen Mills Inc). The blend is then passed in small portions through a 20 mesh sieve (U.S. Standard Testing Sieve, ATM, No. L3-30) with a spatula. The entire blend that passed through the screen is pooled together. The passing of the blend through the 20 mesh sieve is repeated until the entire blend has passed through the sieve three times. 850 mg of the final blend is weighed and pressed into a capsule-shape tablet using a Carver press under the following conditions: (a) two lower punches : size 0.750 x 0.328 capsule shape; (b) die size : 0.750 x 0.3281 capsule shape; and (c) pressure setting:1000 psi. The tablet is collected after compression. The formation of tablets is repeated to make 100 tablets, which are stored in amber glass bottles at 4°C until use or shipping.

**TABLE 2**

| Ingredient: | % w/w | For 1 tablet (mg) | For 120 tablets (g) |
|---|---|---|---|
| Kollidon SR | 11.97 | 101.7 | 12.20 |
| Crospovidone | 28.59 | 243 | 29.16 |
| Carbopol 71G | 6.67 | 56.7 | 6.80 |
| HPMCAS | 4.76 | 40.46 | 4.86 |
| LG | | | |
| Mg Stearate | 0.95 | 8.08 | 0.97 |
| Therapeutic Agent | 47.06 | 400 | 48.00 |
| Total | 100 | 850 | 102.00 |

The dissolution profile for Matrix Tablet II is shown in Figure 2. A significant burst release was observed for Matrix Tablet II after switching the dissolution media from pH 1.2 to pH 6.8 due to the pH sensitive polymers such as Carbopol and hydroxypropyl methylcellulose acetate succinate (HPMCAS) in the formulation of the invention.

The above results demonstrate that formulations prepared according to the teaching of the present invention will provide sustained release of therapeutic agent in the acidic pH of the stomach and rapid release in the increased pH of the small intestine.

Additional tablet formulations are set forth Table 3A. The dissolution profiles of such matrix tablets are presented in Table 3B.

**Table 3A**

| | | | | | |
|---|---|---|---|---|---|
| (All tablet formulations in Table 3A have a total weight of 900 mg, including 400 mg of drug substance plus therapeutic agent i.e. Compound 1 per table (44.44%); all tablets also include 1.0% magnesium stearate.) | | | | | |

| Formulation | Ingredient | | | | |
|---|---|---|---|---|---|
| | Kollidon SR | Crospovidone | Carbopol | HPMCAS | Avicel PH102 |
| #1 | 18.0% | 23.6% | 9.0% | 4.0% | - |
| #2 | 16.6% | 25.0% | 9.5% | 3.5% | - |
| #3 | 17.1% | 24.0% | 9.5% | 4.0% | - |
| #4 | 17.6% | 25.0% | 8.0% | 4.0% | - |
| #5 | 15.6% | 28.0% | 8.0% | 3.0% | - |
| #6 | 16.0% | 23.6% | 12.0% | 3.0% | - |
| #7 | 12.6% | 32.0% | 5.0% | 5.0% | - |
| #8 | 12.6% | 30.0% | 7.0% | 5.0% | - |
| #9 | 12.6% | 34.0% | 5.0% | 3.0% | - |
| #10 | 11.0% | 26.6% | 12.0% | 5.0% | - |
| #12 | 10.0% | 29.6% | 10.0% | 5.0% | - |
| #13 | 12.6% | 32.0% | 7.0% | 3.0% | - |
| #14 | 13.6% | 32.0% | 6.0% | 3.0% | - |
| #15 | 10.6% | 32.0% | 7.5% | 4.5% | - |
| #16 | 11.6% | 32.0% | 8.0% | 3.0% | - |
| #17 | 10.6% | 34.0% | 7.0% | 3.0% | - |
| #18 | 8.6 % | 34.0% | 9.0% | 3.0% | - |
| #19 | 8.6 % | 34.0% | 7.0% | 5.0% | - |
| #20 | 11.6% | 34.0% | 6.0% | 3.0% | - |
| #21 | 13.6% | 31.0% | 5.0% | 5.0% | - |
| #22 | 14.6% | 32.0% | 5.0% | 3.0% | - |
| #23 | 14.6% | 25.0% | 5.0% | 5.0% | 5.0% |
| #24 | 14.6% | 30.0% | 5.0% | 5.0% | - |

**Table 3B**

| Formulation | % Compound 1 dissolved | | | | | |
|---|---|---|---|---|---|---|
| | 1 hour | 2 hour | 3 hour | 4 hour | 5 hour | 6 hour |
| #1 | 13.5 | 27.9 | 32.5 | 39.9 | 63.6 | 69.6 |
| #2 | 12.5 | 26.1 | 38.7 | 44.1 | 67.8 | 72.6 |
| #3 | 13.1 | 28.3 | 40.1 | 48.5 | 70.5 | 75.7 |
| #4 | 15.7 | 32.5 | 41.3 | 46.4 | 72.9 | 77.8 |
| #5 | 15.2 | 26.6 | 48.1 | 58.4 | 76.1 | 79.8 |
| #6 | 8.2 | 15.1 | 18.2 | 25.2 | 64.2 | 68.0 |
| #7 | 66.4 | 81.6 | 87.9 | 91.6 | 98.1 | 99.2 |
| #8 | 34.5 | 47.5 | 55.4 | 61.7 | 89.5 | 93.4 |
| #9 | 62.1 | 78.8 | 86.8 | 91.2 | 99.8 | 101.3 |
| #10 | 21.7 | 32.8 | 35.6 | 39.3 | 58.6 | 82.9 |
| #12 | 33.4 | 43.1 | 48.8 | 52.6 | 88.1 | 93.1 |
| #13 | 28.5 | 40.6 | 49.0 | 56.9 | 80.0 | 92.5 |
| #14 | 33.8 | 49.6 | 56.6 | 62.1 | 80.1 | 85.2 |
| #15 | 27.6 | 39.1 | 45.4 | 50.5 | 79.6 | 87.9 |
| #16 | 29.4 | 42.1 | 51.0 | 57.1 | 80.1 | 92.5 |
| #17 | 22.8 | 34.1 | 40.1 | 44.6 | 85.4 | 95.6 |
| #18 | 16.9 | 25.7 | 31.5 | 36.1 | 85.5 | 93.1 |
| #19 | 25.3 | 37.8 | 44.9 | 50.2 | 87.2 | 93.9 |
| #20 | 32.5 | 44.5 | 57.0 | 66.1 | 90.3 | 95.6 |
| #21 | 29.4 | 44.8 | 53.1 | 59.8 | 86.5 | 91.0 |
| #22 | 50.2 | 70.2 | 79.9 | 84.9 | 95.6 | 98.3 |
| #23 | 39.1 | 59.8 | 71.1 | 78.9 | 92.3 | 95.7 |
| #24 | 32.0 | 45.2 | 53.4 | 62.6 | 85.5 | 90.9 |

### Example 2

The following example illustrates a wet granulation process for preparation of a formulation of the present invention comprising compound 1. The granulation process is summarized as following:
1. Dissolve povidone K30 and sodium lauryl sulfate (SLS) in water.
2. Charge compound 1 and low-substituted HPC (L-HPC) to a granulator and mix.
3. Granulate the mix from Step 2 with povidone and sodium lauryl sulfate solution.
4. Pass through 8 mesh screen.
5. Dry the wet granulation from Step 4 using a tray dryer.
6. Pass the dried granulation from Step 5 through a suitably-sized screening mill or 18 mesh screen.
7. Blend the granulation with selected excipients.
8. Press tablets.

Compound 1 is easily granulated with various binders such as pregelatined starch, hydroxypropyl cellulose (HPC) and povidone (K-30). The granules show good flowability and compressibility. Pregelatined starch and HPC decrease the swelling and retard the dissolution of the tablets. The inclusion of a disintegrant promotes dissolution.

A particular wet granulation formation of the invention comprising compound 1 comprises 88.2 % compound 1, 8.1% L-HPC, 3.2% Povidone and 0.5% SLS, as disclosed in Table 4. Swellable matrix tablets of HCV protease compound 1 were formulated using high drug loading granules as disclosed in Table 4.

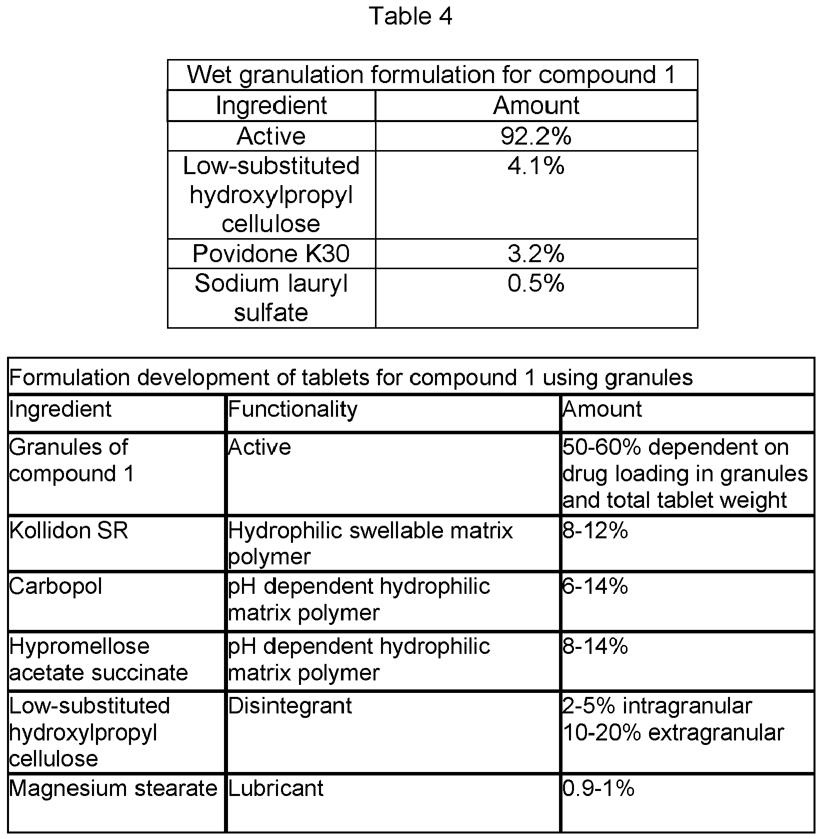

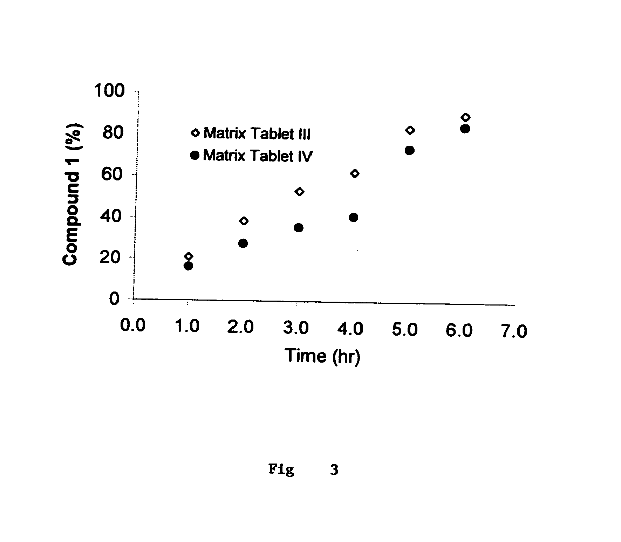

Matrix Tablets III and IV, containing compound 1 were prepared targeting for 4-6 hours release. Formulations are disclosed in Table 5, and dissolution profiles are disclosed in Figure 3. The dissolution profile was obtained using the procedure generally described above herein in Table P, Dissolution Procedure. A significant burst release was observed for both formulations after switching the dissolution media from pH 1.2 to pH 6.8 due to the pH sensitive polymers such as Carbopol and HPMCAS in the formulations of the invention.

**Table 5**

| Formulation of Matrix Tablets III and IV for compound 1 | | |
|---|---|---|
| Ingredient | Matrix Tablet III | Matrix Tablet IV |
| Kollidon SR | 8.30% | 8.30% |
| Low substituted hydroxylpropyl cellulose | 11.80% | 8.80% |
| Carbopol | 10.50% | 14.50% |
| HPMCAS | 8.50% | 7.50% |
| Mg stearate | 0.90% | 0.90% |
| Granules of compound 1 | 60.00% | 60.00% |

### Example 3

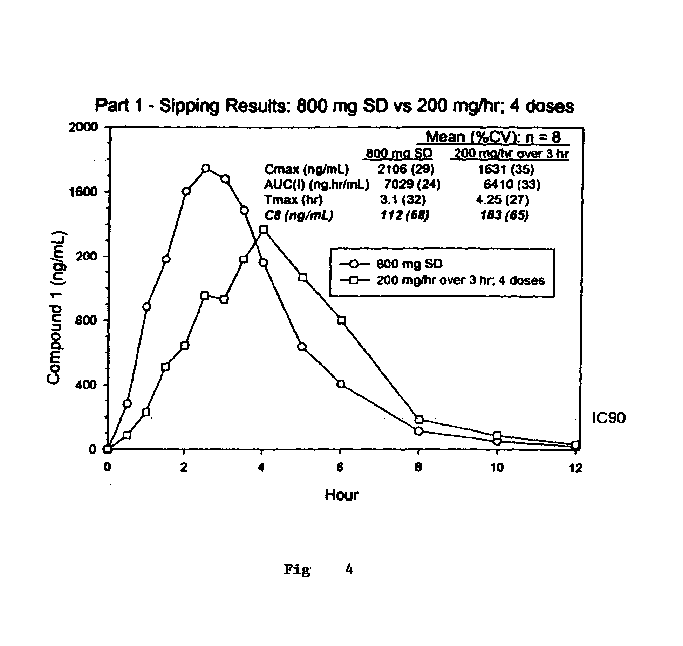

The delivery mechanism of gastric retentive dosage forms can be mimicked by administering small divided doses over time (4 doses, 200 mg/dose, over 4 hours) (sipping dose). In this way the feasibility of increasing trough blood levels, through a sustained release gastric retentive dosage form, can be assessed without the need for formulation development time. This dosing schedule has been tested in humans, and data from this study is shown in Figure 4. The results demonstrate that at an input rate of 200 mg/h, the AUC is only slightly lower that of bolus drug input, indicating a minimum risk of a first-pass barrier for sustained drug delivery from the stomach. Additionally, these results show that a further extension of the drug input time (from currently 3 h to 4, or 5, or 6 h) is likely to elevate the drug concentration at C8h.

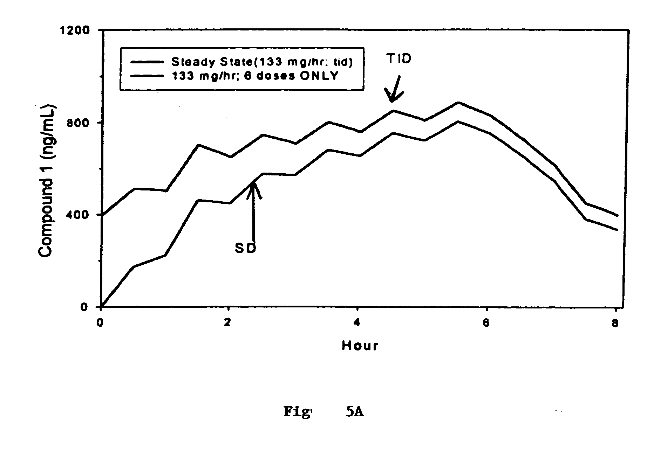

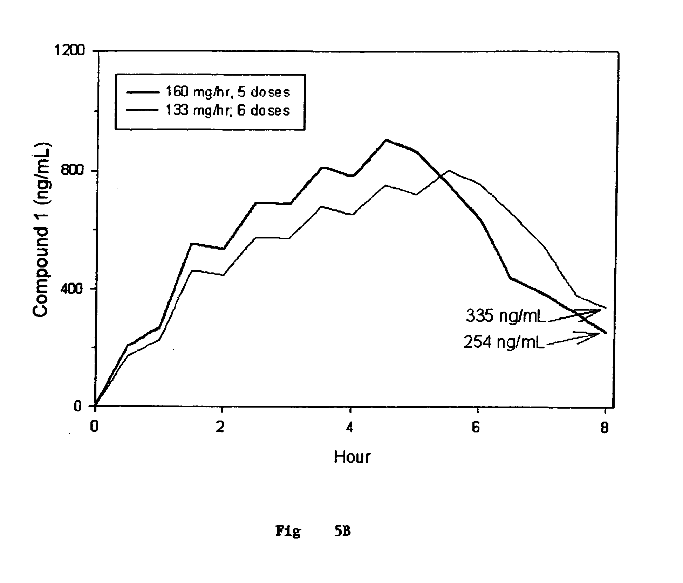

Additionally, Figure 5A demonstrates simulated profiles for input rates of 160 mg/hr for 5 hours and 133 mg/hr for 6 hours. The C8h is progressively increased as the input rate is reduced to 133 mg/hr. Figure 5B simulates that at steady state a formulation providing an input rate of 133mg/hr and dosed every 8 hours will provide an elevated C8h of approximately 400 ng/ml.

### Example 4

Compound 1, a potent, orally active, novel HCV-specific serine protease inhibitor, as a treatment for chronic hepatitis C. The chemical name of compound 1 is (1R,5S)-N-[Amino-1-(cyclobutYmmethYI)-2,3-dioxopropyl]-3-[2(S)-[[[(1,1-dimethylethyl) amino] carbonyl] amino]-3-3-dimethYl-1-oxbutyl]-6,6-dimethyl-3-azabicyclo[3.1.0]hexan-2(S)-carboxamide. Compound 1 binds specifically to the HCV non-structural protein 3 (NS3), which is the protease that mediates the cleavage of the HCV polyprotein to form the functional proteins essential for viral propagation. The ketoamide portion of compound 1 forms a stable, covalent and reversible complex that inhibits the NS3 protease, preventing generation of functional viral proteins and suppressing viral replication in infected host cells.

In addition to its low solubility and relatively slow dissolution rate, compound 1 is also thought to be a P-gp substrate and subject to metabolism by CYP 3A4 and/or AKR enzymes. These factors combine to give an overall low bioavailability of compound 1. Furthermore, the molecule is subject to rapid clearance and has a half life of less than 2 hours. This short half life leads to a drop in drug blood levels to below the IC 90 value of 200 ng/mL at 8 hours (08) postdose. For maximum efficacy, blood levels of compound 1 should be maintained above the IC 90 value at least until the 8-hours postdose time point for TID dosing.

Altering the pharmacokinetic profile and increasing the bioavailability of compound 1 will, in addition to improving efficacy, potentially allow less drug to be used. One approach to increase the bioavailability of compound 1 and in particular to raise the plasma concentrations at 8 hours postdose is to extend the duration of drug release to the upper GI tract via a gastro-retentive controlled-release dosage form.

The following doses are administered to subjects to determine the pharmacokinetics of the gastro-retentive controlled release dosage forms of the invention:
Treatment A A single 800 mg dose (4 x 200 mg 3% SLS formulation) of compound 1 with 240 mL of water,
Treatment B 2 x 400 mg dose of compound 1 Matrix Tablet I (see, Example 1) with 240 mL of water
Treatment C 2 x 400 mg dose of compound 1 Matrix Tablet II with 240 mL of water (see, Example 1).

The treatments are administered orally to the subjects in a seated position after a standard breakfast with the dose level specified volume of water. No additional food intake occurs until after collection of the 8 hour PK sample.

To determine the pharmacokinetics of the gastro-retentive controlled release dosage forms of the invention, the enantiomers of compound 1 are measured from PK samples and summed to provide concentrations of compound 1. Plasma concentration data for compound 1 is used to estimate the following primary pharmacokinetic variables for the determination of bioavailability comparisons: (i) C8, the concentration 8 hours postdose; (ii) C12, the concentration 12 hours postdose; (iii) Cmax, the maximum observed plasma concentration; (iv) Tmax, the time to maximum observed plasma concentration; (v) AUC, the area under the concentration-time curve; and (vi) t ½, the terminal phase half life. For maximum efficacy, blood levels of compound 1 should be maintained above the IC 90 value of 200 ng/mL at 8 hours postdose time point for TID dosing.

Additionally, pharmacogenetics studies are performed. For example, DNA sequence analysis of genes determined to play a role in the pharmacokinetics of compound 1 is performed. In the event of a serious adverse event, genes thought to be involved in creating a genetic predisposition to that event may also be examined. The scope of research performed on samples obtained for pharmacogenetic analysis is the investigation of polymorphisms or variations in drug metabolizing enzymes, receptors, transporters, or cellular pathways that result in a clinical phenotype in patients.

Safety variables to be assessed after administration of the drug include vital signs, physical examinations, ECGs, reporting of adverse events, hematology and blood chemistry.

### Example 5

Matrix Tablets of compound 2 were prepared according to the present invention to provide a novel controlled release gastroretentive formulation designed for the controlled release of compound 2 in the stomach followed by a pH-sensitive more enhanced release of remaining drug from the matrix in the small intestine.

Matrix Tablets of compound 2 were prepared by using a dry blending and a direct compressing by a. Carver Press, summarized as following:
1. Weigh all ingredients in pre-determined amounts, pool them into a blender
2. Blend all ingredients in a blender (turbula mixer).
3. Pass the blend through a 16 mesh screen.
4. Repeat steps 2 and 3 three times.
5. Compress the blend into tablets by hand using a Carver press
6. Pack in HDPE bottles or glass bottles.

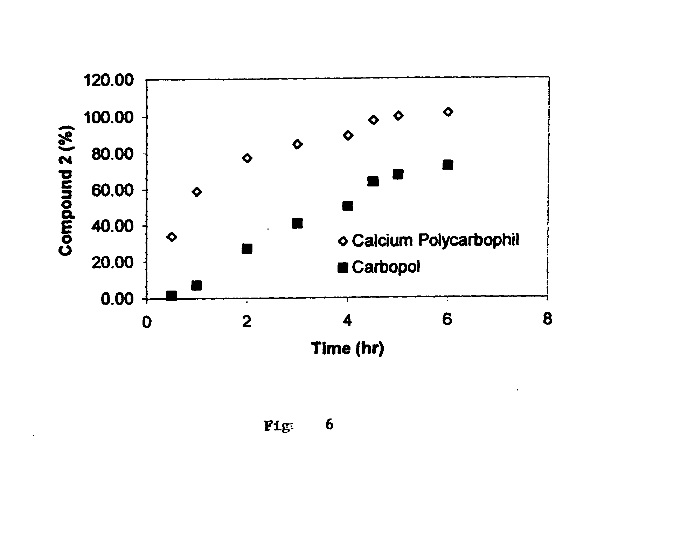

More than 20 prototype formulations with various combinations of Kollidon SR, crospovidone, Carbopol, Calcium Polycarbophil and HPMCAS were prepared and tested in vitro. These tablets expanded rapidly in pH 1.2 media and their volume more than tripled on expansion. Representative formulations are listed in Table 6 and the dissolution profiles obtained using the procedure generally described above herein in Table P are shown in Figure 6.

**Table 6**

| Ingredients | Formulation #16 | Formulation #19 |
|---|---|---|
| HCV protease inhibitor compound 2 granules | 57.9% | 57.9% |
| (containing 43.2% API) | | |
| Kollidon SR | 10.0% | 10.0% |
| Carbopol | 10.0% | |
| Calcium Polycarbophil | | 10.0% |
| Crospovidone | 21.1% | 21.1% |
| Mg Stearate | 1.0% | 1.0% |
| Tablet Weight | 1000 | 1000 |
| (mg) | | |

Controlled release was observed in pH 1.2 media. A burst release profile was also observed after switching media from pH 1.2 to pH 6.8 in accord with the teaching of the present invention.

### Example 7

Matrix Tablets of Metfomin were prepared to provide a novel controlled release gastroretentive formulation designed for the controlled release of Metformin in the stomach followed by a pH-enhanced more complete release of remaining drug from the matrix.

Matrix Tablets of Metformin were prepared by using a dry blending and a direct compressing by a.Carver Press, as summarized in Example 6:

About 30 formulations with various combinations of Kollidon SR, crospovidone, Low-substituted Hydroxylpropyl Cellulose, Carbopol, and HPMCAS were prepared as summarized in Table 7.

**Table 7**

| Ingredient | Functionality | Amount |
|---|---|---|
| Metformin | Active | 25-55% |
| | | (250-500 mg per tablet) |
| Kollidon SR | Hydrophilic Swellable Matrix Polymer | 5-25% |
| Carbopol | pH dependent hydrophilic Polymer | 15-60% |
| Hypromellose acetate succinate | pH dependent hydrophilic matrix Polymer | 5-40% |
| Crospovidone | Disintegrant | 0-12% |
| Low substituted hydroxypropylcellulose (L-HPC) | Disintegrant | 0-6% |
| Magnesium Stearate | Lubricant | 1% |
| Total | | 100% |
| | | (900-1000 mg per tablet) |

Matrix tablet formulations prepared according to the teaching of the present invention expanded rapidly in pH 1.2 media and volume more than tripled on expansion. The rate of expansion and erosion appear related to the amount of disintegrant and hydrophilic swellable polymer in the formulations. A representative formulation is listed in Table 8. In contrast, unexpectedly, when a swellable polymer such as Kollidon SR was removed from formulations shown in Table 7, the tablet structure was poor and they easily disintegrated.

**Table 8**

| Ingredient | %Amount | Amount per tablet |
|---|---|---|
| Metformin | 27.8% | 250 mg |
| Kollidon SR | 21.1% | 190 mg |
| Carbopol | 42.3% | 381 mg |
| Hypromellose acetate succinate | 5.6% | 50 mg |
| Crospovidone | 2.2% | 20 mg |
| Magnesium Stearate | 1% | 9 mg |
| Total | 100% | 900 mg |

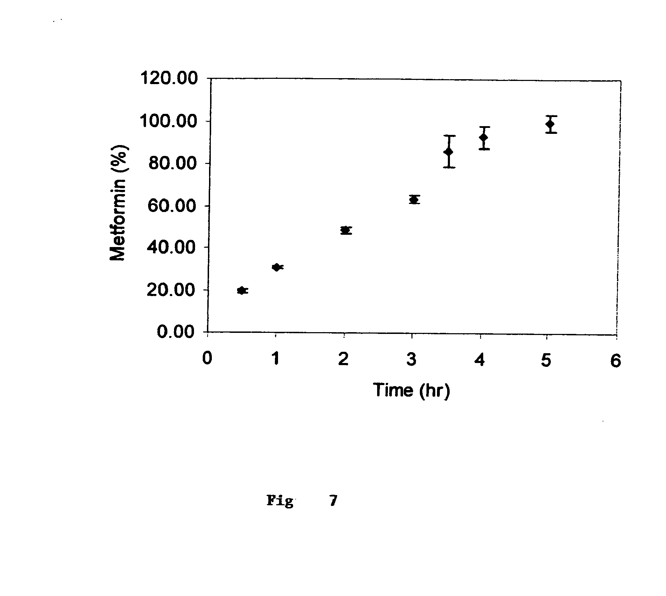

The dissolution profile was obtained using the procedure generally described above herein in Table P, Dissolution Procedure. Dissolution profiles are shown in Figure 7. Controlled release was observed in pH 1.2 media with 60% release within 3 hours. A burst release profile was also observed after switching media from pH 1.2 to pH 6.8 due to the presence of pH sensitive polymers.

### Example 8

Matrix Tablets of Niacin were prepared to provide a novel controlled release gastroretentive formulation designed for the controlled release of Niacin in the stomach followed by a pH-enhanced more complete release of any remaining drug from the matrix.

Matrix Tablets of Niacin were prepared by using a dry blending and a direct compressing by a.Carver Press, as summarized in Example 6:

About 8 formulations with various combinations of Kollidon SR, crospovidone, Carbopol, and HPMCAS were prepared as summarized inTable 9

**Table 9**

| Ingredient | Functionality | Amount |
|---|---|---|
| Niacin | Active | 27.8% |
| | | 250 mg/900 mg tablet |
| Kollidon SR | Hydrophilic Swellable Matrix Polymer | 0-25% |
| Carbopol | pH dependent hydrophilic matrix Polymer | 20-60% |
| Hypromellose acetate succinate | pH dependent hydrophilic matrix Polymer | 5-30% |
| Crospovidone | Disintegrant | 2-12% |
| Magnesium Stearate | Lubricant | 1% |
| Total | | 100% |
| | | (900 mg per tablet) |

.Matrix tablet formulations expanded rapidly in pH 1.2 media and volume more than tripled on expansion. The rate of expansion and erosion appear related to the amount of superdisintegrant and swellable hydrophilic polymer in formulations. A representative formulation is listed in Table 10.

**Table 10**

| Ingredient | %Amount | Amount per tablet |
|---|---|---|
| Niacin | 27.8% | 250 mg |
| Kollidon SR | 12.2% | 110 mg |
| Carbopol | 25.6% | 230 mg |
| Hypromellose acetate succinate | 27.8% | 250 mg |
| Crospovidone | 5.7% | 51 mg |
| Magnesium Stearate | 1% | 9 mg |
| Total | 100% | 900 mg |

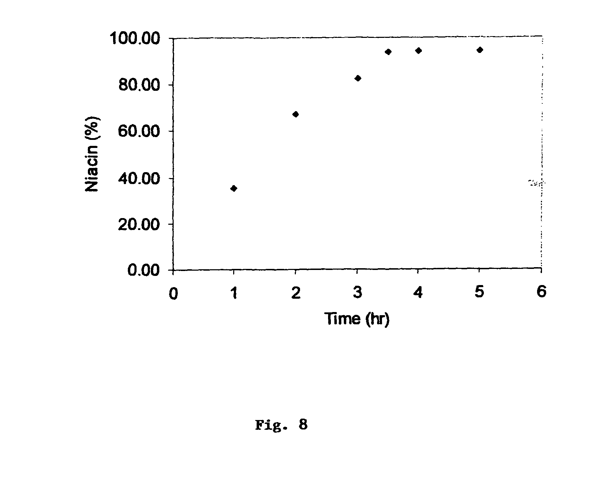

The dissolution profile was obtained using the procedure generally described above herein in Table P, Dissolution Procedure The dissolution profiles are shown in Figure 8. Controlled release was observed in pH 1.2 media with 80% release within 3 hours. The remaining drug was fully released within 30 min after switching the media from pH 1.2 and 6.8.

The present invention is not to be limited in scope by the specific embodiments described herein which are intended as single illustrations of individual aspects of the invention, and functionally equivalent methods and components are within the scope of the invention. Indeed, various modifications of the invention, in addition to those shown and described herein will become apparent to those skilled in the art from the foregoing description and accompanying drawings. Such modifications are intended to fall within the scope of the claims. Various publications are cited herein, the contents of which are hereby incorporated, by reference, in their entireties.

The present application claims priority benefits of U.S. provisional application Nos. 60/873872 and 60/873928 both filed on December 7, 2006, the entire contents of each of which are incorporated herein by reference in their entirety.

## Claims

1. A controlled release formulation for oral administration comprising one or more swellable polymers, one or more pH sensitive polymers and a therapeutic agent, wherein the controlled release formulation sustainedly releases the therapeutic agent in the acidic pH of stomach; and rapidly releases the therapeutic agent in the increased pH of the small intestine.

2. The controlled release formulation of claim 1 wherein the controlled release formulation expands in size for gastric retention after oral administration.

3. The controlled release formulation of claim 1 wherein the pH sensitive polymer is Carbapol 71 G, hydroxypropyl methycellulose acetate succinate, Eudragit L-100, Eudragit S-1 00, Eudragit L-30D, Euragit FS 30D, Eudragit L-100-55, polyvinyl acetate, hydroxypropyl methylcellulose phthalate, hydroxypropyl methylcellulose phthalate 50, hydroxypropyl methylcellulose phthalate 55, cellulose acetate phthalate, cellulose acetate trimellate, or a mixture of 2 or more of the above.

4. The controlled release formulation of claim 3 wherein the pH sensitive polymer is hydroxypropyl methylcellulose acetate succinate or Carbopol or a mixture thereof.

5. The controlled release formulation of claim 1 wherein the swellable polymer is hydrophilic and is a: polyalkylene oxide; cellulosic polymer; acrylic acid or methacrylic acid polymer, or a copolymer or ester thereof; maleic anhydride copolymer; polymaleic acid; poly(acrylamide); poly(olefinic alcohol); polyol; polyoxazoline; polyvinylamine; polyvinylacetate; polyimine ; starch or starch-based polymer; polyurethane hydrogel; chitosan; polysaccharide gum; zein; shellac, ammoniated shellac, shellac-acetyl alcohol, or shellac n-butyl stearate; or a mixture of 2 or more of the above.

6. The controlled release formulation of claim 5 wherein the swellable polymer is polyvinyl acetate.

7. The formulation of claim 5 wherein the polyvinyl acetate is Kollidon SR.

8. The controlled release formulation of claim 1 further comprising one or more disintegrants.

9. The controlled release formulation of claim 8 wherein the disintegrant is a superdisintergrant.

10. The controlled release formulation of claim 8 wherein the disintegrant is cross-linked carboxymethyl cellulose sodium, sodium starch glycolate, low-substituted hydroxypropyl cellulose, cross-linked polyvinyl pyrollidone, or a mixture of 2 or more of the above.

11. The formulation of claim 1 wherein the pH sensitive polymer is hydroxypropyl methylcellulose acetate succinate or Carbopol or a mixture thereof; the swellable polymer is polyvinyl acetate wherein the polyvinyl acetate is Kollidon SR; and further comprising a disintegrant, wherein the disintegrant is cross-linked carboxymethyl cellulose sodium, sodium starch glycolate, low-substituted hydroxypropyl cellulose cross-linked polyvinyl pyrollidone, or a mixture of 2 or more of the above.

12. The formulation of claim 1 further comprising a lubricant, a surfactant, or a lubricant and a surfactant.

13. The formulation of claim 1 wherein the therapeutic agent is 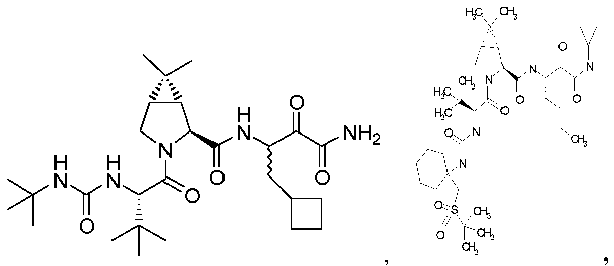 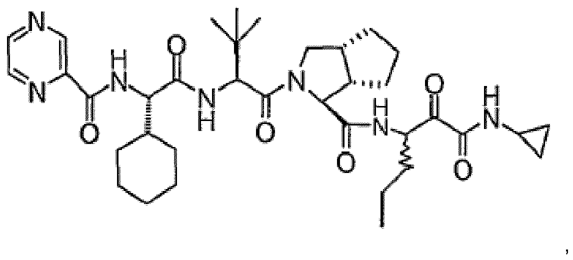 Metformin or Niacin, or a pharmaceutically acceptable, salt, solvate or ester thereof.

14. A controlled release formulation comprising: 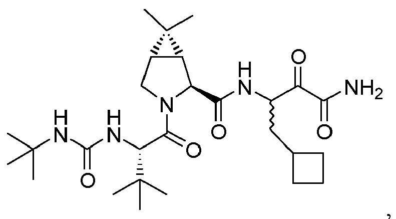 one or more swellable hydrophilic polymers, one or more pH sensitive polymers and a disintegrant, which, when tested in a USP2 apparatus Paddle Stirrer filled with 900 ml pH 1.2 HCl dissolution medium with or without 0.5% Tween 80 at for three or four hours, followed by 900 ml of phosphate buffer at pH 6.8 with or without 0.5% Tween 80 for 2 hours, 50 to 100 rpm stir speed, at 37 °C, has the dissolution profile shown in Figure 2.

15. A controlled release formulation comprising: 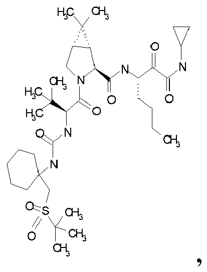 one or more swellable hydrophilic polymers, one or more pH sensitive polymers and a disintegrant, which, when tested in a USP2 apparatus Paddle Stirrer filled with 900 ml pH 1.2 HCl dissolution medium with or without 0.5% Tween 80 at for three or four hours, followed by 900 ml of phosphate buffer at pH 6.8 with or without 0.5% Tween 80 for 2 hours, 50 to 100 rpm stir speed, at 37 °C, has the dissolution profile shown in Figure 6.

16. The controlled release formulation of claim 1 wherein the therapeutic agent in an HCV protease inhibitor of Formula 1 - XXVIII.

17. A controlled release formulation comprising: Metformin one or more swellable hydrophilic polymers, one or more pH sensitive polymers and optionally a disintegrant, which, when tested in a USP2 apparatus Paddle Stirrer filled with 900 ml pH 1.2 HCl dissolution medium with or without 0.5% Tween 80 at for three or four hours, followed by 900 ml of phosphate buffer at pH 6.8 with or without 0.5% Tween 80 for 2 hours, 50 to 100 rpm stir speed, at 37 °C, has the dissolution profile shown in Figure 7.

18. A controlled release formulation comprising: Niacin one or more swellable hydrophilic polymers, one or more pH sensitive polymers and optionally a disintegrant, which, when tested in a USP2 apparatus Paddle Stirrer filled with 900 ml pH 1.2 HCl dissolution medium with or without 0.5% Tween 80 at for three or four hours, followed by 900 ml of phosphate buffer at pH 6.8 with or without 0.5% Tween 80 for 2 hours, 50 to 100 rpm stir speed, at 37 °C, has the dissolution profile shown in Figure 8.
